(19) **Europäisches Patentamt / European Patent Office / Office européen des brevets**

(11) **EP 3 373 175 B1**

(12) **EUROPEAN PATENT SPECIFICATION**

(45) Date of publication and mention of the grant of the patent:
**28.05.2025 Bulletin 2025/22**

(21) Application number: **17207702.6**

(22) Date of filing: **22.11.2006**

(51) International Patent Classification (IPC):
**G16B 20/00** (2019.01)  **G16B 30/00** (2019.01)
**G16B 20/20** (2019.01)  **G16B 40/20** (2019.01)
**G16B 40/30** (2019.01)  **C12Q 1/6809** (2018.01)
**C12Q 1/6869** (2018.01)  **C12Q 1/6874** (2018.01)

(52) Cooperative Patent Classification (CPC):
(C-Sets available)
**G16B 30/00; C12Q 1/6809; C12Q 1/6869; C12Q 1/6874; G16B 20/00; G16B 20/20; G16B 40/20; G16B 40/30;** G16B 20/10; G16B 20/40; G16B 40/00        (Cont.)

(54) **SYSTEM AND METHOD FOR CLEANING NOISY GENETIC DATA AND USING DATA TO MAKE PREDICTIONS**

SYSTEM UND VERFAHREN ZUM REINIGEN VON RAUSCHENDEN GENETISCHEN DATEN UND VERWENDEN VON DATEN ZUR STELLUNG VON PROGNOSEN

SYSTÈME ET PROCÉDÉ POUR NETTOYER DES DONNÉES GÉNÉTIQUES BRUYANTES ET AU MOYEN DE DONNÉES POUR FAIRE DES PRÉDICTIONS

(84) Designated Contracting States:
**AT BE BG CH CY CZ DE DK EE ES FI FR GB GR HU IE IS IT LI LT LU LV MC NL PL PT RO SE SI SK TR**

(30) Priority:
**26.11.2005 US 739882 P**
**06.12.2005 US 742305 P**
**29.12.2005 US 754396 P**
**21.02.2006 US 774976 P**
**04.04.2006 US 789506 P**
**30.06.2006 US 817741 P**
**31.07.2006 US 496982**
**22.09.2006 US 846610 P**

(43) Date of publication of application:
**12.09.2018 Bulletin 2018/37**

(60) Divisional application:
**18212459.4 / 3 599 609**

(62) Document number(s) of the earlier application(s) in accordance with Art. 76 EPC:
**06838311.6 / 1 960 929**

(73) Proprietor: **Natera, Inc.**
**San Carlos, CA 94070 (US)**

(72) Inventors:
• **RABINOWITZ, Matthew**
**Portola Valley, CA California 94028 (US)**
• **BANJEVIC, Milena**
**New York, NY New York 10009 (US)**
• **DEMKO, Zachary Paul**
**Somerville, MA Massachusetts 02144 (US)**
• **JOHNSON, David Scott**
**Portola Valley, CA California 94028 (US)**

(74) Representative: **Marks & Clerk LLP**
**15 Fetter Lane**
**London EC4A 1BW (GB)**

(56) References cited:
**US-A1- 2004 137 470**

• **RAOUL-SAM DARUWALA ET AL: "A versatile statistical analysis algorithm to detect genome copy number variation", vol. 101, no. 46, 16 November 2004 (2004-11-16), pages 16292 - 16297, XP008155946, ISSN: 0027-8424, Retrieved from the Internet <URL:http://www.ncbi.nlm.nih.gov> [retrieved on 20041108], DOI: 10.1073/ PNAS.0407247101**

**(Cont. next page)**

• **YASUHITO NANNYA ET AL: "A Robust Algorithm for Copy Number Detection Using High-Density Oligonucleotide Single Nucleotide Polymorphism Genotyping Arrays", vol. 65, no. 14, 14 July 2005 (2005-07-14), pages 6071 - 6079, XP008155967, ISSN: 0008-5472, Retrieved from the Internet <URL:http://cancerres.aacrjoumals. org> [retrieved on 20050715], DOI: 10.1158/ 0008-5472.CAN-05-0465**

(52) Cooperative Patent Classification (CPC): (Cont.)

C-Sets
**C12Q 1/6809, C12Q 2535/122, C12Q 2537/165;
C12Q 1/6869, C12Q 2535/122, C12Q 2537/165;
C12Q 1/6874, C12Q 2535/122, C12Q 2537/165**

## Description

<u>Field of the Technology</u>

The disclosure relates generally to the field of acquiring, manipulating and using genetic data for medically predictive purposes, and specifically to a system in which imperfectly measured genetic data is made more precise by using known genetic data of genetically related individuals, thereby allowing more effective identification of genetic irregularities that could result in various phenotypic outcomes. It also relates generally to the field of analyzing, managing and acting upon genetic, phenotypic and clinical information, and using that information to predict phenotypic outcomes of medical decisions. More specifically, it relates to methods and systems which use integrated, validated genetic and phenotypic data from a group of subjects to make better decisions regarding a particular subject.

<u>Description of the Related Art</u>

*Prenatal and Preimplantation Genetic Diagnosis*

Current methods of prenatal diagnosis can alert physicians and parents to abnormalities in growing fetuses. Without prenatal diagnosis, one in 50 babies is born with serious physical or mental handicap, and as many as one in 30 will have some form of congenital malformation. Unfortunately, standard methods require invasive testing and carry a roughly 1 per cent risk of miscarriage. These methods include amniocentesis, chorion villus biopsy and fetal blood sampling. Of these, amniocentesis is the most common procedure; in 2003, it was performed in approximately 3% of all pregnancies, though its frequency of use has been decreasing over the past decade and a half. A major drawback of prenatal diagnosis is that given the limited courses of action once an abnormality has been detected, it is only valuable and ethical to test for very serious defects. As result, prenatal diagnosis is typically only attempted in cases of high-risk pregnancies, where the elevated chance of a defect combined with the seriousness of the potential abnormality outweighs the risks. A need exists for a method of prenatal diagnosis that mitigates these risks.

It has recently been discovered that cell-free fetal DNA and intact fetal cells can enter maternal blood circulation. Consequently, analysis of these cells can allow early Non-Invasive Prenatal Genetic Diagnosis (NIPGD). A key challenge in using NIPGD is the task of identifying and extracting fetal cells or nucleic acids from the mother's blood. The fetal cell concentration in maternal blood depends on the stage of pregnancy and the condition of the fetus, but estimates range from one to forty fetal cells in every milliliter of maternal blood, or less than one fetal cell per 100,000 maternal nucleated cells. Current techniques are able to isolate small quantities of fetal cells from the mother's blood, although it is very difficult to enrich the fetal cells to purity in any quantity. The most effective technique in this context involves the use of monoclonal antibodies, but other techniques used to isolate fetal cells include density centrifugation, selective lysis of adult erythrocytes, and FACS. Fetal DNA isolation has been demonstrated using PCR amplification using primers with fetal-specific DNA sequences. Since only tens of molecules of each embryonic SNP are available through these techniques, the genotyping of the fetal tissue with high fidelity is not currently possible.

Normal humans have two sets of 23 chromosomes in every diploid cell, with one copy coming from each parent. Aneuploidy, a cell with an extra or missing chromosomes, and uniparental disomy, a cell with two of a given chromosome that originate from one parent, are believed to be responsible for a large percentage of failed implantations, miscarriages, and genetic diseases. When only certain cells in an individual are aneuploid, the individual is said to exhibit mosaicism. Detection of chromosomal abnormalities can identify individuals or embryos with conditions such as Down syndrome, Klinefelters syndrome, and Turner syndrome, among others, in addition to increasing the chances of a successful pregnancy. Testing for chromosomal abnormalities is especially important as mothers age: between the ages of 35 and 40 it is estimated that between 40% and 50% of the embryos are abnormal, and above the age of 40, more than half of the embryos are abnormal.

Karyotyping, the traditional method used for the prediction of aneuploides and mosaicism is giving way to other more high throughput, more cost effective methods. One method that has attracted much attention recently is Flow cytometry (FC) and fluorescence in situ hybridization (FISH) which can be used to detect aneuploidy in any phase of the cell cycle. One advantage of this method is that it is less expensive than karyotyping, but the cost is significant enough that generally a small selection of chromosomes are tested (usually chromosomes 13, 18, 21, X, Y; also sometimes 8, 9, 15, 16, 17, 22); in addition, FISH has a low level of specificity. Using FISH to analyze 15 cells, one can detect mosaicism of 19% with 95% confidence. The reliability of the test becomes much lower as the level of mosaicism gets lower, and as the number of cells to analyze decreases. The test is estimated to have a false negative rate as high as 15% when a single cell is analysed. There is a great demand for a method that has a higher throughput, lower cost, and greater accuracy.

Much research has been done towards the use of pre-implantation genetic diagnosis (PGD) as an alternative to classical prenatal diagnosis of inherited disease. Most PGD today focuses on high-level chromosomal abnormalities such as aneuploidy and balanced translocations with the primary outcomes being successful implantation and a take-home baby. A need exists for a method for more extensive genotyping of embryos at the pre-implantation stage. The number of known disease associated genetic alleles is currently at 389 according to OMIM and steadily climbing. Consequently, it is becoming increasingly relevant to analyze multiple embryonic SNPs that are associated with disease phenotypes. A clear advantage of pre-implantation genetic diagnosis over prenatal diagnosis is that it avoids some of the ethical issues regarding possible choices of action once undesirable phenotypes have been detected.

*Genotyping*

Many techniques exist for isolating single cells. The FACS machine has a variety of applications; one important application is to discriminate between cells based on size, shape and overall DNA content. The FACS machine can be set to sort single cells into any desired container. Many different groups have used single cell DNA analysis for a number of applications, including prenatal genetic diagnosis, recombination studies, and analysis of chromosomal imbalances. Single-sperm genotyping has been used previously for forensic analysis of sperm samples (to decrease problems arising from mixed samples) and for single-cell recombination studies.

Isolation of single cells from human embryos, while highly technical, is now routine in *in vitro* fertilization clinics. To date, the vast majority of prenatal diagnoses have used fluorescent *in situ* hybridization (FISH), which can determine large chromosomal aberrations (such as Down syndrome, or trisomy 21) and PCR/electrophoresis, which can determine a handful of SNPs or other allele calls. Both polar bodies and blastomeres have been isolated with success. It is critical to isolate single blastomeres without compromising embryonic integrity. The most common technique is to remove single blastomeres from day 3 embryos (6 or 8 cell stage). Embryos are transferred to a special cell culture medium (standard culture medium lacking calcium and magnesium), and a hole is introduced into the zona pellucida using an acidic solution, laser, or mechanical drilling. The technician then uses a biopsy pipette to remove a single visible nucleus. Clinical studies have demonstrated that this process does not decrease implantation success, since at this stage embryonic cells are undifferentiated.

There are three major methods available for whole genome amplification (WGA): ligation-mediated PCR (LM-PCR), degenerate oligonucleotide primer PCR (DOP-PCR), and multiple displacement amplification (MDA). In LM-PCR, short DNA sequences called adapters are ligated to blunt ends of DNA. These adapters contain universal amplification sequences, which are used to amplify the DNA by PCR. In DOP-PCR, random primers that also contain universal amplification sequences are used in a first round of annealing and PCR. Then, a second round of PCR is used to amplify the sequences further with the universal primer sequences. Finally, MDA uses the phi-29 polymerase, which is a highly processive and nonspecific enzyme that replicates DNA and has been used for single-cell analysis. Of the three methods, DOP-PCR reliably produces large quantities of DNA from small quantities of DNA, including single copies of chromosomes. On the other hand, MDA is the fastest method, producing hundred-fold amplification of DNA in a few hours. The major limitations to amplification material from a single cells are (1) necessity of using extremely dilute DNA concentrations or extremely small volume of reaction mixture, and (2) difficulty of reliably dissociating DNA from proteins across the whole genome. Regardless, single-cell whole genome amplification has been used successfully for a variety of applications for a number of years.

There are numerous difficulties in using DNA amplification in these contexts. Amplification of single-cell DNA (or DNA from a small number of cells, or from smaller amounts of DNA) by PCR can fail completely, as reported in 5-10% of the cases. This is often due to contamination of the DNA, the loss of the cell, its DNA, or accessibility of the DNA during the PCR reaction. Other sources of error that may arise in measuring the embryonic DNA by amplification and microarray analysis include transcription errors introduced by the DNA polymerase where a particular nucleotide is incorrectly copied during PCR, and microarray reading errors due to imperfect hybridization on the array. The biggest problem, however, remains allele drop-out (ADO) defined as the failure to amplify one of the two alleles in a heterozygous cell. ADO can affect up to more than 40% of amplifications and has already caused PGD misdiagnoses. ADO becomes a health issue especially in the case of a dominant disease, where the failure to amplify can lead to implantation of an affected embryo. The need for more than one set of primers per each marker (in heterozygotes) complicate the PCR process. Therefore, more reliable PCR assays are being developed based on understanding the ADO origin. Reaction conditions for single-cell amplifications are under study. The amplicon size, the amount of DNA degradation, freezing and thawing, and the PCR program and conditions can each influence the rate of ADO.

All those techniques, however, depend on the minute DNA amount available for amplification in the single cell. This process is often accompanied by contamination. Proper sterile conditions and microsatellite sizing can exclude the chance of contaminant DNA as microsatellite analysis detected only in parental alleles exclude contamination. Studies to reliably transfer molecular diagnostic protocols to the single-cell level have been recently pursued using first-round multiplex PCR of microsatellite markers, followed by real-time PCR and microsatellite sizing to exclude chance contamination. Multiplex PCR allows for the amplification of multiple fragments in a single reaction, a crucial requirement in the single-cell DNA analysis. Although conventional PCR was the first method used in PGD, fluorescence in situ hybridization (FISH) is now common. It is a delicate visual assay that allows the detection of nucleic acid within undisturbed cellular and tissue architecture. It relies firstly on the fixation of the cells to be analyzed. Consequently, optimization of the fixation and storage condition of the sample is needed, especially for single-cell suspensions.

Advanced technologies that enable the diagnosis of a number of diseases at the single-cell level include interphase chromosome conversion, comparative genomic hybridization (CGH), fluorescent PCR, and whole genome amplification. The reliability of the data generated by all of these techniques rely on the quality of the DNA preparation. PGD is also costly, consequently there is a need for less expensive approaches, such as mini-sequencing. Unlike most mutation-detection techniques, mini-sequencing permits analysis of very small DNA fragments with low ADO rate. Better methods for the preparation of single-cell DNA for amplification and PGD are therefore needed and are under study. The more novel microarrays and comparative genomic hybridization techniques, still ultimately rely on the quality of the DNA under analysis.

Several techniques are in development to measure multiple SNPs on the DNA of a small number of cells, a single cell (for example, a blastomere), a small number of chromosomes, or from fragments of DNA. There are techniques that use Polymerase Chain Reaction (PCR), followed by microarray genotyping analysis. Some PCR-based techniques include whole genome amplification (WGA) techniques such as multiple displacement amplification (MDA), and Molecular Inversion Probes (MIPS) that perform genotyping using multiple tagged oligonucleotides that may then be amplified using PCR with a singe pair of primers. An example of a non-PCR based technique is fluorescence in situ hybridization (FISH). It is apparent that the techniques will be severely error-prone due to the limited amount of genetic material which will exacerbate the impact of effects such as allele drop-outs, imperfect hybridization, and contamination.

Many techniques exist which provide genotyping data. Taqman is a unique genotyping technology produced and distributed by Applied Biosystems. Taqman uses polymerase chain reaction (PCR) to amplify sequences of interest. During PCR cycling, an allele specific minor groove binder (MGB) probe hybridizes to amplified sequences. Strand synthesis by the polymerase enzymes releases reporter dyes linked to the MGB probes, and then the Taqman optical readers detect the dyes. In this manner, Taqman achieves quantitative allelic discrimination. Compared with array based genotyping technologies, Taqman is quite expensive per reaction (~$0.40/reaction), and throughput is relatively low (384 genotypes per run). While only 1ng of DNA per reaction is necessary, thousands of genotypes by Taqman requires microgram quantities of DNA, so Taqman does not necessarily use less DNA than microarrays. However, with respect to the IVF genotyping workflow, Taqman is the most readily applicable technology. This is due to the high reliability of the assays and, most importantly, the speed and ease of the assay (~3 hours per run and minimal molecular biological steps). Also unlike many array technologies (such as 500k Affymetrix arrays), Taqman is highly customizable, which is important for the IVF market. Further, Taqman is highly quantitative, so anueploidies could be detected with this technology alone.

Illumina has recently emerged as a leader in high-throughput genotyping. Unlike Affymetrix, Illumina genotyping arrays do not rely exclusively on hybridization. Instead, Illumina technology uses an allele-specific DNA extension step, which is much more sensitive and specific than hybridization alone, for the original sequence detection. Then, all of these alleles are amplified in multiplex by PCR, and then these products hybridized to bead arrays. The beads on these arrays contain unique "address" tags, not native sequence, so this hybridization is highly specific and sensitive. Alleles are then called by quantitative scanning of the bead arrays. The Illlumina Golden Gate assay system genotypes up to 1536 loci concurrently, so the throughput is better than Taqman but not as high as Affymetrix 500k arrays. The cost of Illumina genotypes is lower than Taqman, but higher than Affymetrix arrays. Also, the Illumina platform takes as long to complete as the 500k Affymetrix arrays (up to 72 hours), which is problematic for IVF genotyping. However, Illumina has a much better call rate, and the assay is quantitative, so anueploidies are detectable with this technology. Illumina technology is much more flexible in choice of SNPs than 500k Affymetrix arrays.

One of the highest throughput techniques, which allows for the measurement of up to 250,000 SNPs at a time, is the Affymetrix GeneChip 500K genotyping array. This technique also uses PCR, followed by analysis by hybridization and detection of the amplified DNA sequences to DNA probes, chemically synthesized at different locations on a quartz surface. A disadvantage of these arrays are the low flexibility and the lower sensitivity. There are modified approaches that can increase selectivity, such as the "perfect match" and "mismatch probe" approaches, but these do so at the cost of the number of SNPs calls per array.

Pyrosequencing, or sequencing by synthesis, can also be used for genotyping and SNP analysis. The main advantages to pyrosequencing include an extremely fast turnaround and unambiguous SNP calls, however, the assay is not currently conducive to high-throughput parallel analysis. PCR followed by gel electrophoresis is an exceedingly simple technique that has met the most success in preimplantation diagnosis. In this technique, researchers use nested PCR to amplify short sequences of interest. Then, they run these DNA samples on a special gel to visualize the PCR products. Different bases have different molecular weights, so one can determine base content based on how fast the product runs in the gel. This technique is low-throughput and requires subjective analyses by scientists using current technologies, but has the advantage of speed (1-2 hours of PCR, 1 hour of gel electrophoresis). For this reason, it has been used previously for prenatal genotyping for a myriad of diseases, including: thalassaemia, neurofibromatosis type 2, leukocyte adhesion deficiency type I, Hallopeau-Siemens disease, sickle-cell anemia, retinoblastoma, Pelizaeus-Merzbacher disease, Duchenne muscular dystrophy, and Currarino syndrome.

Another promising technique that has been developed for genotyping small quantities of genetic material with very high fidelity is Molecular Inversion Probes (MIPs), such as Affymetrix's Genflex Arrays. This technique has the capability to measure multiple SNPs in parallel: more than 10,000 SNPS measured in parallel have been verified. For small quantities of genetic material, call rates for this technique have been established at roughly 95%, and accuracy of the calls made has been established to be above 99%. So far, the technique has been implemented for quantities of genomic data as small as 150 molecules for a given SNP. However, the technique has not been verified for genomic data from a single cell, or a single strand of DNA, as would be required for pre-implantation genetic diagnosis.

The MIP technique makes use of padlock probes which are linear oligonucleotides whose two ends can be joined by ligation when they hybridize to immediately adjacent target sequences of DNA. After the probes have hybridized to the genomic DNA, a gap-fill enzyme is added to the assay which can add one of the four nucleotides to the gap. If the added nucleotide (A,C,T,G) is complementary to the SNP under measurement, then it will hybridize to the DNA, and join the ends of the padlock probe by ligation. The circular products, or closed padlock probes, are then differentiated from linear probes by exonucleolysis. The exonuclease, by breaking down the linear probes and leaving the circular probes, will change the relative concentrations of the closed vs. the unclosed probes by a factor of 1000 or more. The probes that remain are then opened at a cleavage site by another enzyme, removed from the DNA, and amplified by PCR. Each probe is tagged with a different tag sequence consisting of 20 base tags (16,000 have been generated), and can be detected, for example, by the Affymetrix GenFlex Tag Array. The presence of the tagged probe from a reaction in which a particular gap-fill enzyme was added indicates the presence of the complimentary amino acid on the relevant SNP.

The molecular biological advantages of MIPS include: (1) multiplexed genotyping in a single reaction, (2) the genotype "call" occurs by gap fill and ligation, not hybridization, and (3) hybridization to an array of universal tags decreases false positives inherent to most array hybridizations. In traditional 500K, TaqMan and other genotyping arrays, the entire genomic sample is hybridized to the array, which contains a variety of perfect match and mismatch probes, and an algorithm calls likely genotypes based on the intensities of the mismatch and perfect match probes. Hybridization, however, is inherently noisy, because of the complexities of the DNA sample and the huge number of probes on the arrays. MIPs, on the other hand, uses mutliplex probes (i.e., *not* on an array) that are longer and therefore more specific, and then uses a robust ligation step to circularize the probe. Background is exceedingly low in this assay (due to specificity), though allele dropout may be high (due to poor performing probes).

When this technique is used on genomic data from a single cell (or small numbers of cells) it will - like PCR based approaches - suffer from integrity issues. For example, the inability of the padlock probe to hybridize to the genomic DNA will cause allele dropouts. This will be exacerbated in the context of in-vitro fertilization since the efficiency of the hybridization reaction is low, and it needs to proceed relatively quickly in order to genotype the embryo in a limited time period. Note that the hybridization reaction can be reduced well below vendor-recommended levels, and micro-fluidic techniques may also be used to accelerate the hybridization reaction. These approaches to reducing the time for the hybridization reaction will result in reduced data quality.

*Predictive Genomics*

Once the genetic data has been measured, the next step is to use the data for predictive purposes. Much research has been done in predictive genomics, which tries to understand the precise functions of proteins, RNA and DNA so that phenotypic predictions can be made based on genotype. Canonical techniques focus on the function of Single-Nucleotide Polymorphisms (SNP); but more advanced methods are being brought to bear on multi-factorial phenotypic features. These methods include techniques, such as linear regression and nonlinear neural networks, which attempt to determine a mathematical relationship between a set of genetic and phenotypic predictors and a set of measured outcomes. There is also a set of regression analysis techniques, such as Ridge regression, log regression and stepwise selection, that are designed to accommodate sparse data sets where there are many potential predictors relative to the number of outcomes, as is typical of genetic data, and which apply additional constraints on the regression parameters so that a meaningful set of parameters can be resolved even when the data is underdetermined. Other techniques apply principal component analysis to extract information from undetermined data sets. Other techniques, such as decision trees and contingency tables, use strategies for subdividing subjects based on their independent variables in order to place subjects in categories or bins for which the phenotypic outcomes are similar. A recent technique, termed logical regression, describes a method to search for different logical interrelationships between categorical independent variables in order to model a variable that depends on interactions between multiple independent variables related to genetic data. Regardless of the method used, the quality of the prediction is naturally highly dependant on the quality of the genetic data used to make the prediction.

The cost of DNA sequencing is dropping rapidly, and in the near future individual genomic sequencing for personal benefit will become more common. Knowledge of personal genetic data will allow for extensive phenotypic predictions to be made for the individual. In order to make accurate phenotypic predictions high quality genetic data is critical, whatever the context. In the case of prenatal or pre-implantation genetic diagnoses a complicating factor is the relative paucity of genetic material available. Given the inherently noisy nature of the measured genetic data in cases where limited genetic material is used for genotyping, there is a great need for a method which can increase the fidelity of, or clean, the primary data.

The current methods by which clinical decisions are made do not make the best possible use of existing information. As medical, biochemical and information technology advance, increasing amounts of data are generated and stored both for individual patients, and also in the context of academic and clinical studies. With the recent upsurge in the amounts of genetic, phenotypic and clinical information available for analysis, much effort has gone into finding clinically relevant correlations to help people lead longer, healthier and more enjoyable lives. Whereas previously clinicians and researchers would concentrate their analysis on a handful of obvious potential factors and use a local store of data, it is becoming clear the potential benefit of being able to leverage data measured by scores of other agents, and using more complex models that can identify previously unsuspected factors which correlate with a given genotype or phenotype. This situation will become considerably more complicated once personal genetic data occupies a more central role in understanding the causes and treatments of diseases and other predispositions of subjects. Within the next decade it may be possible to scan the entire genome of a patient as well as to collect a myriad of phenotypic data points, either for clinical trials, or for the purpose of personalized treatments and or drug assignment.

As the amount of data available has become enormous, and is still increasing rapidly, the crux of the problem has become designing and implementing good methods that allow the most appropriate correlations to be uncovered and used to benefit people. As the number of variables available to analyze has increased, it has become more important to develop methods that are able to digest the astronomical number of potential correlations, and do not a-priori rule any of them out. At the same time it is important to develop methods that can integrate and utilize the findings of multiple studies, even when those studies were not conducted with identical protocols. It is also becoming increasingly important, given the large number of prediction models which have been studied, to develop systems that can correctly identify the optimal method to use in a given analysis.

*Bioinformatics in the Context of HIV*

HIV is considered pandemic in humans with more than 30 million people currently living with HIV, and more than 2 million deaths each year attributable to HIV. One of the major characteristics of HIV is its high genetic variability as a result of its fast replication cycle and the high error rate and recombinogenic properties of reverse transcriptase. As a result, various strains of the HIV virus show differing levels of resistance to different drugs, and an optimal treatment regimen may take into account the identity of the infective strain and its particular susceptibilities.

As of today, approved ART drugs consist of a list of eleven RTIs: seven nucleoside, one nucleotide and three non-nucleoside; seven PIs; and one fusion/entry inhibitor. Given the current rollout of ART drugs around the world the appearance of resistance strains of the virus is inevitable, both due to the low genetic barrier to resistance and to poor drug adherence. Consequently, techniques to predict how mutated viruses will respond to anti-retroviral therapy are increasingly important as they will influence the outcome for salvage therapies. The rapidly decreasing cost of viral genetic sequencing - with volume pricing as low as $5 for pre-prepared sequences - makes the selection of drugs based on viral genetic sequence data an attractive option, rather than the more costly and involved in-vitro phenotype measurement. The use of sequence data, however, necessitates accurate predictions of viral drug response, based on the appearance of viral genetic mutations. The many different combinations of viral mutations make it difficult to design a model that includes all the genetic cofactors and their interactions, and to train the model with limited data. The latter problem is exacerbated in the context of modeling in-vivo drug response, where the many different combinations of drug regimens make it difficult to collect sufficiently large data sets for any particular regimen that contain the variables, namely baseline clinical status, treatment history, clinical outcome and genetic sequence.

Resistance to antiviral drugs can be the result of one mutation within the RT or protease sequences, or the combination of multiple mutations. The RT enzyme is coded by a key set of 560 codons; the protease enzyme by 99 codons. By considering only mutations that alter the amino acids, each amino acid locus has 19 possible mutations; so there are a total of 10,640 possible mutations that differ from wild type on the RT enzyme, and 1,981 possible mutations on the protease enzyme. Using a simple linear model, where each mutation encountered in the data (not all mutations will occur) is associated with a particular weighting, or linear regression parameter, several thousand parameters may exist. If only several hundred patient samples are available for each drug, the problem is overcomplete, or ill-posed in the Hadamard sense, since there are more parameters to estimate than independent equations. Many techniques exist that can be applied to the problem of constructing models for the ill-posed problem. These include combining a-priori expert knowledge with observations to create expert-rule based systems, as well as statistical methods including i) ridge regression, ii) principal component analysis, iii) decision trees, iv) stepwise selection techniques, v) Neural Networks, vi) the Least Absolute Shrinkage and Selection Operator (LASSO), and vii) Support Vector Machines (SVM).

Three main industry-standard expert systems are typically used to predict the susceptibility of HIV viruses to ART drugs: the ANRS-AC11 System, the Rega System, and the Stanford HIVdb system. It is commonplace in the literature for new algorithms to be benchmarked against these expert systems. None of these expert systems, however, is designed to perform direct prediction of phenotypic response, but rather to provide a numeric score by which different drugs can be compared, or to classify the drugs into discrete groupings such as *Sensitive, Intermediate* and *Resistant.* In addition, it has been clearly established that statistical algorithms, such as linear regression models trained with stepwise selection, substantially outperform expert systems in prediction of phenotypic outcome. Consequently, only a set of statistical techniques are compared with the novel methods in the detailed description, which includes the best performing methods recently disclosed in the literature.

Current approaches to predicting clinical outcomes of salvage ART do not demonstrate good predictive power, largely due to a lack of statistically significant outcome data, combined with the many different permutations of drug regimens and genetic mutations. This field has a pressing need both for the integration of multiple heterogeneous data sets and the enhancement of drug response prediction.

*Bioinformatics in the Context of Cancer*

Of the estimated 80,000 annual clinical trials, 2,100 are for cancer drugs. Balancing the risks and benefits for cancer therapy represents a clinical vanguard for the combined use of phenotypic and genotypic information. Although there have been great advances in chemotherapy in the past few decades, oncologists still treat their cancer patients with primitive systemic drugs that are frequently as toxic to normal cells as to cancer cells. Thus, there is a fine line between the maximum toxic dose of chemo and the therapeutic dose. Moreover, dose-limiting toxicity may be more severe in some patients than others, shifting the therapeutic window higher or lower. For example, anthracyclines used for breast cancer treatment can cause adverse cardiovascular events. Currently, all patients are treated as though at risk for cardiovascular toxicity, though if a patient could be determined to be at low-risk for heart disease, the therapeutic window could be shifted to allow for a greater dose of anthracycline therapy.

To balance the benefits and risks of chemotherapy for each patient, one may predict the side effect profile and therapeutic effectiveness of pharmaceutical interventions. Cancer therapy often fails due to inadequate adjustment for unique host and tumor genotypes. Rarely does a single polymorphism cause significant variation in drug response; rather, manifold polymorphisms result in unique biomolecular compositions, making clinical outcome prediction difficult. "Pharmacogenetics" is broadly defined as the way in which genetic variations affect patient response to drugs. For example, natural variations in liver enzymes affect drug metabolism. The future of cancer chemotherapy is targeted pharmaceuticals, which require understanding cancer as a disease process encompassing multiple genetic, molecular, cellular, and biochemical abnormalities. With the advent of enzyme-specific drugs, care may be taken to insure that tumors express the molecular target specifically or at higher levels than normal tissues. Interactions between tumor cells and healthy cells may be considered, as a patient's normal cells and enzymes may limit exposure of the tumor drugs or make adverse events more likely.

Bioinformatics will revolutionize cancer treatment, allowing for tailored treatment to maximize benefits and minimize adverse events. Functional markers used to predict response may be analyzed by computer algorithms. Breast, colon, lung and prostate cancer are the four most common cancers. An example of two treatments for these cancers are tamoxifen, which is used to treat breast cancer, and irinotecan which is used in colon cancer patients. Neither tamoxifen or irinotecan are necessary or sufficient for treating breast or colon cancer, respectively. Cancer and cancer treatment are dynamic processes that require therapy revision, and frequently combination therapy, according to a patient's side effect profile and tumor response. If one imagines cancer treatment as a decision tree, to give or withhold any one treatment before, after, or with other therapies, then this tree comprises a subset of decision nodes, where much of the tree (i.e. other treatments) can be considered a black box. Nonetheless, having data to partially guide a physician to the most effective treatment is advantageous, and as more data is gathered, an effective method for making treatment decisions based on this data could significantly improve life expectancies and quality of living in thousands of cancer patients.

The colon, or large intestine, is the terminal 6-foot section of the gastrointestinal (GI) tract. The American Cancer Society estimates that 145,000 cases of colorectal cancer will be diagnosed in 2005, and 56,000 will die as a result. Colorectal cancers are assessed for grade, or cellular abnormalities, and stage, which is subcategorized into tumor size, lymph node involvement, and presence or absence of distant metastases. 95% of colorectal cancers are adenocarcinomas that develop from genetically-mutant epithelial cells lining the lumen of the colon. In 80-90% of cases, surgery alone is the standard of care, but the presence of metastases calls for chemotherapy. One of many first-line treatments for metastatic colorectal cancer is a regimen of 5-fluorouracil, leucovorin, and irinotecan.

Irinotecan is a camptothecin analogue that inhibits topoisomerase, which untangles super-coiled DNA to allow DNA replication to proceed in mitotic cells, and sensitizes cells to apoptosis. Irinotecan does not have a defined role in a biological pathway, so clinical outcomes are difficult to predict. Dose-limiting toxicity includes severe (Grade III-IV) diarrhea and myelosuppression, both of which require immediate medical attention. Irinotecan is metabolized by uridine diphosphate glucuronosyl-transferase isoform 1a1 (UGT1A1) to an active metabolite, SN-38. Polymorphisms in UGT1A1 are correlated with severity of GI and bone marrow side effects.

*Prior Art*

Listed here is a set of prior art which is related to the field of the current invention. None of this prior art contains or in any way refers to the novel elements of the current invention. In US Patent 6,720,140, Hartley et al describe a recombinational cloning method for moving or exchanging segments of DNA molecules using engineered recombination sites and recombination proteins. In US Patent 6,489,135 Parrott et al. provide methods for determining various biological characteristics of in vitro fertilized embryos, including overall embryo health, implantability, and increased likelihood of developing successfully to term by analyzing media specimens of in vitro fertilization cultures for levels of bioactive lipids in order to determine these characteristics. In US Patent Application 20040033596 Threadgill et al. describe a method for preparing homozygous cellular libraries useful for in vitro phenotyping and gene mapping involving site-specific mitotic recombination in a plurality of isolated parent cells. In US Patent 5,994,148 Stewart et al. describe a method of determining the probability of an in vitro fertilization (IVF) being successful by measuring Relaxin directly in the serum or indirectly by culturing granulosa lutein cells extracted from the patient as part of an IVF/ET procedure. In US Patent application 5,635,366 Cooke et al. provide a method for predicting the outcome of IVF by determining the level of 11□-hydroxy steroid dehydrogenase in a biological sample from a female patient. In U.S. Patent No. 7,058,616 Larder et al. describe a method for using a neural network to predict the resistance of a disease to a therapeutic agent. In U.S. Patent No. 6,958,211 Vingerhoets et al. describe a method wherein the integrase genotype of a given HIV strain is simply compared to a known database of HIV integrase genotype with associated phenotypes to find a matching genotype. In U.S. Patent 7,058,517 Denton et al. describe a method wherein an individual's haplotypes are compared to a known database of haplotypes in the general population to predict clinical response to a treatment. In U.S. Patent 7,035,739 Schadt at al. describe a method is described wherein a genetic marker map is constructed and the individual genes and traits are analyzed to give a gene-trait locus data, which are then clustered as a way to identify genetically interacting pathways, which are validated using multivariate analysis. In U.S. Patent No. 6,025,128 Veltri et al. describe a method involving the use of a neural network utilizing a collection of biomarkers as parameters to evaluate risk of prostate cancer recurrence. In U.S. Patent No. 5,824,467 Mascarenhas describes a method to predict drug responsiveness by establishing a biochemical profile for patients and measuring responsiveness in members of the test cohort, and then individually testing the parameters of the patients' biochemical profile to find correlations with the measures of drug responsiveness. US2004137470 provides a method useful for detection of genetic disorders. The method comprises determining the sequence of alleles of a locus of interest, and quantitating a ratio for the alleles at the locus of interest, wherein the ratio indicates the presence or absence of a chromosomal abnormality.

SUMMARY

The invention is as defined in claims 1-7.

The system disclosed enables the cleaning of incomplete or noisy genetic data using secondary genetic data as a source of information, and also using that genetic data to make phenotypic and clinical predictions. While the disclosure focuses on genetic data from human subjects, it should be noted that the methods disclosed apply to the genetic data of a range of organisms, in a range of contexts. The techniques described for cleaning genetic data are most relevant in the context of pre-implantation diagnosis during in-vitro fertilization, prenatal diagnosis in conjunction with amniocentesis, chorion villus biopsy, and fetal blood sampling, and non-invasive prenatal diagnosis, where a small quantity of fetal genetic material is isolated from maternal blood. The diagnoses may focus on inheritable diseases, increased likelihoods of defects or abnormalities, as well as making phenotype predictions for individuals to enhance clinical and lifestyle decisions. The invention addresses the shortcomings of prior art that are discussed above. The techniques described here for making phenotypic and clinical predictions are relevant in multiple contexts, including in the context of pre-implantation diagnosis, prenatal diagnosis, and also in the context of individuals with medical conditions, or susceptibilities. Certain embodiments of the technology disclosed herein describe a system for making accurate predictions of phenotypic outcomes or phenotype susceptibilities for an individual given a set of genetic, phenotypic and or clinical information for the individual. In one aspect of the disclosure, a technique for building linear and nonlinear regression models that can predict phenotype accurately when there are many potential predictors compared to the number of measured outcomes, as is typical of genetic data, is disclosed; the models may be based on contingency tables and built from information available in the public domain. A system is described wherein a number of models are trained on a relevant dataset, and that model which is most accurate in making the relevant prediction is used.

The disclosed methods make use of imperfect knowledge of the genetic data of the mother and the father, together with the knowledge of the mechanism of meiosis and the imperfect measurement of the embryonic DNA, in order to reconstruct, *in silico,* the embryonic DNA at the location of key SNPs with a high degree of confidence. It is important to note that the parental data allows the reconstruction not only of SNPs that were measured poorly, but also of insertions, deletions, and of SNPs or whole regions of DNA that were not measured at all.

The disclosed method is applicable in the context of in-vitro fertilization, where a very small number of blastomeres are available for genotyping from each embryo being considered for implantation. The disclosed method is equally applicable to the context of Non-Invasive Prenatal Diagnosis (NIPD) where only a small number of fetal cells, or fragments of fetal DNA, have been isolated from the mother's blood. The disclosed method is equally applicable in the case of amniocentesis, and other methods where fetal blood is sampled directly. The disclosed method is more generally applicable in any case where a limited amount of genetic data is available from the target individual, and additional genetic data is available from individuals who are genetically related to the target.

The fetal or embryonic genomic data which has been reconstructed can be used to detect if the cell is aneuploid, that is, if fewer or more than two of a particular chromosome is present in a cell. A common example of this condition is trisomy-21, which gives rise to Down syndrome. The reconstructed data can also be used to detect for uniparental disomy, a condition in which two of a given chromosome are present, and both of which originate from one parent. This is done by creating a set of hypotheses about the potential states of the DNA, and testing to see which one has the highest probability of being true given the measured data. Note that the use of high throughput genotyping data for screening aneuploidy enables a single blastomere from each embryo to be used both to measure multiple disease-linked loci as well as screen for chromosomal abnormalities.

The direct measurements of the amount of genetic material, amplified or unamplified, present at a plurality of loci, can be used to detect for aneuploides, or uniparental disomy. The idea behind this method is simply that the amount of genetic material present during amplification is proportional to the amount of genetic information in the initial sample, and measuring these levels at multiple loci will give a statistically significant result. This method of screening for chromosomal abnormalities can be used in conjunction with the related method described herein for cleaning genetic data.

The disclosed method can clean genetic material of the individual which has been contaminated by foreign DNA or RNA by identifying the data generated by extraneous genetic materials. The spurious signals generated by the contaminating DNA can be recognized in a manner similar to that way that chromosome-wide anomalous signals generated by aneuploides can be detected.

Target cells may be isolated, the genetic data contained in those cells are amplified, and measurements of multiple SNPs are made using a combination of one or more of the following techniques: PCR-based amplification techniques, PCR-based measurement techniques, or detection techniques based on Molecular Inversion Probes, or microarrays such as the GeneChip or TaqMan systems. This genetic data is then used in the system described herein.

The genetic data of an individual can be cleaned using diploid and haploid data from both parents. Alternately, haploid data from a parent can be simulated if diploid and haploid data of the parent's parent can be measured. In another aspect of the disclosure, genetic data from any person of a known genetic relation to the individual can be used to clean the data of the individual, including parents, siblings, grandparents, offspring, cousins, uncles, aunts etc.

The target and/or related individual's genetic data may be partly or wholly known *in silico,* obviating the need for some direct measurements. Portions of the genetic data can be generated *in silico* by means of an informatics approach utilizing a hidden Markov model.

It is possible to estimate the confidence one has in the determination of those SNPs.

Note that the techniques described herein are relevant both to measurements of genetic material in one, or a small number of cells, as well as to measurements on smaller amounts of DNA such as that which can be isolated from the mother's blood in the context of Non-invasive Prenatal Diagnosis (NIPD). Also note that this method can be equally applied to genomic data *in silico,* i.e. not directly measured from genetic material.

A technique for creating models based on contingency tables that can be constructed from data that is available through publications such as through the OMIM (Online Mendelian Inheritance in Man) database and using data that is available through the HapMap project and other aspects of the human genome project is provided. Certain embodiments of this technique use emerging public data about the association between genes and about association between genes and diseases in order to improve the predictive accuracy of models.

In yet another aspect of the disclosure, a technique by which the best model can be found for the data that is available for a particular patient is disclosed. In this aspect, many different combinations of variables may be examined, together with many different modeling techniques, and that combination may be chosen which will produce the best prediction for an individual subject based on cross-validation with testing data from other subjects.

In some cases the models that may produce the best at making accurate predictions of phenotypic outcomes or phenotype susceptibilities for an individual are trained using convex optimization techniques to perform continuous subset selection of predictors so that one is guaranteed to find the globally optimal parameters for a particular set of data. This feature is particularly advantageous when the model may be complex and may contain many potential predictors such as genetic mutations or gene expression levels. Furthermore, in some examples convex optimization techniques may be used to make the models sparse so that they explain the data in a simple way. This feature enables the trained models to generalize accurately even when the number of potential predictors in the model is large compared to the number of measured outcomes in the training data. Similar techniques have been published in an academic journal (Rabinowitz, M., et al., 2006, "Accurate prediction of HIV-1 drug response from the reverse transcriptase and protease amino acid sequences using sparse models created by convex optimization." Bioinformatics 22(5): 541-9.). Note that the information from this paper has been included in this document for background and context.

While certain illustrative embodiments disclosed herein focus on genetic data from human subjects, and provide specific embodiments for people suffering from cancer or HIV, or for people who seek to understand their susceptibility to diseases such as Alzheimer's or Myocardial Infarction, it should be noted that the methods disclosed apply to the genetic data of a range of organisms, in a range of numerous, different contexts. The techniques described herein for phenotypic prediction and drug response prediction may be relevant in the context of the treatment of a variety of cancers, genetic illnesses, bacterial, fungal or viral infections, as well as in making phenotypic predictions for individuals to enhance clinical and lifestyle decisions. Furthermore, the system can be used to determine the likelihood of particular phenotypic outcomes given genetic data, specifically SNP (single nucleotide polymorphism) data of an embryo (pre-implantation) in the context of IVF, or of a fetus in the context of non-invasive or invasive prenatal diagnosis including amniocentesis.

In one embodiment, the predictive models may be applied to genetic data for a particular individual that has been stored in a standardized computable format. The individual may describe particular issues that are relevant to them, or the system may automatically determine which phenotypic susceptibilities are relevant to that individual. As new research data becomes available on disease-gene associations, treatments, or lifestyle habits, the individual can be notified of the impact of this information on their decisions and habits, based on predictive models developed from the aggregated genomic and clinical data. Alternately, the system can use new research data to detect hitherto unsuspected risks to the individual and that individual can be notified of the impact of this information.

In another embodiment, enhanced reports can be generated for clinicians using outcome prediction models trained on data integrated from databases of genetic data, phenotypic data, and clinical records including relevant diagnostic tests. This system may provide for the creation of enhanced reports for individuals with diseases and/or disease predispositions, including but not limited to HIV, cancer, Alzheimers and heart diseases. These enhanced reports will indicate to a treating physician which disease-management or preventative treatments may be more or less suitable for a given individual. The report will include predictions and confidence bounds for key outcomes for that individual using models trained on aggregated subject data.

According to another embodiment, a system and method where data about a specific individual is used to make predictions about said individual using models based on contingency tables and built from information available in the public domain, where said data is taken from a group consisting of said individual's genetic data, said individual's phenotypic data, said individual's clinical data, and combinations thereof, and where said predictions concern topics taken from a group comprising said individual's phenotypes, phenotype susceptibilities, and possible clinical outcomes, and where said information is taken from a group comprising information about genotype-phenotype associations, information about the frequency of certain genetic alleles, information about the frequency of certain associations among genetic alleles, information about the probability of one or more states of certain phenotypes given certain combinations of genetic alleles, information about the probability of a certain combinations of genetic alleles given the state of a certain phenotypes, and combinations thereof is disclosed.

According to yet another embodiments, a system and method whereby data about a specific individual can be used to make predictions about said individual using a variety of mathematical models trained on aggregated data in a way that the model which shows the best accuracy can be utilized, where said individual's data is taken from a group consisting of said individual's genetic data, said individual's phenotypic data, and said individual's clinical data, and where said predictions concern topics taken from a group comprising said individual's phenotypes, phenotype susceptibilities, possible clinical outcomes, and combinations thereof is provided. In certain embodiments, the method may examine many or all of the different independent variable and dependant variable combinations in a given set of data, using multiple models and multiple tuning parameters, and then selects that combination of independent variables and dependant variables, that model and those tuning parameters that achieved the highest correlation coefficient with the test data for the purpose of making the best phenotypic predictions.

According to another embodiment, any of the methods disclosed herein may use predictions to generate reports for a specific individual concerning one or more topics that are relevant to said individual, where said topics are taken from a group comprising lifestyle decisions, dietary habits, hormonal supplements, possible treatment regimens for a disease, possible treatment regimens for a pathogen, drug interventions, and combinations thereof, and where said prediction is based on data concerning said individual's genetic makeup, said individual's phenotypic characteristics, said individual's clinical history, and combinations thereof.

According to other embodiments, any of the methods disclosed herein may use predictions to generate reports for an agent of a specific individual, such as a physician or clinician, and where said predictions could aid said agent by providing information relevant to said individual, and where the subject of said information is taken from a group of topics comprising lifestyle decisions, dietary habits, hormonal supplements, possible treatment regimens for a disease, possible treatment regimens for a pathogen, drug interventions, other therapeutic interventions, and combinations thereof, and where said prediction is based on data concerning said individual's genetic makeup, said individual's phenotypic characteristics, said individual's clinical history, and combinations thereof.

According to another embodiment, any of the methods disclosed herein may use predictions to benefit a specific individual afflicted with cancer, and where said predictions could aid clinicians by providing information relevant to that individual and or to the specific cancer of said individual, and where the subject of said information is taken from a group of topics comprising treatment regimens, lifestyle decisions, and dietary habits, drug interventions, other therapeutic interventions, and combinations thereof, and where said prediction is based on data concerning said individual's genetic makeup, said individual's phenotypic characteristics, said individual's clinical history, and combinations thereof.

According to one embodiment, any of the methods disclosed herein may be used to benefit a specific individual afflicted with a pathogen, and where said predictions could aid clinicians by providing information relevant to that individual and or to the specific pathogen infecting said individual, where said pathogen is of a class taken from a group consisting of bacteria, virus, microbe, amoeba, fungus and other parasites, and where the subject of said information is taken from a group of topics comprising treatment regimens, lifestyle decisions, and dietary habits drug interventions, other therapeutic interventions, and combinations thereof, and where said prediction is based on data concerning said individual's genetic makeup, said individual's phenotypic characteristics, said individual's clinical history, and combinations thereof.

According to another embodiment, any of the methods disclosed herein may use predictions regarding a specific individual, new knowledge and data as that knowledge and data becomes available, and which could be used to generate informational reports, automatically or on-demand, regarding topics that are relevant to said individual, where the topics are taken from a group comprising lifestyle decisions, dietary habits, hormonal supplements, possible treatment regimens for a disease, possible treatment regimens for a pathogen, drug interventions, other therapeutic interventions, and combinations thereof, and where the new knowledge and data are medical in nature, and where the prediction is based on data concerning said individual's genetic makeup, said individual's phenotypic characteristics, said individual's clinical history, and combinations thereof.

According to another embodiment, any of the methods disclosed herein may use predictions using genetic data from a specific embryo and said predictions can be used to aid in selection of embryos in the context of IVF based on predicted susceptibility to certain phenotypes of said embryo.

According to one embodiment, any of the methods disclosed herein may use predictions using genetic data from a specific fetus, and said predictions can be used to estimate particular phenotypic outcomes for the potential progeny, such as life expectancy, the probability of psoriasis, or the probability of a particular level of mathematical ability.

It will be recognized by the person of ordinary skill in the art, given the benefit of this disclosure, that other aspects, features and embodiments may implement one or more of the methods and systems disclosed herein.

BRIEF DESCRIPTION OF THE DRAWINGS

Figure 1: an illustration of the concept of recombination in meiosis for gamete formation.
Figure 2: an illustration of the variable rates of recombination along one region of Human Chromosome 1.
Figure 3: determining probability of false negatives and false positives for different hypotheses.
Figure 4: the results from a mixed female sample, all loci hetero.
Figure 5: the results from a mixed male sample, all loci hetero.
Figure 6: Ct measurements for male sample differenced from Ct measurements for female sample.
Figure 7: the results from a mixed female sample; Taqman single dye.
Figure 8: the results from a mixed male; Taqman single dye.
Figure 9: the distribution of repeated measurements for mixed male sample.
Figure 10: the results from a mixed female sample; qPCR measures.
Figure 11: the results from a mixed male sample; qPCR measures.
Figure 12: Ct measurements for male sample differenced from Ct measurements for female sample.
Figure 13: detecting aneuploidy with a third dissimilar chromosome..
Figure 14: an illustration of two amplification distributions with constant allele dropout rate.
Figure 15: a graph of the Gaussian probability density function of alpha.
Figure 16: the general relationship diagram of the input data, the database data, the algorithm and the output.
Figure 17: a visual overview of how to derive P(H|M).
Figure 18: a visual representation of the flow chart describing the algorithm used to demonstrate the effectiveness of the cleaning algorithm on simulated data.
Figure 19: an illustration of a system that is configured to accomplish the method disclosed herein, in the context of phenotype prediction of embryos during IVF.
Figure 20: an illustration of the LASSO tendency to produce sparse solutions. The Ridge regression solution lies at the meeting of the two circles, and the LASSO solution lies at the meeting of the circle and square.
Figure 21: a table of the correlation coefficients (R in %) of measured and predicted response, averaged over ten different 9:1 splits of training and testing data, and then averaged over seven PIs or ten RTIs respectively.

Figure 22: graphic representation of the value of LASSO model parameters associated with mutations in the protease enzyme for predicting PI response. Only 40 parameters with the largest absolute magnitudes are shown.

Figure 23: graphic representation of the value of LASSO model parameters associated with mutations in the RT enzyme for predicting NRTI drug response. Only the 40 parameters with the largest absolute magnitudes are shown.

Figure 24: graphic representation the value of LASSO model parameters associated with mutations in the RT enzyme for predicting NNRTI drug response. Only the 40 parameters with the largest absolute magnitudes are shown.

Table 1: a summary of disease genes as found in OMIM/NCBI.

Table 2: a summary of different aneuploidy detection techniques

Table 3: an example of input data for the method described using SNPs with a low degree of cosegregation.

Table 4: an example of input data for the method described using SNPs with a high degree of cosegregation.

Table 5: an example of the output data for the input data shown in Table 2.

Table 6: an example of the output data for the input data shown in Table 4.

Table 7: the results of the preliminary simulation.

Table 8: the results of the full simulation of the method.

Table 9: three contingency tables representing the results of Farrer (2005), Labert (1998), and Alvarez (1999) for understanding the role of mutations in APOE and ACE in affecting the onset of Alzheimers.

Table 10: results generated from meta-analysis of the studies of Table 7.

Table 11: a table of correlation coefficients (R in %) of measured and predicted response to Protease Inhibitor (PI) drugs for various methods, averaged over ten different 9:1 splits of training and testing data. The standard deviation (Std. dev.) of the results is shown in gray; the number of measured drug responses is shown in the last row.

Table 12: a table of correlation coefficients (R in %) of measured and predicted response to Reverse Transcriptase Inhibitor (RTI) drugs for various methods, averaged over ten different 9:1 splits of training and testing data. The standard deviation (Std. dev.) of the results is shown in gray; the number of measured drug responses is shown in the last row.

Table 13: the number of samples, and total number of mutations used for training for various regression methods, together with the number of mutations with non-zero weights selected by the Least Absolute Selection and Shrinkage Operator (LASSO) as predictors for Protease Inhibitor (PI) drug response.

Table 14: the number of samples, and total number of mutations used for training with various methods, together with the number of mutations with non-zero weights selected by LASSO as predictors for Reverse Transcriptase Inhibitor (RTI) response.

Table 15: phenotypic data for the irinotecan study.

## DETAILED DESCRIPTION OF THE PREFERRED EMBODIMENT

### Conceptual Overview of the System

One goal of the disclosed system is to provide highly accurate genomic data for the purpose of genetic diagnoses. In cases where the genetic data of an individual contains a significant amount of noise, or errors, the disclosed system makes use of the similarities between genetic data of related individuals, and the information contained in that secondary genetic data, to clean the noise in the target genome. This is done by determining which segments of chromosomes were involved in gamete formation and where crossovers occurred during meiosis, and therefore which segments of the secondary genomes are expected to be nearly identical to sections of the target genome. In certain situations this method can be used to clean noisy base pair measurements, but it also can be used to infer the identity of individual base pairs or whole regions of DNA that were not measured. In addition, a confidence can be computed for each reconstruction call made. A highly simplified explanation is presented first, making unrealistic assumptions. A detailed statistical approach that can be applied to the technology of today is presented afterward.

Another goal of the system is to detect abnormal numbers of chromosomes, sections of chromosomes, and origins of chromosomes. In genetic samples that are aneuploid, have unbalanced translocations, uniparental disomy, or other gross chromosomal abnormalities, the amount of genetic material present at a plurality of loci can be used to determine the chromosomal state of the sample. There are multiple approaches to this method, and several of them are described here. In some approaches, the amount of genetic material present in a sample is sufficient to directly detect aneuploides. In other approaches, the method for cleaning the genetic material can be used to enhance the efficiency of detection of chromosomal imbalances. A confidence can be computed for each chromosomal call made.

Another goal of the system is to provide an effective and efficient means of extracting the most simple and intelligible statistical models from from genetic data by exploring a wide array of terms that are designed to model the effect of variables related to genetic data. More specifically, most or all of the currently available methods for modeling phenotype or phenotype susceptibility based on genetic data have the following drawbacks: (i) they do not use convex optimization techniques and thus are not guaranteed to find the local minimum solution for the model parameters for a given training data set; (ii) they do not use techniques that minimize the complexity of the model and thus they do not build models that generalize well when there are a small number of outcomes relative to the number of independent variables; (iii) they do not enable the extraction of the most simple intelligible rules from the data in the context of logistic regression without making the simplifying assumption of normally distributed data; (iv) they do not leverage a-priori information about gene-gene associations, gene-phenotype associations, and gene-disease associations in order to make the best possible prediction of phenotype or phenotype susceptibility; (v) they do not provide more than one model, and thus do not provide a general approach for selecting the best possible data based on cross-validating various models against training data. These shortcomings are critical in the context of predicting outcomes based on the analysis of vast amounts of data classes relating to genetic and phenotypic information. In summary the currently available methods do not effectively empower individuals to ask questions about the likelihood of particular phenotypic features given genotype, or about the likelihood of particular phenotypic features in an offspring given the genotypic features of the parents.

Note that some of the explanation given below includes work that has been previously published by authors of this document. It is provided as background information to facilitate understanding of and to give a greater context to the material disclosed herein.

One may consider genotype-phenotype predictive models in three categories: i) genetic defects or alleles are known to cause the disease phenotype with 100% certainty; ii) genetic defects and alleles that increase the probability of disease phenotype, where the number of predictors is small enough that the phenotype probability can be modeled with a contingency table; and iii) complex combinations of genetic markers that can be used to predict phenotype using multidimensional linear or nonlinear regression models. Of the 359 genes (See Table 1, row 2) with currently known sequences and disease phenotypes in the Online Mendelian Inheritance Database (OMIM), the majority fall into category (i); and the remainder fall predominantly into category (ii). However, over time, it is expected that multiple genotype-phenotype models will arise in category (iii), where the interaction of multiple alleles or mutations will need to be modeled in order to estimate the probability of a particular phenotype. For example, scenario (iii) is certainly the case today in the context of predicting the response of HIV viruses to anti-retroviral therapy based on the genetic data of the HIV virus.

For scenario (i), it is usually straightforward to predict the occurrence of the phenotype based on expert rules. In one aspect, statistical techniques are described that can be used to make accurate predictions of phenotype for scenarios (ii). In another aspect, statistical techniques are described that can be used to make accurate predictions for scenario (iii). In another aspect, methods are described by which the best model can be selected for a particular phenotype, a particular set of aggregated data, and a particular individual's data.

Certain embodiments of the methods disclosed herein implement contingency tables to accurately make predictions in scenario (ii). These techniques leverage a-priori information about gene-gene associations and gene-disease associations in order to improve the prediction of phenotype or phenotype susceptibility. These techniques enable one to leverage data from previous studies in which not all the relevant independent variables were sampled. Instead of discarding these previous results because they have missing data, the technique leverages data from the HapMap project and elsewhere to make use of the previous studies in which only a subset of the relevant independent variables were measured. In this way, a predictive model can be trained based on all the aggregated data, rather than simply that aggregated data from subjects for which all the relevant independent variables were measured.

Certain methods described herein use convex optimization to create sparse models that can be used to make accurate predictions in scenario (iii). Genotype-phenotype modeling problems are often overcomplete, or ill-posed, since the number of potential predictors - genes, proteins, mutations and their interactions - is large relative to the number of measured outcomes. Such data sets can still be used to train sparse parameter models that generalize accurately, by exerting a principle similar to Occam's Razor: When many possible theories can explain the observations, the most simple is most likely to be correct. This philosophy is embodied in one aspect relating to building genotype-phenotype models in scenario (iii) discussed above. The techniques described here for application to genetic data involve generating sparse parameter models for underdetermined or ill-conditioned genotype-phenotype data sets. The selection of a sparse parameter set exerts a principle similar to Occam's Razor and consequently enables accurate models to be developed even when the number of potential predictors is large relative to the number of measured outcomes. In addition, certain embodiments of the techniques described here for building genotype-phenotype models in scenario (iii) use convex optimization techniques which are guaranteed to find the global minimum solution for the model parameters for a given training data set.

Given a set of aggregated data, and a set of available data for an individual, it is seldom clear which prediction approach is most appropriate for making the best phenotypic prediction for that individual. In addition to describing a set of methods that tend to make accurate phenotypic predictions, embodiments disclosed herein present a system that tests multiple methods and selects the optimal method for a given phenotypic prediction, a given set of aggregated data, and a given set of available data for the individual for whom the prediction is to be made. The disclosed methods and systems examine all the different independent variable and dependant variable combinations in a given set of data using multiple models and multiple tuning parameters, and then selects that combination of independent variables, dependant variables, and those tuning parameters that achieve the best modeling accuracy as measured with test data. In cases corresponding to scenario (i) expert rules may be drawn; in other cases with few independent variables, such as in category (ii), contingency tables will provide the best phenotype prediction; and in other cases such as scenario (iii) linear or non-linear regression techniques may be used to provide the optimal method of prediction. Note that it will be clear to one skilled in the art, after reading this disclosure, how the approach to selecting the best model to make a prediction for an individual may be used to select from amongst many modeling techniques beyond those disclosed here.

Certain embodiments of the technology are demonstrated in several contexts. First, it is demonstrated in the context of predicting the likelihood of developing Alzheimer's disease using contingency tables and an incomplete set of data integrated from many clinical studies focusing on predicting Alzheimer's disease based on genetic markers. Next, the system is demonstrated in the context of modeling the drug response of Type-1 Human Immunodeficiency Virus (HIV-1) using regression analysis and the knowledge of genetic markers in the viral genome. Finally the system is demonstrated in the context of predicting the side-effects caused by the usage of tamoxifen and irinotecan in the treatment of various cases of breast and colon cancer, respectively, using regression analysis and the incomplete data of both genetic markers on the individuals and also laboratory and clinical subject information relevant to the cancer.

Due to the decreasing expense of genotypic testing, statistical models that reliably predicts viral drug response, cancer drug response, and other phenotypic responses or outcomes from genetic data are important tools in the selection of appropriate courses of action whether they be disease treatments, lifestyle or habit decisions, or other actions. The optimization techniques described will have application to many genotype-phenotype modeling problems for the purpose of enhancing clinical decisions.

Technical Description of the System

*Cleaning Data: A Simplified Example*

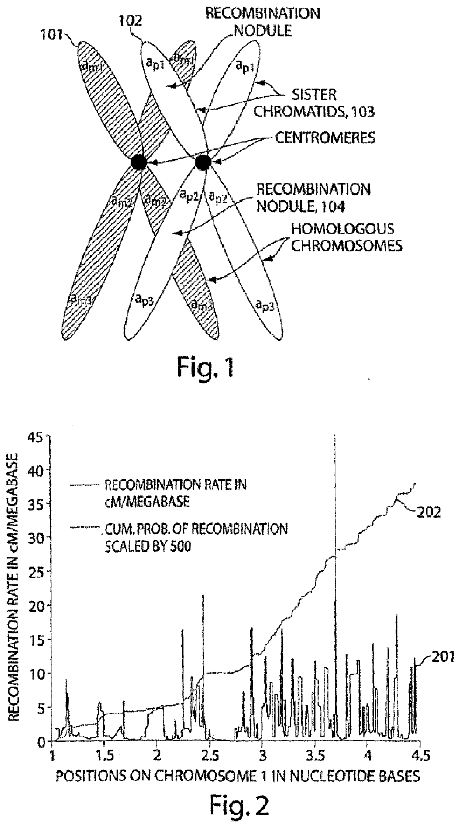

Figure 1 illustrates the process of recombination that occurs during meiosis for the formation of gametes in a parent. The chromosome 101 from the individual's mother is shown in orange (or grey). The chromosome 102 from the individual's father is shown in white. During this interval, known as Diplotene, during Prophase I of Meiosis, a tetrad of four chromatids 103 is visible. Crossing over between non-sister chromatids of a homologous pair occurs at the points known as recombination nodules 104. For the purpose of illustration, the example will focus on a single chromosome, and three Single Nucleotide Polymorphisms (SNPs), which are assumed to characterize the alleles of three genes. For this discussion it is assumed that the SNPs may be measured separately on the maternal and paternal chromosomes. This concept can be applied to many SNPs, many alleles characterized by multiple SNPs, many chromosomes, and to the current genotyping technology where the maternal and paternal chromosomes cannot be individually isolated before genotyping.

Attention must be paid to the points of potential crossing over in between the SNPs of interest. The set of alleles of the three maternal genes may be described as $(a_{m1}, a_{m2}, a_{m3})$ corresponding to SNPs $(SNP_1, SNP_2, SNP_3)$. The set of alleles of the three paternal genes may be described as $(a_{p1}, a_{p2}, a_{p3})$. Consider the recombination nodules formed in Figure 1, and assume that there is just one recombination for each pair of recombining chromatids. The set of gametes that are formed in this process will have gene alleles: $(a_{m1}, a_{m2}, a_{p3})$, $(a_{m1}, a_{p2}, a_{p3})$, $(a_{p1}, a_{m2}, a_{p3})$, $(a_{p1}, a_{p2}, a_{m3})$. In the case with no crossing over of chromatids, the gametes will have alleles $(a_{m1}, a_{m2}, a_{m3})$, $(a_{p1}, a_{p2}, a_{p3})$. In the case with two points of crossing over in the relevant regions, the gametes will have alleles $(a_{m1}, a_{p2}, a_{m3})$, $(a_{p1}, a_{m2}, a_{p3})$. These eight different combinations of alleles will be referred to as the hypothesis set of alleles, for that particular parent.

The measurement of the alleles from the embryonic DNA will be noisy. For the purpose of this discussion take a single chromosome from the embryonic DNA, and assume that it came from the parent whose meiosis is illustrated in Figure 1. The measurements of the alleles on this chromosome can be described in terms of a vector of indicator variables: $A = [A_1\, A_2\, A_3]^T$ where $A_1 = 1$ if the measured allele in the embryonic chromosome is $a_{m1}$, $A_1 = -1$ if the measured allele in the embryonic chromosome is $a_{p1}$, and $A_1 = 0$ if the measured allele is neither $a_{m1}$ or $a_{p1}$. Based on the hypothesis set of alleles for the assumed parent, a set of eight vectors may be created which correspond to all the possible gametes describe above. For the alleles described above, these vectors would be $a_1 = [1\,1\,1]^T$, $a_2 = [1\,1\,-1]^T$, $a_3 = [1\,-1\,1]^T$, $a_4 = [1\,-1\,-1]^T$, as = $[-1\,1\,1]^T$, $a_6 = [-1\,1\,-1]^T$, $a_7 = [-1\,-1\,1]^T$, as = $[-1\,-1\,-1]^T$. In this highly simplified application of the system, the likely alleles of the embryo can be determined by performing a simple correlation analysis between the hypothesis set and the measured vectors:

$$i^* = \arg\max_i A^T a_i, \quad i = 1 \dots 8 \tag{1}$$

Once i* is found, the hypothesis $a_i^*$ is selected as the most likely set of alleles in the embryonic DNA. This process is then repeated twice, with two different assumptions, namely that the embryonic chromosome came from the mother or the father. That assumption which yields the largest correlation $A^T a_i^*$ would be assumed to be correct. In each case a hypothesis set of alleles is used, based on the measurements of the respective DNA of the mother or the father. Note that in a typical embodiment of the disclosed method, one measures a large number of SNPs between those SNPs that are important due to their association with particular disease phenotypes - these will be referred to these as Phenotype-associated SNPs or PSNPs. The Non-phenotype-associated SNPs (NSNPs) between the PSNPs may be chosen a-priori (for example, for developing a specialized genotyping array) by selecting from the NCBI dbSNP database those RefSNPs that tend to differ substantially between individuals. Alternatively, the NSNPs between the PSNPs may be chosen for a particular pair of parents because they differ between the parents. The use of the additional SNPs between the PSNPs enables one to determine with a higher level of confidence whether crossover occurs between the PSNPs. It is important to note that while different "alleles" are referred to in this notation, this is merely a convenience; the SNPs may not be associated with genes that encode proteins.

*The System in the Context of Current Technology*

In another more complex embodiment, the a-posteriori probability of a set of alleles is computed given a particular measurement, taking into account the probability of particular crossovers. In addition, the scenario typical of microarrays and other genotyping technologies is addressed where SNPs are measured for pairs of chromosomes, rather than for a single chromosome at a time. The measurements of the genotype at the locus i for the embryonic, paternal and maternal chromosomes may be characterized respectively by random variables representing the pairs of SNP measurements ($e_{1,i}$, $e_{2,i}$), ($p_{1,i}$, $p_{2,i}$) and ($m_{1,i}$, $m_{2,i}$). Since one cannot determine the presence of crossovers in the maternal and paternal chromosomes if all measurements are made as pairs, the method is modified: in addition to genotyping the fertilized embryos and paternal and maternal diploid tissue, one haploid cell from each parent, namely, a sperm cell and an egg cell, is also genotyped. The measured alleles of the sperm cell are represented by $p_{1,i}$, i=1... N and the complementary alleles measured from the paternal diploid tissue by $p_{2,i}$. Similarly, the measured alleles of the egg cell are represented by $m_{1,i}$ and their complement in the mother's diploid cell by $m_{2,i}$. These measurements provide no information on where the parental chromosomes crossed over in generating the measured sperm and egg cells. However, one can assume that the sequence of N alleles on the egg or sperm was created from the parental chromosomes by a small number of, or no, crossovers. This is sufficient information to apply the disclosed algorithm. A certain error probability is associated with calling the paternal and maternal SNPs. The estimation of this error probability will vary based on the measurements made ($p_{1,i}$, $p_{2,i}$) and ($m_{1,i}$, $m_{2,i}$) and the signal-to-noise ratio for the technology used. Although these error probabilities can be uniquely computed for each locus without affecting the disclosed method, the algebra is simplified here by assuming that the probabilities of correctly calling the paternal and maternal SNPs are constant at $p_p$ and $p_m$ respectively.

Assume that a measurement is performed on the embryonic DNA which is termed measurement M. In addition, the notation is slightly modified so that A is now a set and not a vector: A refers to a particular hypothesis about the combination (or set) of alleles derived from each parent. The set of all possible combinations of alleles A from both parents is denoted as $S_A$. The goal is to determine the combination of alleles (or that hypothesis) $A \in S_A$ with the highest a-posteriori probability, given the measurement M:

$$A^* = \arg\max_A P(A \mid M), \forall A \in S_A \tag{2}$$

Using the law of conditional probabilities, P(A|M) = P(M|A)P(A)/P(M). Since P(M) is common for all different A's, the optimizing search can be recast as:

$$A^* = \arg\max_A P(M / A)P(A), \forall A \in S_A \tag{3}$$

Now consider the computation of P(M/A). Begin with a single locus i, and let the hypothesis be that this locus on the embryo is derived from the parental SNPs $p_{t,1,i}$ and $m_{t,1,i}$, where the underscore $_t$ is used to denote the true value of these Parental SNPs, as opposed to the measurements performed, $p_{1,i}$ and $m_{1,i}$, which may or may not be correct. The true value of the embryonic SNPs is denoted as ($e_{t,1,i}$, $e_{t,2,i}$). If hypothesis A is true, then ($e_{t,1,i}$, $e_{t,2,i}$) = ($p_{t,1,i}$, $m_{t,1,i}$) or ($m_{t,1,i}$, $p_{t,1,i}$). Since one cannot differentiate which of the measurements ($e_{1,i}$, $e_{2,i}$) comes from which parent, both orders must be considered so the hypothesis set A = [($p_{t,1,i}$, $m_{t,1,i}$), ($m_{t,1,i}$, $p_{t,1,i}$)]. The probability of a particular measurement M depends on the true values or the underlying states of the parental SNPs, namely ($p_{t,1,i}$, $p_{t,2,i}$) and ($m_{t,1,i}$, $m_{t,2,i}$). Since there are four SNPs, $p_{t,1,i}$, $p_{t,2,i}$, $m_{t,1,i}$, $m_{t,2,i}$, and each of these can assume the value of four nucleotide bases, A,C,T,G, there are $4^4$ or 256 possible states. The algorithm is illustrated for one state $s_1$ for which it is assumed that $p_{t,1,i} \neq p_{t,2,i} \neq m_{t,1,i} \neq m_{t,2,i}$. From this explanation, it will be clear how to apply the method to all 256 possible states, $s_k$, k=1... 256. Assume a measurement M of embryonic SNPs ($e_{1,i}$, $e_{2,i}$) is performed, and the result $e_{1,i} = p_{1,i}$, $e_{2,i} = m_{1,I}$ is obtained. The a priori probability for this measurement given that hypothesis A and state $s_1$ are true is computed:

$$P(e_{1,i} = p_{1,i}, e_{2,i} = m_{1,i} \mid A, s_1) =$$
$$P(e_{t,1,i} = p_{t,1,i}, e_{t,2,i} = m_{t,1,i} \mid A, s_1) P(e_{1,i} = p_{1,i} \mid e_{t,1,i} = p_{t,1,i}) P(e_{2,i} = m_{1,i} \mid e_{t,2,i} = m_{t,1,i})$$
$$+ P(e_{t,1,i} = m_{t,1,i}, e_{t,2,i} = p_{t,1,i} \mid A, s_1) P(e_{1,i} = p_{1,i} \mid e_{t,1,i} = m_{t,1,i}, p_{t,1,i} \neq m_{t,1,i}) P(e_{2,i} = m_{1,i} \mid e_{t,2,i} = p_{t,2,i}, p_{t,2,i} \neq m_{t,1,i})$$

$$(4)$$

Consider the first expressions in the first term and second term: $P(e_{1,i}=p_{1,i}, e_{2,i}=m_{1,i}|A,s_1)= P(e_{1,i}=M_{1,i}, e_{2,i}=p_{1,i}|A,s_1)=0.5$ since the hypothesis $A=[(p_{t,1,i}, m_{t,1,i}), (m_{t,1,i}, p_{t,1,i})]$ makes two orderings for the embryonic SNPs equally likely. Now consider the second expression of the first term, $P(e_{1,i}=p_{1,i} |e_{t,1,i}=p_{t,1,i})$, the probability of measuring $e_{1,i}=p_{1,i}$ given the assumption that embryonic SNP $e_{t,1,i}$ actually is derived from paternal SNP $p_{t,1,i}$. The probabilities for correctly measuring the paternal SNPs, maternal SNPs, and embryonic SNPs are $p_p$, $p_m$, and $p_e$. Given the assumption $(e_{t,1,i}=p_{t,1,i})$, the measurement $(e_{1,i}=p_{1,i})$ requires either that both embryonic and paternal SNPs are correctly measured, or that both are incorrectly measured and they happen to be incorrectly measured as the same nucleotide (A,C,T, or G). So, $P(e_{1,i}=p_{1,i}| e_{t,1,i}=p_{t,1,i}) = p_e p_p+(1-p_e)(1-p_p)/3$ where it is assumed for simplicity that the probability of incorrectly calling all of the four nucleotides is equally likely - the algorithm can be easily modified to accommodate different probabilities of calling a particular nucleotide (A,C,T,G) given a measurement on another particular nucleotide. The same approach may be applied to the third expression in the first term to obtain $P(e_{2,i}=m_{1,i} |e_{t,2,i}=m_{t,1,i}) = p_e p_m+(1-p_e)(1-p_m)/3$. Now consider the second expression of the second term. $P(e_{1,i}=p_{1,i} |e_{t,1,i}=m_{t,1,i}, m_{t,1,i}\neq p_{t,1,i})$ requires either that $e_{1,i}$ or $p_{1,i}$ be an incorrect measurement, or that both be incorrect measurements, so that the measured values happen to be equal: $P(e_{1,i}=p_{1,i} | e_{t,1,i}=m_{t,1,i}, m_{t,1,i}\neq p_{t,1,i}) = p_e(1-p_p)/3+(1-p_e)p_p/3+(1-p_e)(1-p_p)2/9$. The same argument can be applied to the last expression of the second term to yield $P(e_{2,i}=m_{1,i} |e_{t,2,i}=p_{t,2,i}, m_{t,1,i}\neq p_{t,2,i}) = p_e(1-p_m)/3+(1-p_e)p_m/3+(1-p_e)(1-p_m)2/9$. Now, combining all of these terms, and making the assumption - merely to simplify the algebra - that $p_e=p_p=p_m=p$, one can compute:

$$P\big(M(e_{1,i} = p_{1,i}, e_{2,i} = m_{1,i}) \mid A, s_1\big) = \frac{1}{162}\big(160p^4 - 160p^3 + 96p^2 - 28p + 13\big) \qquad (5)$$

Although the computation will vary, a similar conceptual approach to that described here would be used for all 256 possible states, $s_k$, k=1... 256. Computing $P(e_{1,i}=p_{1,i}, e_{2,i}=m_{1,i}|A,s_i)$ for all 256 states $s_i$ and summing over the probability of each $s_i$ one obtains $P(e_{1,i}=p_{1,i}, e_{2,i}=m_{1,i} |A)$. In other words:

$$P(M \mid A) = \sum_{i=1\ldots256} P(M \mid A, s_i) P(s_i) \qquad (6)$$

In order to compute the probabilities of each state $s_i$, $P(s_i)$, one must treat all the separate alleles making up a state as separate events since they are on separate chromosomes, in other words: $P(s_i) = P(p_{t,1,i}, p_{t,2,i}, m_{t,1,i}, m_{t,2,i}) = P(p_{t,1,i}) P(p_{t,2,i})P(m_{t,1,i})P(m_{t,2,i})$. Bayesian techniques may be applied to estimate the probability distribution for the individual measurements. Every measurement of an allele on the maternal or paternal chromosomes at locus i may be treated as a coin toss experiment to measure the probability of this allele being a particular value (A,C,T or G). These measurements are made on the adult tissue samples and may be treated as being totally reliable, even though pairs of alleles are measured for each SNP, and it is not possible to determine which allele comes from which chromosome. Let $w_{p,1,i} = P(p_{t,1,i})$, corresponding to the probability of the SNP i on the father's chromosome being value $p_{t,1,i}$. In the following explanation, w is used instead of $w_{p,1,i}$. Let the measurements performed on SNP i of the father's chromosome be characterized as collecting data D. One can create a probability distribution for w, p(w) and update this after the data is measurement according to Bayes Theorem: p(w|D)= p(w)p(D|w)/p(D). Assume n alleles of SNP i are observed and that the particular allele corresponding to w comes up h times - in other words, heads is observed h times. The probability of this observation can be characterized by the binomial distribution

$$p(D \mid w) = \binom{n}{h} w^h (1-w)^{n-h} \qquad (7)$$

Before data is collected, assume there is a prior distribution p(w) which is uniform between 0 and 1. By applying the Bayes theorem, it is straightforward to show that the resulting distribution for p(w|D) will be a beta distribution of the form:

$$p(w \mid D) = \frac{1}{c} w^h (1-w)^{n-h} \ where \ c = \int_0^1 w^h (1-w)^{n-h} dw \tag{8}$$

and c is a normalizing constant. However many times p(w|D) is then updated by applying Bayes theorem and new measurements, it will continue to have a beta distribution as above. The estimates of p(w) are updated every time a new measurement is collected. Note that there will be a different function p(w) for different races and different genders, using the same groupings used in the Hapmap project, since the probability of different alleles at particular SNPs is dependent on these groupings of race and gender. For the computation of $P(s_i)$, each allele on each chromosome will be associated with an estimated probability distribution, namely $p_{p,1,i}(w_{p,1,i})$, $p_{p,2,i}(w_{p,2,i})$, $p_{m,1,i}(w_{m,1,i})$ and $p_{m,2,i}(w_{m,2,i})$. One may then compute the maximum a-posteriori (MAP) estimate for $P(s_i)$ according to the MAP estimate for each of the individual distributions. For example, let $w_{p,1,i}^*$ be the argument that maximizes $p_{p,1,i}(w_{p,1,i})$.
The MAP estimate of $P(s_i)$ may be found according to

$$P(s_i)_{MAP} = w_{p,1,i}^* \cdot w_{p,2,i}^* \cdot w_{m,1,i}^* \cdot w_{m,2,i}^* \tag{9}$$

Since there is the a probability distribution for each w, one can also compute conservative estimates of the values $P(s_i)$ to any specified confidence level, by integrating over the probability distribution, rather than simply using the MAP estimates. It is possible to do this, for example, to conservatively estimate P(M|A) to within some confidence level. Whether a conservative estimate or a MAP estimate is used, the estimate of $P(s_i)$ is continually refined for the computation of P(M|A). In what follows, reference to the assumed state will be eliminated to simplify the notation, and state $s_1$ is assumed for all explanations of detailed computation. Bear in mind that in actuality these calculations would be performed for each of 256 states and be summed over the probability of each.

The method of computing P(M|A) is now extended to multiple SNP loci, assuming that M represents the set of measurements of N pairs of SNPs on the embryo, $M = [M_1,...,M_N]$. Assume also that A represents the set of hypotheses for each SNP about which parental chromosomes contributed to that SNP, $A = [A_1,...,A_N]$. Let $S_{A'}$ represent the set of all other possible hypotheses that are different from A or are in the set A'. P(M|A) and P(M|A') may be computed:

$$P(M \mid A) = \prod_{i=1...N} P(M_i \mid A_i), \quad P(M \mid A') = \sum_{A \in S_{A'}} P(A) \prod_{i=1...N} P(M_i \mid A_i) \tag{10}$$

Consider the computation of P(A). In essence, this is based on the likelihood of particular crossovers occurring in the formation of the gametes that form the embryo. The probability of a particular allele set depends on two factors, namely the probability that the embryonic chromosome comes from the mother or the father, and the probability of a particular combination of crossovers. For a normal set of embryonic chromosomes that do not suffer from aneuploidy, the a-priori probability that the embryonic chromosome comes from the mother or father is ~50% and is consequently common for all A. Now, consider the probability of a particular set of recombination nodes. The number of relevant recombination sites R depends on the number of measured SNPS: R=N-1. Since the DNA segment constituting N NSNPs around the PSNP of interest will be relatively short, crossover interference makes it highly improbable that two crossovers on the same chromosome can occur in one region. For reasons of computational efficiency this method assumes that only one crossover will occur in each region for each relevant chromosome, and this can occur at R possible sites. It will be obvious to someone skilled in the art how this method may be extended to include the possibility where there are multiple crossovers in a given region.

Let the probability of a crossover in each region between SNPs be denoted $P_r$, r = 1...N-1. To first order, the probability of a recombination node in a region r between two SNPs is proportional to the genetic distance between those SNPs (measured in cMorgans). However, much recent research has enabled a precise modeling of the probability of recombination between two SNP loci. Observations from sperm studies and patterns of genetic variation show that recombination rates vary extensively over kilobase scales and that much recombination occurs in recombination hotspots, and causes linkage disequilibrium to display a block-like structure. The NCBI data about recombination rates on the Human Genome is publicly available through the UCSC Genome Annotation Database.

Various data sets can be used singly or in combination. Two of the most common data sets are from the Hapmap Project and from the Perlegen Human Haplotype Project. The latter is higher density; the former is higher quality. See Figure 2 for the regional recombination rates from positions 1,038,423 to 4,467,775 of chromosome 1, based on the HapMap Phase I data, release 16a. These rates were estimated using the reversible-jump Markov Chain Monte Carlo (MCMC) method which is available in the package LDHat. The state-space considered is the distribution of piece-wise constant recombination rate maps. The Markov chain explores the distribution of the number and location of rate change-points, in addition to the rates for each segment, 201. These results may be used to generate an estimate of $P_r$ by integrating over the recombination rates times by the length of each constant segment between the SNPS. The cumulative recombination rate over the nucleotides 202 is shown in Figure 2 in red.

Let C be a set of indicator variables $c_r$ such that $c_r=1$ if a crossover occurred in region r and 0 otherwise. $c_0=1$ if no crossovers occurred and 0 otherwise. Since it is assumed that only one crossover can occur in a region of N SNPs, only one element of the set C is non-zero. Hence, the probability of crossover represented by set C is found to be:

$$P_c = \left(1 - \sum_{r=1...N-1} P_r\right)^{c_0} \prod_{r=1} P_r{}^{c_r} \tag{11}$$

In the hypothesis A about SNPs 1... N, there are four potential crossovers of relevance. Namely, the potential crossovers in i) the paternal chromosomes that formed the embryo (denoted by set $C_{pe}$ of indicator variables), ii) the paternal chromosomes that formed the sequenced sperm (set $C_{ps}$), iii) the maternal chromosomes that formed the embryo (set $C_{me}$) and iv) the maternal chromosomes that formed the sequenced egg (set $C_{ee}$). Two additional assumptions are v) whether the first paternal embryonic SNP comes from $p_{t,1,1}$ or $p_{t,2,1}$ and vi) whether the first maternal embryonic SNP comes from $m_{t,1,1}$ or $m_{t,2,1}$. Since the probabilities of crossovers between SNPs is found to differ between races and sexes, different crossover probabilities will be denoted as $P_{p,r}$ for the paternal chromosomes, and $P_{m,r}$ for the maternal chromosomes. Therefore, the probability of a particular hypothesis A, which subsumes the sets $C_{pe}$, $C_{ps}$, $C_{me}$, $C_{ee}$ is expressed as:

$$P(A) = \frac{1}{4}\left(1 - \sum_{r=1...N-1} P_{p,r}\right)^{c_{pe,0}} \prod_{r=1...N-1} P_{p,r}{}^{c_{ps,r}} \left(1 - \sum_{r=1...N-1} P_{p,r}\right)^{c_{ps,0}} \prod_{r=1...N-1} P_{p,r}{}^{c_{ps,r}} \left(1 - \sum_{r=1...N-1} P_{m,r}\right)^{c_{me,0}} \prod_{r=1...N-1} P_{m,r}{}^{c_{me,r}} \left(1 - \sum_{r=1...N-1} P_{m,r}\right)^{c_{ee,0}} \prod_{r=1...N-1} P_{m,r}{}^{c_{ee,r}} \tag{12}$$

Now with the equations for determining P(A) and P(M/A), all the elements necessary to compute A* per Equation 3 above have been defined. Hence, it is possible to determine from the highly error-prone measurements of the embryonic SNPs where crossovers occurred, and to consequently clean the embryonic measurements with a high degree of confidence. It remains to determine the degree of confidence in the best hypothesis A*. To determine this, it is necessary to find the odds ratio P(A*|M)/P(A*'|M). The tools have all been described above for this computation:

$$\frac{P(A^*|M)}{P(A^{*'}|M)} = \frac{P(A^*|M)}{1-P(A^*|M)} = \frac{P(A^*)P(M|A^*)}{P(A^{*'})P(M|A^{*'})} = \frac{P(A^*)P(M|A^*)}{(1-P(A^*))P(M|A^{*'})} = OR_{A^*} \tag{13}$$

The confidence in A* is then given as $P(A^*|M) = OR_A{}^*/(1+OR_A{}^*)$. This computation indicates the confidence in a particular hypothesis A*, but it does not indicate a confidence in a particular determination of a SNP. In order to compute the confidence in a determination of embryonic PSNP n, it is necessary to create the set of all hypotheses A that don't change the value of this SNP. This set will be denoted as $S_{A^*,n}$, which corresponds to all hypothesis that result in PSNP n on the embryo having the same value as is predicted by hypothesis A*. Similarly, create a set $S_{A^{*'},n}$ which corresponds to all hypothesis that result in PSNP n having a different value to that predicted by hypothesis A*. Now, it is possible to compute the odds ratio of the probability that the SNP is correctly called versus the probability that the SNP is incorrectly called:

$$OR_{A^*,n} = \frac{\sum_{A \in S_{A^*,n}} P(A|M)}{\sum_{A \in S_{A^{*'},n}} P(A|M)} = \frac{\sum_{A \in S_{A^*,n}} P(A|M)}{1 - \sum_{A \in S_{A^*,n}} P(A|M)} = \frac{\sum_{A \in S_{A^*,n}} P(A)P(M|A)}{\sum_{A \in S_{A^{*'},n}} P(A)P(M|A)} \tag{14}$$

The confidence in the particular call of embryonic SNP n based on the odds ratio $OR_{A^*,n}$ can be computed as:

$$P(correctly\ called\ SNP\ n) = \sum_{A \in S_{A^*,n}} P(A|M) = \frac{OR_{A^*,n}}{1+OR_{A^*,n}} \tag{15}$$

Note that this technique could also be used to detect such defects as uniparental disomy (UPD) wherein two of the same chromosomes are from the same parent, while none of that chromosomes from the other parent is present. Upon attempting to deduce the crossovers in the parent chromosomes, there will be no hypothesis which adequately explains the data with a high confidence, and if alternate hypotheses are allowed that include the possibility of UPD, they will found to be more likely.

*Bounding the Effect of Uncertainty in Recombination Rates and SNP Measurement Reliability*

The disclosed method depends on: assumptions about the probability of recombination between particular SNPs; assumptions about the probability of the correct measurement of each SNP on the embryonic, sperm, egg, paternal and maternal chromosomes; and assumptions about the likelihood of certain alleles within different population groups. Consider each of these assumptions: the mechanism of recombination is not perfectly understood and modeled, and the crossover probability has been established to vary based on an individual's genotype. Furthermore, the techniques by which the recombination rates are measured show substantial variability. For example, the package LDHat, which implements the reversible-jump Markov Chain Monte Carlo (MCMC) method, makes a set of assumptions and requires a set of user inputs about the mechanism and characterization of recombination. These assumptions can affect predicted recombination rates between SNPs as is evinced by the different results obtained by various studies.

It is anticipated that the assumptions about recombination rates, out of all assumptions listed above, will have the most impact on Equation 15. The computations described above should be based on the best estimates of the probability for crossover between SNPS, $P_r$. Thereafter, conservative estimates can be used for $P_r$ using values at, for example, the 95% confidence bounds for the recombination rates, in the direction that reduces the confidence measure P(correctly called SNP n). The 95% confidence bounds can be derived from confidence data produced by various studies of recombination rates, and this can be corroborated by looking at the level of discordance between published data from different groups using different methods.

Similarly, the 95% confidence bounds can be used for the estimates of the probability that each SNP is correctly called: $p_p$, $p_m$, $p_e$. These numbers can be computed based on the actual measured array intensities included in the genotyping assay output files, combined with empirical data on the reliability of the measurement technique. Note that those NSNPs for which these parameters $p_p$, $p_m$ and $p_e$ are not well established may be ignored. For example, since the diploid parental data is reliably measured, one may ignore NSNP measurements of the parents' haploid cells and on the embryo that do not correspond to any of the alleles on the relevant SNPs of the parent's diploid tissue.

Lastly, consider the assumptions about the likelihood of certain alleles within different population groups, which give rise to the computation $P(s_i)$. These assumptions also will not have a large impact on the disclosed method since the measurement of the parental diploid data is reliable i.e. direct measurement of the state $s_i$ from the parental samples typically result in data with high confidence. Nonetheless, it is possible to use the probability distribution for each w as described in Equation 8 in order to compute a confidence bound for the probability of each state $P(s_i)$. As above, one can compute the 95% confidence bound for each $P(s_i)$ in the conservative direction that reduces confidence measure P(correctly called SNP n).

The determination of P(correctly called SNP n) will inform the decision about how many NSNPs need to be measured around each PSNP in order to achieve the desired level of confidence.

Note that there are different approaches to implementing the concept of the disclosed method, namely combining the measurement of the parent's DNA, the measurement of the DNA of one or more embryos, and the a-priori knowledge of the process of meiosis, in order to obtain a better estimate of the embryonic SNPs. It will be clear to one skilled in the art, how similar methods can be applied when different subsets of the a-priori knowledge are known or not known, or known to a greater or lesser degree of certainty. For example, one can use the measurements of multiple embryos to improve the certainty with which one can call the SNPs of a particular embryo or to accommodate missing data from the parents. Note also that one does not need a PSNP of interest to be measured by the measurement technique. Even if that PSNPs is not determined by the measurement system, it can still be reconstructed with a high degree of confidence by the disclosed method.

Also note that once the points of crossover that occurred during meiosis have been determined, and the regions of the target genome have been mapped to the pertinent regions of the parental DNA, it is possible to infer not only the identity of individual SNPs of interest, but also whole regions of DNA that may be missing in the measured target genome due to allele drop-out or other errors in measurement. It is also possible to measure insertions and deletions in the parental DNA, and use the disclosed method to infer that they exist in the target DNA.

Various techniques may be used to improve the computational complexity of the disclosed algorithm described above. For example, one may only or predominantly select those NSNPs that differ between the mother and the father. Another consideration would be to only use NSNPs that are spaced nearby the PSNPs to minimize the chance of crossovers occurring between the NSNPs and PSNPs of interest. One could also use NSNPs that were spaced along the chromosome so as to maximize coverage of multiple PSNPs. Another consideration will be to initially use only a small number of NSNPs to determine roughly where crossovers occurred, and with only a limited degree of certainty. Additional NSNPs can then be used to refine the crossover model and increase the probability of correctly calling the PSNPs. The number of crossover combinations to consider scales roughly as $N^C$ where N is the number of SNPs and C is the maximum number of crossovers. Consequently, for C=4 it is possible to accommodate roughly N=100 for each PSNP while remaining computationally tractable on a Pentium-IV processor. Using the approaches described above and other approaches for increased computational efficiency, N>100, C>4 can be easily accommodated. One such approach will be described below.

Note that there are many other approaches to make a call on a PSNP and generate an estimate of the probability that a PSNPs has been correctly determined, based on a particular set of embryonic data, parent data, and algorithm used, without changing the underlying concept. This probability can be used for individual decision-making, and for implementing a reliable service in the context of IVF or NIPGD.

*Recursive solution to the genetic data cleaning algorithm*

Given the limited nature of computation power, the length of the computation may be a significant factor in the use of the disclosed method. When running computations, any algorithm that must compute certain values where the number of computations needed rises exponentially with the number of SNPs can become unwieldy. A solution that involves a number of calculations that increase linearly with the number of SNPs will always be preferred from a time standpoint as the number of SNPs gets large. Below this approach is described.

A simple approach, which is to consider all possible hypotheses must contend with the running time being an exponential function in number of SNPs. Suppose, as before, that measured data are a collection of measured embryo, father and mother chromosome measurements on k SNPs, i.e. $M = \{M_1,...,M_k\}$ where $M_i = (e_{1i}, e_{2i}, p_{1i}, p_{2i}, m_{1i}, m_{2i})$. As before, the hypotheses space is $S_H = \{H^1,...,H^q\}=\{$set of all the hypotheses$\}$, where each hypothesis is of the format $H^j = (H^j_1,...H^j_k)$ where $H^j_i$ is the "mini" hypothesis for snip i, of the format $H^j_i = (p_i^*, m_i^*)$ where $p_i^* \in \{p_{1i}, p_{2i}\}$ and $m_i^* \in \{m_{1i}, m_{2i}\}$. There are 4 different "mini" hypotheses $H^j_i$, in particular:

$H^j_i1: (e_{1i},e_{2i})=\{(p_{1i},m_{1i})$ or $(m_{1i},p_{1i})\}$
$H^j_i2: (e_{1i},e_{2i})=\{(p_{1i},m_{2i})$ or $(m_{2i},p_{1i})\}$
$H^j_i3: (e_{1i},e_{2i})=\{(p_{2i},m_{1i})$ or $(m_{1i},p_{2i})\}$
$H^j_i4: (e_{1i},e_{2i})=\{(p_{2i},m_{2i})$ or $(m_{2i},p_{2i})\}$

The goal is to choose the most likely hypothesis H* as:

$$H^* = \arg\max_{H \in S_H} P(H \mid M) = \arg\max_{H \in S_H} F(M,H)$$

where function F(M,H)=P(H|M)

There are $4^k$ different hypotheses in the space $S^H$. By trying to find the best hypothesis by exhaustively exploring the entire space $S^H$, the necessary algorithm would be of exponential order in k O(exp(k)), where k is the number of SNPs involved. For large k, even k>5, this is immensely slow and unpractical. Therefore, it is more practical to resort to a recursive solution which solves the problem of size k as a function of the problem of size (k-1) in constant time. The solution shown here is of the linear order in k, O(k).

*Recursive solution linear in the number of SNPs*

Begin with F(M,H)=P(H|M) = P(M|H)*P(H)/P(M). Then argmax $_H$ F(M,H) = argmax $_H$ P(M|H)*P(H) and the goal is to solve P(M|H)*P(H) in linear time. Suppose that $M_{(s,k)}$= measurement on SNPs s to k, $H_{(s,k)}$ = hypothesis on SNPs s to k, and to simplify notation $M_{(k,k)} = M_k$, $H_{(k,k)} = H_k$ = measurement and hypothesis on SNP k. As shown before:

$$P(M_{(1,k)} \mid H_{(1,k)}) = \prod_{i=1}^{k} P(M_i \mid H_i) = P(M_k \mid H_k) * \prod_{i=1}^{k-1} P(M_i \mid H_i) = P(M_k \mid H_k) * P(M_{(1,k-1)} \mid H_{(1,k-1)})$$

Also:

$$P(H_{(1,k)}) = 1/4 * \prod_{i=2}^{k} PF(H_{i-1}, H_i) = PF(H_{k-1}, H_k) * 1/4 * \prod_{i=2}^{k-1} PF(H_{i-1}, H_i) = PF(H_{k-1}, H_k) * P(H_{(1,k-1)})$$

where

$$PF(H_{i-1}, H_i) = \begin{cases} 1 - PC(H_{i-1}, H_i) & H_{i-1} = H_i \\ PC(H_{i-1}, H_i) & H_{i-1} \neq H_i \end{cases}$$

and $PC(H_{i-1}, H_i)$ = probability of crossover between $H_{i-1}$, $H_i$

Finally, for k SNPs:

$$F(M, H) = P(M \mid H) * P(H) = P(M_{(1,k)} \mid H_{(1,k)}) * P(H_{(1,k)})$$

$$= P(M_{(1,k-1)} \mid H_{(1,k-1)}) * P(H_{(1,k-1)}) * P(M_k \mid H_k) * PF(H_{k-1}, H_k)$$

so in short $F(M,H) = F(M_{(1,k)}, H_{(1,k)})) = F(M_{(1,k-1)}, H_{(1,k-1)}) * P(M_k \mid H_k) * PF(H_{k-1}, H_k)$ i.e. we can reduce the calculation of F on k SNPs to the calculation of F on k-1 SNPs.

For $H = (H_1, ... H_k)$, the hypothesis on k SNPs:

$$\max_H F(M, H) = \max_{(H_{(1,k-1)}, H_k)} F(M, (H_{(1,k-1)}, H_k)) = \max_{H_k} \max_{H_{(1,k-1)}} F(M, (H_{(1,k-1)}, H_k)) = \max_{H_k} G(M_{(1,k)}, H_k)$$

where

$$G(M_{(1,n)}, H_n) = \max_{H_{(1,n-1)}} F(M_{(1,n)}, (H_{(1,n-1)}, H_n)) =$$

$$\max_{H_{(1,n-1)}} F(M_{(1,n-1)}, H_{(1,n-1)}) * P(M_n \mid H_n) * PF(H_{n-1}, H_n) =$$

$$P(M_n \mid H_n) * \max_{H_{(1,n-1)}} F(M_{(1,n-1)}, H_{(1,n-1)}) * PF(H_{n-1}, H_n) =$$

$$P(M_n \mid H_n) * \max_{H_{n-1}} \max_{H_{(1,n-2)}} F(M_{(1,n-1)}, (H_{(1,n-2)}, H_{n-1})) * PF(H_{n-1}, H_n) =$$

$$P(M_n \mid H_n) * \max_{H_{n-1}} PF(H_{n-1}, H_n) * G(M_{(1,n-1)}, H_{n-1})$$

To summarize this:

$$\max_H F(M, H) = \max_{H_n} G(M_{(1,k)}, H_k)$$

where G can be found recursively: for n=2,..,k

$$G(M_{(1,n)}, H_n) = P(M_n \mid H_n) * \max_{H_{n-1}} \left[ PF(H_{n-1}, H_n) * G(M_{(1,n-1)}, H_{n-1}) \right]$$

and

$$G(M_{(1,1)}, H_1) = 0.25 * P(M_1 \mid H_1)$$

The algorithm is as follows:

For n = 1: Generate 4 hypotheses $H_1 i$, calculate $G(M_1 \mid H_1 i)$ for i=1,...,4.

For n = 2: Generate 4 hypothesis for $H_2 i$, calculate $G(M_{(1,2)} \mid H_2 i)$ ,i=1,...,4 in constant time using the formula:

$$G(M_{(1,2)}, H_2 i) = P(M_2 \mid H_2 i) * \max_{j=1,...,4} \left[ PF(H_1, H_2 i) * G(M_1, H_1 j) \right]$$

For n = k: Generate 4 hypothesis for $H_k i$, make $G(M_{(1,k)} \mid H_k i)$, i=1,...,4 by

$$G(M_{(1,k)}, H_k i) = P(M_k \mid H_k i) * \max_{j=1,...,4} \left[ PF(H_{k-1}, H_k i) * G(M_{(1,k-1)}, H_{k-1} j) \right]$$

At any time there are only 4 hypotheses to remember and a constant number of operations. So the algorithm is linear in k, number of SNPs, as opposed to exponential.

*Solving P(M) in linear time*

It is not necessary to solve for P(M) to get the best hypothesis, since it is constant for all H. But in order to get the actual, meaningful number for the conditional probability P(H|M) = P(MIH)*P(H)/P(M), it is also necessary to derive P(M). As above, we can write:

$$P(M) = P(M_{(1,k)}) = \sum_{H_{(1,k)}} P(M_{(1,k)} \mid H_{(1,k)}) * P(H_{(1,k)})$$

$$= \sum_{H_k} P(M_K \mid H_k) \sum_{H_{(1,k-1)}} P(M_{(1,k-1)} \mid H_{(1,k-1)}) * P(H_{(1,k-1)}) * PF(H_{k-1}, H_k)$$

$$= \sum_{H_k} P(M_K \mid H_k) * W(M_{(1,k-1)} \mid H_k)$$

where
$$W(M_{(1,k-1)} \mid H_k) = \sum_{H_{(1,k-1)}} P(M_{(1,k-1)} \mid H_{(1,k-1)}) * P(H_{(1,k-1)}) * PF(H_{k-1}, H_k)$$
We can solve for W(M,H) by recursion:

$$W(M_{(1,k-1)} \mid H_k) = \sum_{H_{(1,k-1)}} P(M_{(1,k-1)} \mid H_{(1,k-1)}) * P(H_{(1,k-1)}) * PF(H_{k-1}, H_k)$$

$$= \sum_{H_{k-1}} P(M_{k-1} \mid H_{k-1}) \sum_{H_{(1,k-2)}} P(M_{(1,k-2)} \mid H_{(1,k-2)}) * P(H_{(1,k-2)}) * PF(H_{k-2}, H_{k-1}) * PF(H_{k-1}, H_k)$$

$$= \sum_{H_{k-1}} P(M_{k-1} \mid H_{k-1}) * PF(H_{k-1}, H_k) * W(M_{(1,k-2)} \mid H_{k-1})$$

so in short, problem of size k is reduced to the problem of size (k-1) by

$$W(M_{(1,k-1)} \mid H_k) = \sum_{H_{k-1}} P(M_{k-1} \mid H_{k-1}) * PF(H_{k-1}, H_k) * W(M_{(1,k-2)} \mid H_{k-1})$$

and

$$W(M_{(1,1)} \mid H_2) = \sum_{H_1} P(M_1 \mid H_1) * 0.25 * PF(H_1, H_2)$$

As before, for n = 2:k, generate *W(2),..., W(K)* = *W(M_{(1,k-1)} | H_k)* recursively, until finally, it is possible to derive
$$P(M) = \sum_{H_k} P(M_K \mid H_k) * W(M_{(1,k-1)} \mid H_k)$$
.

At each level there are only four different hypotheses $H_k$, so the algorithm is again linear in the number of SNPs k.

*Individual SNP confidence in linear time*

Once we the best hypothesis H* = (H$_1$*,... ,H$_k$*), has been computed, it is now may be desired to derive the confidence in the final answer for each SNP, namely P(H$_i$*|M), for i=1,...,k. As before P(H$_i$*|M) = P(M| H$_i$*)P(H$_i$*)/P(M) =W(H$_i$*,M)/P(M), where P(M) is already known.

$$W(M, H_i^*) = \sum_{H, H_i = H_i^*} P(M \mid H) * P(H) = \sum_{H = (H_{(1,i-1)}, H_i^*, H_{(i+1,k)})} P(M \mid H) * P(H)$$
, i.e. hypothesis H has been broken up to the hypothesis on first i-1 SNPs, ith SNP, and hypothesis on the i+1 to kth SNP. As before:

$$P(M_{(1,k)} \mid H_{(1,k)}) = \prod_{j=1}^{k} P(M_j \mid H_j) = \prod_{j=1}^{i-1} P(M_j \mid H_j) * P(M_i \mid H_i^*) * \prod_{j=i+1}^{k} P(M_j \mid H_j)$$
$$= P(M_{(1,i-1)} \mid H_{(1,i-1)}) * P(M_i \mid H_i^*) * P(M_{(i+1,k)} \mid H_{(i+1,k)})$$

and

$$P(H_{(1,k)}) = 1/4 * \prod_{j=2}^{k} PF(H_{j-1}, H_j)$$
$$= 1/4 * \prod_{j=2}^{i-1} PF(H_{j-1}, H_j) * PF(H_{i-1}, H_i^*) * PF(H_{i-1}, H_i^*) * \prod_{j=j+2}^{k} PF(H_{j-1}, H_j)$$
$$= 1/4 * T(H_{(1,i-1)}) * PF(H_{i-1}, H_i^*) * PF(H_{i-1}, H_i^*) * T(H_{(i+1,k)})$$

So

$$P(H_{(1,k)}) = 1/4 * T(H_{(1,k)}) = 1/4 * T(H_{(1,i-1)}) * PF(H_{i-1}, H_i^*) * PF(H_{i-1}, H_i^*) * T(H_{(i+1,k)})$$

where

$$T(H_{(1,k)}) = \prod_{j=2}^{k} PF(H_{j-1}, H_j).$$

From this it is possible to show that

$$W(M_{(1,k)}, H_i^*) = \sum_{H, H_i = H_i^*} P(M \mid H) * P(H) = \sum_{H, H_i = H_i^*} P(M \mid H) * 1/4 * T(H)$$
$$= \sum_{H=(H_{(1,i-1)}, H_i^*, H_{(i+1,k)})} \frac{P(M_{(1,i-1)} \mid H_{(1,i-1)}) * P(M_i \mid H_i^*) * P(M_{(i+1,k)} \mid H_{(i+1,k)}) *}{1/4 * T(H_{(1,i-1)}) * PF(H_{i-1}, H_i^*) * PF(H_{i-1}, H_i^*) * T(H_{(i+1,k)})}$$
$$= 4 * P(M_i \mid H_i^*) * \left( \sum_{H_{i-1}} P(M_{(1,i-1)} \mid H_{(1,i-1)}) * 1/4 * T(H_{(1,i-1)}) * PF(H_{i-1}, H_i^*) \right)$$
$$* \left( \sum_{H_{i+1}} P(M_{(i+1,k)} \mid H_{(i+1,k)}) * 1/4 * T(H_{(i+1,k)}) * PF(H_i^*, H_{i+1}) \right)$$
$$= 4 * P(M_i \mid H_i^*) * \left( \sum_{H_{i-1}} W(M_{(1,i-1)}, H_{i-1}) * PF(H_{i-1}, H_i^*) \right) * \left( \sum_{H_{i+1}} W(M_{(i+,k)}, H_{i+1}) * PF(H_i^*, H_{i+1}) \right)$$

Again a case of size k has been reduced to two pieces of smaller size, albeit a bit more complicated than before. Each of the pieces can be calculated as

$$W(M_{(1,n)}, H_n) = P(M_n \mid H_n) * \left( \sum_{H_{n-1}} W(M_{(1,n-1)}, H_{n-1}) * PF(H_{n-1}, H_n) \right)$$

$$W(M_{(m,k)}, H_m) = P(M_m \mid H_m) * \left( \sum_{H_{m+1}} W(M_{(m+1,k)}, H_{m+1}) * PF(H_m, H_{m+1}) \right)$$

So the algorithm will, for n = 1,..,k, m = k,..1, for each of 4 different $H_n$, $H_m$ calculate $W(M_{(1,n)}, H_n)$, $W(M_{(m,k)}, H_m)$ and then combine them as needed to calculate $W(M_{(1,k)}, H_i^*)$, for i=1,... ,k. The number of operations is still linear in k.

*Application of the disclosed method to embryonic data when a smaller or different set of data is available*

In one embodiment of the system it is only necessary to make use of diploid data from one parent (presumably the mother), with or without haploid data from either or both of the parents, and when that data is known to a greater or lesser degree of certainty. For example it is expected that, given the grueling nature of egg donation, there will be occasions when maternal haploid data is not readily available. It will be clear to one skilled in the art, after reading this description, how the statistical methods for computing the likelihood of a particular SNP can be modified given a limited data set.

An alternative approach uses data from more distant relatives to make up for missing diploid or haploid data of one or both parents. For example, since it is known that one set of an individual's chromosomes come from each of his or her parents, diploid data from the maternal grandparents could be used to partially reconstruct missing or poorly measured maternal haploid data.

Note the recursive nature of this method: given the naturally noisy measurement of single cell parental haploid data, along with the diploid and/or haploid data of the appropriate grandparents, the disclosed method could be used to clean the parental haploid data, which in turn will provide more accurate genotyping of the embryo. It should be obvious to one skilled in the arts how to modify the method for use in these cases.

It is preferable to use more information rather than less, as this can increase the chances of making the right call at a given SNP, and can increase the confidence in those calls. This must be balanced with the increasing complexity of the system as additional techniques and sources of data are used. There are many sources of additional information, as well as techniques available to use the information to augment the data. For example, there are informatics based approaches which take advantage of correlations which can be found in Hapmap data, or other repositories of genomic data. In addition there are biological approaches which can allow for the direct measurement of genetic data that otherwise would need to be recreated in silico. For example, haploid data otherwise unavailable may be measureable by extracting individual chromosomes from diploid cells using flow cytometry techniques to isolate fluorescently tagged chromosomes. Alternately, one may use cell fusion to create monoallelic hybrid cells to effect diploid to haploid conversion.

*Application of the disclosed method to selecting which embryo is likely to implant*

In one embodiment, the system can be used to determine the likelihood of an embryo to implant in the mother and develop into a baby. To the extent that the likelihood of the embryo implanting is determined by SNPs of the embryo, and/or their relation to SNPs of the mother, the disclosed method will be important in helping the selection of embryos, based on making a reliable prediction of which will successfully implant based on the clean SNP data. To best predict the likelihood it will be necessary to take into account the determined genotype of the embryo possibly combined with the levels of gene expression in the embryo, the levels of gene expression in the mother, and/or the determined genotype of the mother.

In addition, it is well known that aneuploid embryos are less likely to implant, less likely to result in a successful pregnancy, and less likely to result in a healthy child. Consequently, screening for aneuploides is an important facet to selecting the embryo that is most likely to result in a successful outcome. More detail on this approach is given below.

*Deducing Parental Haploid Data*

In one embodiment of the method, it may be necessary to deduce parental haplotypes, given detailed knowledge of the diploid data of a parent. There are multiple ways this can be done. In the simplest case, haplotypes have already been inferred by molecular assay of single haploid cells of a direct relation (mother, father, son or daughter). In this case, it is a trivial matter to one skilled in the art to deduce the sister haplotype by subtracting the known haplotype from the diploid genotype measured by molecular assay. For example, if a particular locus is heterozygous, an unknown parental haplotype is the opposite allele from the known parental haplotype.

In another case, the noisy haploid data of the parent may be known from molecular biological haplotyping of individual parental haploid cells, such as a sperm cell, or from individual chromosomes, which may be isolated by various methods including magnetic beads and flow cytometry. In this case, the same procedure can be used as above, except that the determined haplotype will be as noisy as the measured haplotype.

There are also methods for deducing haploid data sets directly from diploid data, using statistical methods that utilize known haplotype blocks in the general population (such as those created for the public Hapmap project). A haplotype block is essentially a series of correlated alleles that occur repeatedly in a variety of populations. Since these haplotype blocks are often ancient and common, they may be used to predict haplotypes from diploid genotypes. The parents' inferred haplotype blocks can then be used as input for the method described herein to clean the noisy data from the embryos. Publicly available algorithms that would accomplish this task include an imperfect phylogeny approach, Bayesian approaches based on conjugate priors, and priors from population genetics. Some of these algorithms use hidden Markov models. One study used public trio and unrelated individual data to demonstrate that these algorithms perform with error rates as low as 0.05% across 1MB of sequence. However, as expected, accuracy is lower for individuals with rare haplotype blocks. In one estimate, computational methods failed to phase as many as 5.1% of loci with minor allele frequency of 20%.

Genetic data from multiple blastomeres taken from different embryos during an IVF cycle may be used to infer the haplotype blocks of the parents with greater reliability.

*Techniques for Screening Aneuploidy using High and Medium Throughput Genotyping*

In one embodiment of the system the measured genetic data can be used to detect for the presence of aneuploides and/or mosaicism in an individual. Disclosed herein are several methods of using medium or high-throughput genotyping to detect the number of chromosomes or DNA segment copy number from amplified or unamplified DNA from tissue samples. The goal is to estimate the reliability that can be achieved in detecting certain types of aneuploidy and levels of mosaicism using different quantitative and/or qualitative genotyping platforms such as ABI Taqman, MIPS, or Microarrays from Illumina, Agilent and Affymetrix. In many of these cases, the genetic material is amplified by PCR before hybridization to probes on the genotyping array to detect the presence of particular alleles. How these assays are used for genotyping is described elsewhere in this disclosure.

Described below are several methods for screening for abnormal numbers of DNA segments, whether arising from deletions, aneuploides and/or mosaicism. The methods are grouped as follows: (i) quantitative techniques without making allele calls; (ii) qualitative techniques that leverage allele calls; (iii) quantitative techniques that leverage allele calls; (iv) techniques that use a probability distribution function for the amplification of genetic data at each locus. All methods involve the measurement of multiple loci on a given segment of a given chromosome to determine the number of instances of the given segment in the genome of the target individual. In addition, the methods involve creating a set of one or more hypotheses about the number of instances of the given segment; measuring the amount of genetic data at multiple loci on the given segment; determining the relative probability of each of the hypotheses given the measurements of the target individual's genetic data; and using the relative probabilities associated with each hypothesis to determine the number of instances of the given segment. Furthermore, the methods all involve creating a combined measurement M that is a computed function of the measurements of the amounts of genetic data at multiple loci. In all the methods, thresholds are determined for the selection of each hypothesis $H_i$ based on the measurement M, and the number of loci to be measured is estimated, in order to have a particular level of false detections of each of the hypotheses.

The probability of each hypothesis given the measurement M is $P(H_i|M)= P(M|H_i)P(H_i)/P(M)$. Since $P(M)$ is independent of $H_i$, we can determine the relative probability of the hypothesis given M by considering only $P(M|H_i)P(H_i)$. In what follows, in order to simplify the analysis and the comparison of different techniques, we assume that $P(H_i)$ is the same for all $\{H_i\}$, so that we can compute the relative probability of all the $P(H_i|M)$ by considering only $P(M|H_i)$. Consequently, our determination of thresholds and the number of loci to be measured is based on having particular probabilities of selecting false hypotheses under the assumption that $P(H_i)$ is the same for all $\{H_i\}$. It will be clear to one skilled in the art after reading this disclosure how the approach would be modified to accommodate the fact that $P(H_i)$ varies for different hypotheses in the set $\{H_i\}$. In some embodiments, the thresholds are set so that hypothesis $H_i^*$ is selected which maximizes $P(H_i|M)$ over all i. However, thresholds need not necessarily be set to maximize $P(H_i|M)$, but rather to achieve a particular ratio of the probability of false detections between the different hypotheses in the set $\{H_i\}$.

It is important to note that the techniques referred to herein for detecting aneuploides can be equally well used to detect for uniparental disomy, unbalanced translocations, and for the sexing of the chromosome (male or female; XY or XX). All of the concepts concern detecting the identity and number of chromosomes (or segments of chromosomes) present in a given sample, and thus are all addressed by the methods described in this document. It should be obvious to one skilled in the art how to extend any of the methods described herein to detect for any of these abnormalities.

*The Concept of Matched Filtering*

The methods applied here are similar to those applied in optimal detection of digital signals. It can be shown using the Schwartz inequality that the optimal approach to maximizing Signal to Noise Ratio (SNR) in the presence of normally distributed noise is to build an idealized matching signal, or matched filter, corresponding to each of the possible noise-free signals, and to correlate this matched signal with the received noisy signal. This approach requires that the set of possible signals are known as well as the statistical distribution - mean and Standard Deviation (SD) - of the noise. Herein is described the general approach to detecting whether chromosomes, or segments of DNA, are present or absent in a sample. No differentiation will be made between looking for whole chromosomes or looking for chromosome segments that have been inserted or deleted. Both will be referred to as DNA segments. It should be clear after reading this description how the techniques may be extended to many scenarios of aneuploidy and sex determination, or detecting insertions and deletions in the chromosomes of embryos, fetuses or born children. This approach can be applied to a wide range of quantitative and qualitative genotyping platforms including Taqman, qPCR, Illumina Arrays, Affymetrix Arrays, Agilent Arrays, the MIPS kit etc.

*Formulation of the General Problem*

Assume that there are probes at SNPs where two allelic variations occur, x and y. At each locus i, i=1... N, data is collected corresponding to the amount of genetic material from the two alleles. In the Taqman assay, these measures would be, for example, the cycle time, $C_t$, at which the level of each allele-specific dye crosses a threshold. It will be clear how this approach can be extended to different measurements of the amount of genetic material at each locus or corresponding to each allele at a locus. Quantitative measurements of the amount of genetic material may be nonlinear, in which case the change in the measurement of a particular locus caused by the presence of the segment of interest will depend on how many other copies of that locus exist in the sample from other DNA segments. In some cases, a technique may require linear measurements, such that the change in the measurement of a particular locus caused by the presence of the segment of interest will not depend on how many other copies of that locus exist in the sample from other DNA segments. An approach will be described for how the measurements from the Taqman or qPCR assays may be linearized, but there are many other techniques for linearizing nonlinear measurements that may be applied for different assays.

The measurements of the amount of genetic material of allele x at loci 1... N is given by data $d_x = [d_{x1}... d_{xN}]$. Similarly for allele y, $d_y = [d_{y1}... d_{yN}]$. Assume that each segment j has alleles $a_j = [a_{j1} .... a_{jN}]$ where each element $a_{ji}$ is either x or y. Describe the measurement data of the amount of genetic material of allele x as $d_x = s_x + \upsilon_x$ where $s_x$ is the signal and $\upsilon_x$ is a disturbance. The signal $s_x= [f_x(a_{11},...,a_{J1}) ... f_x(a_{JN},..., a_{JN})]$ where $f_x$ is the mapping from the set of alleles to the measurement, and J is the number of DNA segment copies. The disturbance vector $\upsilon_x$ is caused by measurement error and, in the case of nonlinear measurements, the presence of other genetic material besides the DNA segment of interest. Assume that measurement errors are normally distributed and that they are large relative to disturbances caused by nonlinearity (see section on linearizing measurements) so that $\upsilon_{xi} \approx n_{xi}$ where $n_{xi}$ has variance $\sigma_{xi}^2$ and vector $n_x$ is normally distributed $\sim N(0,R)$, $R=E(n_x n_x^T)$. Now, assume some filter h is applied to this data to perform the measurement $m_x = h^T d_x = h^T s_x + h^T \upsilon_x$. In order to maximize the ratio of signal to noise ($h^T s_x/h^T n_x$) it can be shown that h is given by the matched filter h $= \mu R^{-1} s_x$ where $\mu$ is a scaling constant. The discussion for allele x can be repeated for allele y.

*Method 1a: Measuring Aneuploidy or Sex by Quantitative Techniques that Do Not Make Allele Calls When the Mean and Standard Deviation for Each Locus is Known*

Assume for this section that the data relates to the amount of genetic material at a locus irrespective of allele value (e.g. using qPCR), or the data is only for alleles that have 100% penetrance in the population, or that data is combined on multiple alleles at each locus (see section on linearizing measurements) to measure the amount of genetic material at that locus. Consequently, in this section one may refer to data $d_x$ and ignore $d_y$. Assume also that there are two hypotheses: $h_0$ that there are two copies of the DNA segment (these are typically not identical copies), and $h_1$ that there is only 1 copy. For each hypothesis, the data may be described as $d_{xi}(h_0) = s_{xi}(h_0)+n_{xi}$ and $d_{xi}(h_1) = s_{xi}(h_1)+n_{xi}$ respectively, where $s_{xi}(h_0)$ is the expected measurement of the genetic material at locus i (the expected signal) when two DNA segments are present and $s_{xi}(h_1)$ is the expected data for one segment. Construct the measurement for each locus by differencing out the expected signal for hypothesis $h_0$: $m_{xi} = d_{xi}-s_{xi}(h_0)$. If $h_1$ is true, then the expected value of the measurement is $E(m_{xi}) = s_{xi}(h_1)-s_{xi}(h_0)$. Using the matched filter concept discussed above, set $h = (1/N)R^{-1}(s_{xi}(h_1)-s_{xi}(h_0))$. The measurement is described as $m = h^T d_x = (1/N)\Sigma_{i=1...N}((s_{xi}(h_1)-s_{xi}(h_0))/\sigma_{xi}^2)m_{xi}$.

If $h_1$ is true, the expected value of $E(m|h_1) = m_1 = (1/N)\Sigma_{i=1...N}(s_{xi}(h_1)-s_{xi}(h_0))^2/\sigma_{xi}^2$ and the standard deviation of m is $\sigma_{m|h1}^2 = (1/N^2)\Sigma_{i=1...N}((s_{xi}(h_1)-s_{xi}(h_0))^2/\sigma_{xi}^4)\sigma_{xi}^2 = (1/N^2)\Sigma_{i=1...N}(s_{xi}(h_1)-s_{xi}(h_0))^2/\sigma_{xi}^2$.

If $h_0$ is true, the expected value of m is $E(m|h_0) = m_0 = 0$ and the standard deviation of m is again $\sigma_{m|h0}^2 = (1/N^2)\Sigma_{i=1...N}(s_{xi}(h_1)-s_{xi}(h_0))^2/\sigma_{xi}^2$.

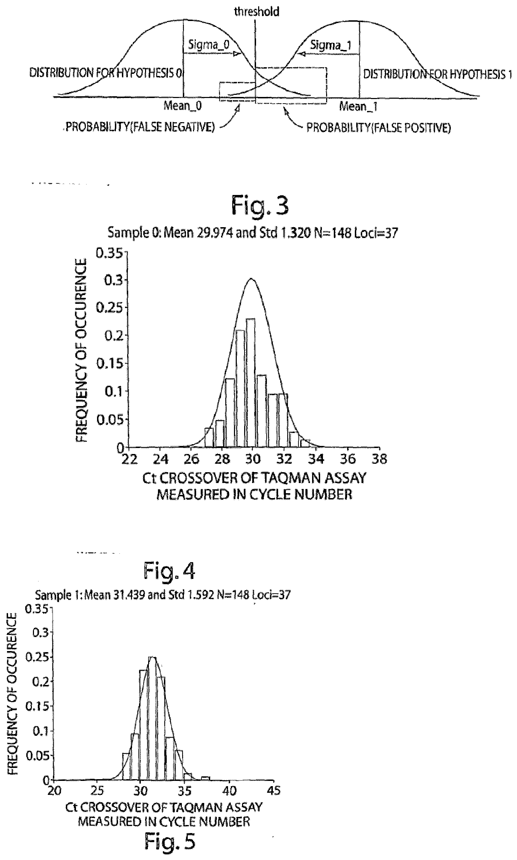

Figure 3 illustrates how to determine the probability of false negatives and false positive detections. Assume that a threshold t is set half-way between $m_1$ and $m_0$ in order to make the probability of false negatives and false positives equal (this need not be the case as is described below). The probability of a false negative is determined by the ratio of $(m_1-t)/\sigma_{m|h1}=(m_0-m_0)/(2\sigma_{m|h1})$. "5-Sigma" statistics may be used so that the probability of false negatives is $1-\text{normcdf}(5,0,1) = 2.87e-7$. In this case, the goal is for $(m_1-m_0)/(2\sigma_{m|h0}) > 5$ or $10\text{sqrt}((1/N^2)\Sigma_{i=1...N}(s_{xi}(h_1)-s_{xi}(h_0))^2/\sigma_{xi}^2) < (1/N)\Sigma_{i=1...N}(s_{xi}(h_1)-s_{xi}(h_0))^2/\sigma_{xi}^2$ or $\text{sqrt}(\Sigma_{i=1...N}(s_{xi}(h_1)-s_{xi}(h_0))^2/\sigma_{xi}^2) > 10$. In order to compute the size of N, Mean Signal to Noise Ratio can be computed from aggregated data: $\text{MSNR} = (1/N)\Sigma_{i=1...N}(s_{xi}(h_1)-s_{xi}(h_0))^2/\sigma_{xi}^2$. N can then be found from the inequality above: $\text{sqrt}(N).\text{sqrt}(\text{MSNR}) > 10$ or $N > 100/\text{MSNR}$.

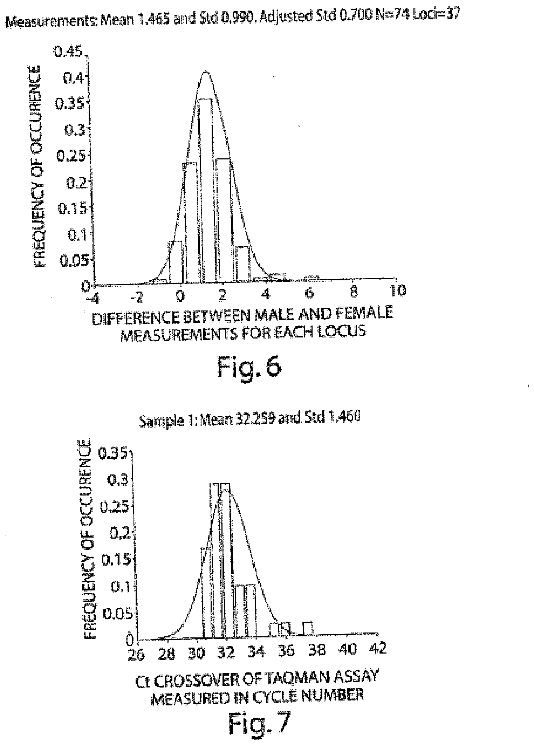

This approach was applied to data measured with the Taqman Assay from Applied BioSystems using 48 SNPs on the X chromosome. The measurement for each locus is the time, Ct, that it takes the die released in the well corresponding to this locus to exceed a threshold. Sample 0 consists of roughly 0.3ng (50 cells) of total DNA per well of mixed female origin where subjects had two X chromosomes; sample 1 consisted of roughly 0.3ng of DNA per well of mixed male origin where subject had one X chromosome. Figure 4 and Figure 5 show the histograms of measurements for samples 1 and 0. The distributions for these samples are characterized by $m_0= 29.97$; $SD_0=1.32$, $m_1=31.44$, $SD_1=1.592$. Since this data is derived from mixed male and female samples, some of the observed SD is due to the different allele frequencies at each SNP in the mixed samples. In addition, some of the observed SD will be due to the varying efficiency of the different assays at each SNP, and the differing amount of dye pipetted into each well. Figure 6 provides a histogram of the difference in the measurements at each locus for the male and female sample. The mean difference between the male and female samples is 1.47 and the SD of the difference is 0.99. While this SD will still be subject to the different allele frequencies in the mixed male and female samples, it will no longer be affected the different efficiencies of each assay at each locus. Since the goal is to differentiate two measurements each with a roughly similar SD, the adjusted SD may be approximated for each measurement for all loci as 0.99/sqrt(2)=0.70. Two runs were conducted for every locus in order to estimate $\sigma_{xi}$ for the assay at that locus so that a matched filter could be applied. A lower limit of $\sigma_{xi}$ was set at 0.2 in order to avoid statistical anomalies resulting from only two runs to compute $\sigma_{xi}$. Only those loci (numbering 37) for which there were no allele dropouts over both alleles, over both experiment runs and over both male and female samples were used in the plots and calculations. Applying the approach above to this data, it was found that MSNR=2.26, hence N = $2^2 5^2/2.26$^2 = 17 loci.

*Method 1b: Measuring Aneuploidy or Sex by Quantitative Techniques that Do Not Make Allele Calls When the Mean and Std. Deviation is Not Known or is Uniform*

When the characteristics of each locus are not known well, the simplifying assumptions that all the assays at each locus will behave similarly can be made, namely that $E(m_{xi})$ and $\sigma_{xi}$ are constant across all loci i, so that it is possible to refer instead only to $E(m_x)$ and $\sigma_x$. In this case, the matched filtering approach m= $h^T d_x$ reduces to finding the mean of the distribution of $d_x$. This approach will be referred to as comparison of means, and it will be used to estimate the number of loci required for different kinds of detection using real data.

As above, consider the scenario when there are two chromosomes present in the sample (hypothesis $h_0$) or one chromosome present ($h_1$). For $h_0$, the distribution is $N(\mu_0, \sigma_0^2)$ and for $h_1$ the distribution is $N(\mu_1, \sigma_1^2)$. Measure each of the distributions using $N_0$ and $N_1$ samples respectively, with measured sample means and SDs: $m_1, m_0, s_1,$ and $s_0$. The means can be modeled as random variables $M_0, M_1$ that are normally distributed as $M_0 \sim N(\mu_0, \sigma_0^2/N_0)$ and $M_1 \sim N(\mu_1, \sigma_1^2/N_1)$. Assume $N_1$ and $N_0$ are large enough (> 30) so that one can assume that $M_1 \sim N(m_1, s_1^2/N_1)$ and $M_0 \sim N(m_0, s_0^2/N_0)$. In order to test whether the distributions are different, the difference of the means test may be used, where d = $m_1$-$m_0$. The variance of the random variable D is $\sigma_d^2 = \sigma_1^2/N_1 + \sigma_0^2/N_0$ which may be approximated as $\sigma_d^2 = s_1^2/N_1 + s_0^2/N_0$. Given $h_0$, E(d) = 0; given $h_1$, E(d)=$\mu_1$-$\mu_0$. Different techniques for making the call between $h_1$ for $h_0$ will now be discussed.

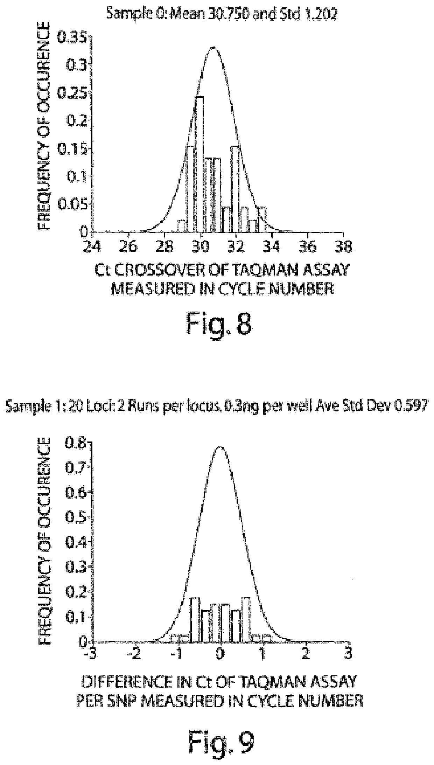

Data measured with a different run of the Taqman Assay using 48 SNPs on the X chromosome was used to calibrate performance. Sample 1 consists of roughly 0.3ng of DNA per well of mixed male origin containing one X chromosome; sample 0 consisted of roughly 0.3ng of DNA per well of mixed female origin containing two X chromosomes. $N_1$ = 42 and $N_0$ = 45. Figure 7 and Figure 8 show the histograms for samples 1 and 0. The distributions for these samples are characterized by $m_1$=32.259, $s_1$=1.460, ($\sigma_{m1}=s_1$/sqrt($N_1$)=0.225; $m_0$= 30.75; $s_0$=1.202, $\sigma_{m0}=s_0$/sqrt($N_0$)=0.179. For these samples d=1.509 and $\sigma_d$=0.2879.

Since this data is derived from mixed male and female samples, much of the standard deviation is due to the different allele frequencies at each SNP in the mixed samples. SD is estimated by considering the variations in Ct for one SNP at a time, over multiple runs. This data is shown in Figure 9. The histogram is symmetric around 0 since $C_t$ for each SNP is measured in two runs or experiments and the mean value of Ct for each SNP is subtracted out. The average std. dev. across 20 SNPs in the mixed male sample using two runs is s=0.597. This SD will be conservatively used for both male and female samples, since SD for the female sample will be smaller than for the male sample. In addition, note that the measurement from only one dye is being used, since the mixed samples are assumed to be heterozygous for all SNPs. The use of both dyes requires the measurements of each allele at a locus to be combined, which is more complicated (see section on linearizing measurements). Combining measurements on both dyes would double signal amplitude and increase noise amplitude by roughly sqrt(2), resulting in an SNR improvement of roughly sqrt(2) or 3dB.

*Detection Assuming No Mosaicism and No Reference Sample*

Assume that $m_0$ is known perfectly from many experiments, and every experiment runs only one sample to compute $m_1$ to compare with $m_0$. $N_1$ is the number of assays and assume that each assay is a different SNP locus. A threshold t can be set half way between $m_0$ and $m_1$ to make the likelihood of false positives equal the number of false negatives, and a sample is labeled abnormal if it is above the threshold. Assume $s_1 = s_2 = s = 0.597$ and use the 5-sigma approach so that the probability of false negatives or positives is 1-normcdf(5,0,1) = 2.87e-7. The goal is for 5$s_1$/sqrt($N_1$) < ($m_1$-$m_0$)/2, hence $N_1$ = 100 s $_1^2/(m_1-m_0)^2$ = 16. Now, an approach where the probability of a false positive is allowed to be higher than the probability of a false negatives, which is the harmful scenario, may also be used. If a positive is measured, the experiment may be rerun. Consequently, it is possible to say that the probability of a false negative should be equal to the square of the probability of a false positive. Consider Figure 3, let t = threshold, and assume Sigma_0 = Sigma_1 = s. Thus (1-normcdf((t-$m_0$)/s,0,1))^2 = 1-normcdf(($m_1$-t)/s,0,1). Solving this, it can be shown that t = $m_0$+0.32($m_1$-$m_0$). Hence the goal is for 5s/sqrt($N_1$)<$m_1$-$m_0$-0.32($m_1$-$m_0$) = ($m_1$-$m_0$)/1.47, hence $N_1$ = ($5^2$)($1.47^2$)s$^2/(m_1-m_0)^2$ = 9.

*Detection with Mosaicism without Running a Reference Sample*

Assume the same situation as above, except that the goal is to detect mosaicism with a probability of 97.7% (i.e. 2-sigma approach). This is better than the standard approach to amniocentesis which extracts roughly 20 cells and photographs them. If one assumes that 1 in 20 cells is aneuploid and this is detected with 100% reliability, the probability of having at least one of the group being aneuploid using the standard approach is $1 - 0.95^{20} = 64\%$. If 0.05% of the cells are aneuploid (call this sample 3) then $m_3 = 0.95 m_0 + 0.05 m_1$ and $var(m_3) = (0.95 s_0^2 + 0.05 s_1^2)/N_1$. Thus $std(m_3) < (m_3 - m_0)/2$ => $sqrt(0.95 s_0^2 + 0.05 s_1^2)/sqrt(N_1) < 0.05(m_1 - m_2)/4$ => $N_1 = 16(0.95 s_2^2 + 0.05 s_1^2)/(0.05^2 (m_1 - m_2)^2) = 1001$. Note that using the goal of 1-sigma statistics, which is still better than can be achieved using the conventional approach (i.e. detection with 84.1% probability), it can be shown in a similar manner that $N_1 = 250$.

*Detection with No Mosaicism and Using a Reference Sample*

Although this approach may not be necessary, assume that every experiment runs two samples in order to compare $m_1$ with truth sample $m_2$. Assume that $N = N_1 = N_0$. Compute $d = m_1 - m_0$ and, assuming $\sigma_1 = \sigma_0$, set a threshold $t = (m_0 + m_1)/2$ so that the probability of false positives and false negatives is equal. To make the probability of false negatives 2.87e-7, it must be the case that $(m1 - m2)/2 > 5 sqrt(s_1^2/N + s_2^2/N)$ => $N = 100(s_1^2 + s_2^2)/(m1 - m2)^2 = 32$.

*Detection with Mosaicism and Running a Reference Sample*

As above, assume the probability of false negatives is 2.3% (i.e. 2-sigma approach). If 0.05% of the cells are aneuploid (call this sample 3) then $m_3 = 0.95 m_0 + 0.05 m_1$ and $var(m_3) = (0.95 s_0^2 + 0.05 s_1^2)/N_1$. $d = m_3 - m_2$ and $\sigma_d^2 = (1.95 s_0^2 + 0.05 s_1^2)/N$. It must be that $std(m_3) 2 < (m_0 - m_2)/2$ => $sqrt(1.95 s_2^2 + 0.05 s_1^2)/sqrt(N) < 0.05(m_1 - m_2)/4$ => $N = 16(1.95 s_2^2 + 0.05 s_1^2)/(0.05^2 (m_1 - m_2)^2) = 2002$. Again using 1-sigma approach, it can be shown in a similar manner that $N = 500$.

Consider the case if the goal is only to detect 5% mosaicism with a probability of 64% as is the current state of the art. Then, the probability of false negative would be 36%. In other words, it would be necessary to find x such that $1 - normcdf(x,0,1) = 36\%$. Thus $N = 4(0.36^2)(1.95 s_2^2 + 0.05 s_1^2)/(0.05^2 (m_1 - m_2)^2) = 65$ for the 2-sigma approach, or $N = 33$ for the 1-sigma approach. Note that this would result in a very high level of false positives, which needs to be addressed, since such a level of false positives is not currently a viable alternative.

Also note that if N is limited to 384 (i.e. one 384 well Taqman plate per chromosome), and the goal is to detect mosaicism with a probability of 97.72%, then it will be possible to detect mosaicism of 8.1% using the 1-sigma approach. In order to detect mosaicism with a probability of 84.1% (or with a 15.9% false negative rate), then it will be possible to detect mosaicism of 5.8% using the 1-sigma approach. To detect mosaicism of 19% with a confidence of 97.72% it would require roughly 70 loci. Thus one could screen for 5 chromosomes on a single plate.

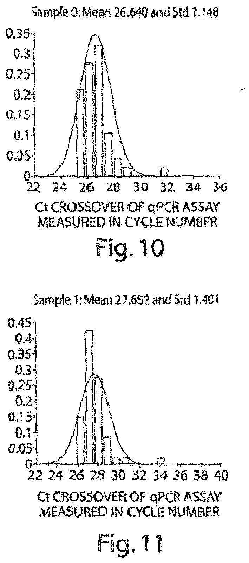

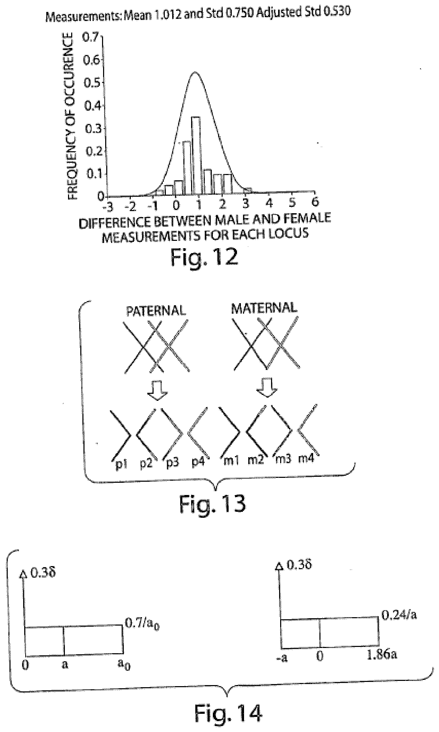

The summary of each of these different scenarios is provided in Table 2. Also included in this table are the results generated from qPCR and the SYBR assays. The methods described above were used and the simplifying assumption was made that the performance of the qPCR assay for each locus is the same. Figure 10 and Figure 11 show the histograms for samples 1 and 0, as described above. $N_0 = N_1 = 47$. The distributions of the measurements for these samples are characterized by $m_1 = 27.65$, $s_1 = 1.40$, ($\sigma_{m1} = s_1/sqrt(N_1) = 0.204$; $m_0 = 26.64$; $s_0 = 1.146$, $\sigma_{m0} = s_0/sqrt(N_0) = 0.167$). For these samples $d = 1.01$ and $\sigma_d = 0.2636$. Figure 12 shows the difference between $C_t$ for the male and female samples for each locus, with a standard deviation of the difference over all loci of 0.75. The SD was approximated for each measurement of each locus on the male or female sample as $0.75/sqrt(2) = 0.53$.

*Method 2: Qualitative Techniques that Use Allele Calls*

In this section, no assumption is made that the assay is quantitative. Instead, the assumption is that the allele calls are qualitative, and that there is no meaningful quantitative data coming from the assays. This approach is suitable for any assay that makes an allele call. Figure 13 describes how different haploid gametes form during meiosis, and will be used to describe the different kinds of aneuploidy that are relevant for this section. The best algorithm depends on the type of aneuploidy that is being detected.

Consider a situation where aneuploidy is caused by a third segment that has no section that is a copy of either of the other two segments. From Figure 13, the situation would arise, for example, if $p_1$ and $p_4$, or $p_2$ and $p_3$, both arose in the child cell in addition to one segment from the other parent. This is very common, given the mechanism which causes aneuploidy. One approach is to start off with a hypothesis $h_0$ that there are two segments in the cell and what these two segments are. Assume, for the purpose of illustration, that $h_0$ is for $p_3$ and $m_4$ from Figure 13 In a preferred embodiment this hypothesis comes from algorithms described elsewhere in this document. Hypothesis $h_1$ is that there is an additional segment that has no sections that are a copy of the other segments. This would arise, for example, if $p_2$ or $m_1$ was also

present. It is possible to identify all loci that are homozygous in $p_3$ and $m_4$. Aneuploidy can be detected by searching for heterozygous genotype calls at loci that are expected to be homozygous.

Assume every locus has two possible alleles, x and y. Let the probability of alleles x and y in general be $p_x$ and $p_y$ respectively, and $p_x+p_y=1$. If $h_1$ is true, then for each locus i for which $p_3$ and $m_4$ are homozygous, then the probability of a non-homozygous call is $p_y$ or $p_x$, depending on whether the locus is homozygous in x or y respectively. Note: based on knowledge of the parent data, i.e. $p_1$, $p_2$, $p_4$ and $m_1$, $m_2$, $m_3$, it is possible to further refine the probabilities for having non-homozygous alleles x or y at each locus. This will enable more reliable measurements for each hypothesis with the same number of SNPs, but complicates notation, so this extension will not be explicitly dealt with. It should be clear to someone skilled in the art how to use this information to increase the reliability of the hypothesis.

The probability of allele dropouts is $p_d$. The probability of finding a heterozygous genotype at locus i is $p_{0i}$ given hypothesis $h_0$ and $p_{1i}$ given hypothesis $h_1$.

Given $h_0$: $p_{0i} = 0$
Given $h_1$: $p_1 i = p_x(1-p_d)$ or $p_{1i} = p_y(1-p_d)$ depending on whether the locus is homozygous for x or y.

Create a measurement $m = 1/N_h \Sigma_{i=1...Nh} I_i$ where $I_i$ is an indicator variable, and is 1 if a heterozygous call is made and 0 otherwise. $N_h$ is the number of homozygous loci. One can simplify the explanation by assuming that $p_x=p_y$ and $p_{0i}$, $p_{1i}$ for all loci are the same two values $p_0$ and $p_1$. Given $h_0$, $E(m) = p_0 = 0$ and $\sigma^2_{m|h0} = p_0(1-p_0)/N_h$. Given $h_1$, $E(m) = p_1$ and $\sigma^2_{m|h1} = p_1(1-p_1)/N_h$. Using 5 sigma-statistics, and making the probability of false positives equal the probability of false negatives, it can be shown that $(p_1-p_0)/2 > 5\sigma_{m|h1}$ hence $N_h = 100(p_0(1-p_0)+p_1(1-p_1))/(p_1-p_0)^2$. For 2-sigma confidence instead of 5-sigma confidence, it can be shown that $N_h = 4.2^2(p_0(1-p_0)+p_1(1-p_1))/(p_1-p_0)^2$.

It is necessary to sample enough loci N that there will be sufficient available homozygous loci $N_{h\text{-avail}}$ such that the confidence is at least 97.7% (2-sigma). Characterize $N_{h\text{-avail}} = \Sigma_{i=1...N} J_i$ where $J_i$ is an indicator variable of value 1 if the locus is homozygous and 0 otherwise. The probability of the locus being homozygous is $p_x^2+p_y^2$. Consequently, $E(N_{h\text{-avail}}) =N(p_x^2+p_y^2)$ and $\sigma_{Nh\text{-avail}}^2= N(p_x^2+p_y^2)(1- p_x^2-p_y^2)$. To guarantee N is large enough with 97.7% confidence, it must be that $E(N_{h\text{-avail}}) - 2\sigma_{Nh\text{-avail}} = N_h$ where $N_h$ is found from above.

For example, if one assumes $p_d = 0.3$, $p_x = p_y = 0.5$, one can find $N_h = 186$ and $N = 391$ for 5-sigma confidence. Similarly, it is possible to show that $N_h = 30$ and $N = 68$ for 2-sigma confidence i.e. 97.7% confidence in false negatives and false positives.

Note that a similar approach can be applied to looking for deletions of a segment when $h_0$ is the hypothesis that two known chromosome segment are present, and $h_1$ is the hypothesis that one of the chromosome segments is missing. For example, it is possible to look for all of those loci that should be heterozygous but are homozygous, factoring in the effects of allele dropouts as has been done above.

Also note that even though the assay is qualitative, allele dropout rates may be used to provide a type of quantitative measure on the number of DNA segments present.

*Method 3: Making use of Known Alleles of Reference Sequences, and Quantitative Allele Measurements*

Here, it is assumed that the alleles of the normal or expected set of segments are known. In order to check for three chromosomes, the first step is to clean the data, assuming two of each chromosome. The data cleaning in the first step is done using methods described elsewhere in this document. Then the signal associated with the expected two segments is subtracted from the measured data. One can then look for an additional segment in the remaining signal. A matched filtering approach is used, and the signal characterizing the additional segment is based on each of the segments that are believed to be present, as well as their complementary chromosomes. For example, considering Figure 13, if the results of PS indicate that segments p2 and m1 are present, the technique described here may be used to check for the presence of p2, p3, m1 and m4 on the additional chromosome. If there is an additional segment present, it is guaranteed to have more than 50% of the alleles in common with at least one of these test signals. Note that another approach, not described in detail here, is to use an algorithm described elsewhere in the document to clean the data, assuming an abnormal number of chromosomes, namely 1, 3, 4 and 5 chromosomes, and then to apply the method discussed here. The details of this approach should be clear to someone skilled in the art after having read this document.

Hypothesis $h_0$ is that there are two chromosomes with allele vectors $a_1$, $a_2$. Hypothesis $h_1$ is that there is a third chromosome with allele vector $a_3$. Using a method described in this document to clean the genetic data, or another technique, it is possible to determine the alleles of the two segments expected by $h_0$: $a_1 = [a_{11} ... a_{1N}]$ and $a_2 = [a_{21} ... a_{2N}]$ where each element $a_{ji}$ is either x or y. The expected signal is created for hypothesis $h_0$: $s_{0x} = [f_{0x}(a_{11}, a_{21}) ... f_{x0}(a_{1N}, a_{2N})]$, $s_{0y} = [f_y(a_{11}, a_{21}) ... f_y(a_{1N}, a_{2N})]$ where $f_x$, $f_y$ describe the mapping from the set of alleles to the measurements of each allele. Given $h_0$, the data may be described as $d_{xi} = s_{0xi}+n_{xi}$, $n_{xi}\sim N(0,\sigma_{xi}^2)$; $d_{yi} = s_{0yi}+n_{yi}$, $n_{yi}\sim N(0,\sigma_{yi}^2)$. Create a measurement by differencing the data and the reference signal: $m_{xi}=d_{xi}-s_{xi}$; $m_{yi}=d_{yi}-s_{yi}$. The full measurement vector is $m=[m_x^T\ m_y^T]^T$.

Now, create the signal for the segment of interest - the segment whose presence is suspected, and will be sought in the residual - based on the assumed alleles of this segment: $a_3 = [a_{31} \dots a_{3N}]$. Describe the signal for the residual as: $s_r = [s_{rx}^T \; s_{ry}^T]^T$ where $s_{rx} = [f_{rx}(a_{31}) \dots f_{rx}(a_{3N})]$, $s_{ry} = [f_{ry}(a_{31}) \dots f_{ry}(a_{3N})]$ where $f_{rx}(a_{3i}) = \delta_{xi}$ if $a_{3i} = x$ and 0 otherwise, $f_{ry}(a_{3i}) = \delta_{yi}$ if $a_{3i} = y$ and 0 otherwise. This analysis assumes that the measurements have been linearized (see section below) so that the presence of one copy of allele $x$ at locus $i$ generates data $\delta_{xi} + n_{xi}$ and the presence of $\kappa_x$ copies of the allele $x$ at locus $i$ generates data $\kappa_x \delta_{xi} + n_{xi}$. Note however that this assumption is not necessary for the general approach described here. Given $h_1$, if allele $a_{3i} = x$ then $m_{xi} = \delta_{xi} + n_{xi}$, $m_{yi} = n_{yi}$ and if $a_{3i} = y$ then $m_{xi} = n_{xi}$, $m_{yi} = \delta_{yi} + n_{yi}$. Consequently, a matched filter $h = (1/N)R^{-1}s_r$ can be created where $R = \mathrm{diag}([\sigma_{x1}^2 \dots \sigma_{xN}^2 \; \sigma_{y1}^2 \dots \sigma_{yN}^2])$. The measurement is $m = h^T d$.

$$h_0: m = (1/N) \, \Sigma_{i=1..N} \, s_{rxi} n_{xi}/\sigma_{xi}^2 + s_{ryi} n_{yi}/\sigma_{yi}^2$$

$$h_1: m = (1/N) \, \Sigma_{i=1..N} \, s_{rxi}(\delta_{xi} + n_{xi})/\sigma_{xi}^2 + s_{ryi}(\delta_{yi} + n_{yi})/\sigma_{yi}^2$$

In order to estimate the number of SNPs required, make the simplifying assumptions that all assays for all alleles and all loci have similar characteristics, namely that $\delta_{xi} = \delta_{yi} = \delta$ and $\sigma_{xi} = \sigma_{yi} = \sigma$ for $i = 1 \dots N$. Then, the mean and standard deviation may be found as follows:

$$h_0: E(m) = m_0 = 0; \; \sigma_{m|h0}^2 = (1/N^2\sigma^4)(N/2)(\sigma^2\delta^2 + \sigma^2\delta^2) = \delta^2/(N\sigma^2)$$

$$h_1: E(m) = m_1 = (1/N)(N/2\sigma^2)(\delta^2 + \delta^2) = \delta^2/\sigma^2; \; \sigma_{m|h1}^2 = (1/N^2\sigma^4)(N)(\sigma^2\delta^2) = \delta^2/(N\sigma^2)$$

Now compute a signal-to-noise ratio (SNR) for this test of $h_1$ versus $h_0$. The signal is $m_1 - m_0 = \delta^2/\sigma^2$, and the noise variance of this measurement is $\sigma_{m|h0}^2 + \sigma_{m|h1}^2 = 2\delta^2/(N\sigma^2)$. Consequently, the SNR for this test is $(\delta^4/\sigma^4)/(2\delta^2/(N\sigma^2)) = N\delta^2/(2\sigma^2)$.

Compare this SNR to the scenario where the genetic information is simply summed at each locus without performing a matched filtering based on the allele calls. Assume that $h = (1/N)\bar{\imath}$ where $\bar{\imath}$ is the vector of $N$ ones, and make the simplifying assumptions as above that $\delta_{xi} = \delta_{yi} = \delta$ and $\sigma_{xi} = \sigma_{yi} = \sigma$ for $i = 1 \dots N$. For this scenario, it is straightforward to show that if $m = h^T d$:

$$h_0: E(m) = m_0 = 0; \; \sigma_{m|h0}^2 = N\sigma^2/N^2 + N\sigma^2/N^2 = 2\sigma^2/N$$

$$h_1: E(m) = m_1 = (1/N)(N\delta/2 + N\delta/2) = \delta; \; \sigma_{m|h1}^2 = (1/N^2)(N\sigma^2 + N\sigma^2) = 2\sigma^2/N$$

Consequently, the SNR for this test is $N\delta^2/(4\sigma^2)$. In other words, by using a matched filter that only sums the allele measurements that are expected for segment $a_3$, the number of SNPs required is reduced by a factor of 2. This ignores the SNR gain achieved by using matched filtering to account for the different efficiencies of the assays at each locus.

Note that if we do not correctly characterize the reference signals $s_{xi}$ and $s_{yi}$ then the SD of the noise or disturbance on the resulting measurement signals $m_{xi}$ and $m_{yi}$ will be increased. This will be insignificant if $\delta \ll \sigma$, but otherwise it will increase the probability of false detections. Consequently, this technique is well suited to test the hypothesis where three segments are present and two segments are assumed to be exact copies of each other. In this case, $s_{xi}$ and $s_{yi}$ will be reliably known using techniques of data cleaning based on qualitative allele calls described elsewhere. In one embodiment method 3 is used in combination with method 2 which uses qualitative genotyping and, aside from the quantitative measurements from allele dropouts, is not able to detect the presence of a second exact copy of a segment.

We now describe another quantitative technique that makes use of allele calls. The method involves comparing the relative amount of signal at each of the four registers for a given allele. One can imagine that in the idealized case involving a single, normal cell, where homogenous amplification occurs, (or the relative amounts of amplification are normalized), four possible situations can occur: (i) in the case of a heterozygous allele, the relative intensities of the four registers will be approximately 1:1:0:0, and the absolute intensity of the signal will correspond to one base pair; (ii) in the case of a homozygous allele, the relative intensities will be approximately 1:0:0:0, and the absolute intensity of the signal will correspond to two base pairs; (iii) in the case of an allele where ADO occurs for one of the alleles, the relative intensities will be approximately 1:0:0:0, and the absolute intensity of the signal will correspond to one base pair; and (iv) in the case of an allele where ADO occurs for both of the alleles, the relative intensities will be approximately 0:0:0:0, and the absolute intensity of the signal will correspond to no base pairs.

In the case of aneuploides, however, different situations will be observed. For example, in the case of trisomy, and there is no ADO, one of three situations will occur: (i) in the case of a triply heterozygous allele, the relative intensities of the four registers will be approximately 1:1:1:0, and the absolute intensity of the signal will correspond to one base pair; (ii) in the case where two of the alleles are homozygous, the relative intensities will be approximately 2:1:0:0, and the absolute intensity of the signal will correspond to two and one base pairs, respectively; (iii) in the case where are alleles are homozygous, the relative intensities will be approximately 1:0:0:0, and the absolute intensity of the signal will correspond to three base pairs. If allele dropout occurs in the case of an allele in a cell with trisomy, one of the situations expected for a normal cell will be observed. In the case of monosomy, the relative intensities of the four registers will be approximately 1:0:0:0, and the absolute intensity of the signal will correspond to one base pair. This situation corresponds to the case of a normal cell where ADO of one of the alleles has occurred, however in the case of the normal cell, this will only be observed at a small percentage of the alleles. In the case of uniparental disomy, where two identical chromosomes are present, the relative intensities of the four registers will be approximately 1:0:0:0, and the absolute intensity of the signal will correspond to two base pairs. In the case of UPD where two different chromosomes from one parent are present, this method will indicate that the cell is normal, although further analysis of the data using other methods described in this patent will uncover this.

In all of these cases, either in cells that are normal, have aneuploides or UPD, the data from one SNP will not be adequate to make a decision about the state of the cell. However, if the probabilities of each of the above hypothesis are calculated, and those probabilities are combined for a sufficient number of SNPs on a given chromosome, one hypothesis will predominate, it will be possible to determine the state of the chromosome with high confidence.

*Methods for linearizing quantitative measurements*

Many approaches may be taken to linearize measurements of the amount of genetic material at a specific locus so that data from different alleles can be easily summed or differenced. We first discuss a generic approach and then discuss an approach that is designed for a particular type of assay.

Assume data $d_{xi}$ refers to a nonlinear measurement of the amount of genetic material of allele x at locus i. Create a training set of data using N measurements, where for each measurement, it is estimated or known that the amount of genetic material corresponding to data $d_{xi}$ is $\beta_{xi}$. The training set $\beta_{xi}$, $1=1...N$, is chosen to span all the different amounts of genetic material that might be encountered in practice. Standard regression techniques can be used to train a function that maps from the nonlinear measurement, $d_{xi}$, to the expectation of the linear measurement, $E(\beta_{xi})$. For example, a linear regression can be used to train a polynomial function of order P, such that $E(\beta_{xi}) = [1\ d_{xi}\ d_{xi}^2 .... d_{xi}^P]c$ where c is the vector of coefficients $c = [c_0\ c_1\ ...\ c_P]^T$. To train this linearizing function, we create a vector of the amount of genetic material for N measurements $\beta_x = [\beta_{x1} ... \beta_{xN}]^T$ and a matrix of the measured data raised to powers 0...P: $D = [[1\ d_{x1}\ d_{x1}^2 .... d_{x1}^P]^T\ [1\ d_{x2}\ d_{x2}^2 ... d_{x2}^P]^T\ ...\ [1\ d_{xN}\ d_{xN}^2 .... d_{xN}^P]^T]^T$. The coefficients can then be found using a least squares fit $c = (D^TD)^{-1}D^T\beta_x$.

Rather than depend on generic functions such as fitted polynomials, we may also create specialized functions for the characteristics of a particular assay. We consider, for example, the Taqman assay or a qPCR assay. The amount of die for allele x and some locus i, as a function of time up to the point where it crosses some threshold, may be described as an exponential curve with a bias offset: $g_{xi}(t) = \alpha_{xi} + \beta_{xi}\exp(\gamma_{xi}t)$ where $\alpha_{xi}$ is the bias offset, $\gamma_{xi}$ is the exponential growth rate, and $\beta_{xi}$ corresponds to the amount of genetic material. To cast the measurements in terms of $\beta_{xi}$, compute the parameter $\alpha_{xi}$ by looking at the asymptotic limit of the curve $g_{xi}(-\infty)$ and then may find $\beta_{xi}$, and $\gamma_{xi}$ by taking the log of the curve to obtain $\log(g_{xi}(t)-\alpha_{xi}) = \log(\beta_{xi}) + \gamma_{xi}t$ and performing a standard linear regression. Once we have values for $\alpha_{xi}$ and $\gamma_{xi}$, another approach is to compute $\beta_{xi}$ from the time, $t_x$, at which the threshold $g_x$ is exceeded. $\beta_{xi} = (g_x - \alpha_{xi})\exp(-\gamma_{xi}t_x)$. This will be a noisy measurement of the true amount of genetic data of a particular allele.

Whatever techniques is used, we may model the linearized measurement as $\beta_{xi} = \kappa_x\delta_{xi}+n_{xi}$ where $\kappa_x$ is the number of copies of allele x, $\delta_{xi}$ is a constant for allele x and locus i, and $n_{xi}\sim N(0, \sigma_x^2)$ where $\sigma_x^2$ can be measured empirically.

*Method 4: Using a probability distribution function for the amplification of genetic data at each locus*

The quantity of material for a particular SNP will depend on the number of initial segments in the cell on which that SNP is present. However, due to the random nature of the amplification and hybridization process, the quantity of genetic material from a particular SNP will not be directly proportional to the starting number of segments. Let $q_{s,A}$, $q_{s,G}$, $q_{s,T}$, $q_{s,C}$ represent the amplified quantity of genetic material for a particular SNP s for each of the four nucleic acids (A,C,T,G) constituting the alleles. Note that these quantities may be exactly zero, depending on the technique used for amplification. Also note that these quantities are typically measured from the intensity of signals from particular hybridization probes This intensity measurement can be used instead of a measurement of quantity, or can be converted into a quantity estimate using standard techniques. Let qs be the sum of all the genetic material generated from all alleles of a particular SNP: $q_s = q_{s,A} + q_{s,G} + q_{s,T} + q_{s,C}$. Let N be the number of segments in a cell containing the SNP s. N is typically 2, but may be 0,1 or 3 or more. For any high or medium throughput genotyping method discussed, the resulting quantity of genetic material can be represented as $q_s = (A+A_{\theta,s})N+\theta_s$ where A is the total amplification that is either estimated a-priori or easily measured

empirically, $A_{\theta,s}$ is the error in the estimate of A for the SNP s, and $\theta_s$ is additive noise introduced in the amplification, hybridization and other process for that SNP. The noise terms $A_{\theta,s}$ and $\theta_s$ are typically large enough that $q_s$ will not be a reliable measurement of N. However, the effects of these noise terms can be mitigated by measuring multiple SNPs on the chromosome. Let S be the number of SNPs that are measured on a particular chromosome, such as chromosome 21. It is possible to generate the average quantity of genetic material over all SNPs on a particular chromosome as follows:

$$q = \frac{1}{S}\sum_{s=1}^{S} q_s = NA + \frac{1}{S}\sum_{s=1}^{S} A_{\theta,s}N + \theta_s \qquad (16)$$

Assuming that $A_{\theta,s}$ and $\theta_s$ are normally distributed random variables with 0 means and variances $\sigma^2_{A\theta,S}$ and $\sigma^2_{\theta s}$, one can

model q =NA+φ where φ is a normally distributed random variable with 0 mean and variance $\frac{1}{S}\left(N^2\sigma^2_{A\theta,S} + \sigma^2_{\theta}\right)$.

Consequently, if sufficient number of SNPs are measured on the chromosome such that $S >> (N^2\sigma^2_{A\theta,S}+\sigma^2_{\theta})$, then N=q/A can be accurately estimated.

In a another embodiment, assume that the amplification is according to a model where the signal level from one SNP is s=a+α where (a+α) has a distribution that looks like the picture in Figure 14, left. The delta function at 0 models the rates of allele dropouts of roughly 30%, the mean is a, and if there is no allele dropout, the amplification has uniform distribution from 0 to $a_0$. In terms of the mean of this distribution $a_0$ is found to be $a_0$=2.86a. Now model the probability density function of α using the picture in Figure 14, right. Let $s_c$ be the signal arising from c loci; let n be the number of segments; let $\alpha_i$ be a random variable distributed according to Figure 14 that contributes to the signal from locus i; and let σ be the standard deviation for all $\{\alpha_i\}$. $s_c$=anc+$\Sigma_{i=1..nc}$ $\alpha_i$; mean($s_c$) = anc; std($s_c$) = sqrt(nc)σ. If σ is computed according to the distribution in Figure 14, right, it is found to be σ=0.907$a^2$. We can find the number of segments from n=$s_c$/(ac) and for "5-sigma statistics" we require std(n)<0.1 so std($s_c$)/(ac) = 0.1 => 0.95a.sqrt(nc)/(ac) = 0.1 so c = $0.95^2$ n/$0.1^2$ = 181.

Another model to estimate the confidence in the call, and how many loci or SNPs must be measured to ensure a given degree of confidence, incorporates the random variable as a multiplier of amplification instead of as an additive noise source, namely s=a(1+α). Taking logs, log(s) = log(a) + log(1+α). Now, create a new random variable γ=log(1+α) and *this* variable may be assumed to be normally distributed ~N(0,σ). In this model, amplification can range from very small to very large, depending on σ, but never negative. Therefore α=$e^{\gamma}$-1; and $s_c$=$\Sigma_{i=1...cn}$a(1+$\alpha_i$). For notation, mean($s_c$) and expectation value E($s_c$) are used interchangeably

$$E(s_c) = acn + aE\left(\sum_{i=1...cn}\alpha_i\right) = acn + aE\left(\sum_{i=1...cn}\alpha_i\right) = acn\left(1 + E(\alpha)\right)$$

To find E(α) the probability density function (pdf) must be found for α which is possible since α is a function of γ which has a known Gaussian pdf. $p_{\alpha}(\alpha)=p_{\gamma}(\gamma)(d\gamma/d\alpha)$. So:

$$p_{\gamma}(\gamma) = \frac{1}{\sqrt{2\pi}\sigma}e^{\frac{-\gamma^2}{2\sigma^2}} \quad \text{and} \quad \frac{d\gamma}{d\alpha} = \frac{d}{d\alpha}\left(\log(1+\alpha)\right) = \frac{1}{1+\alpha} = e^{-\gamma}$$

and:

$$p_{\alpha}(\alpha) = \frac{1}{\sqrt{2\pi}\sigma}e^{\frac{-\gamma^2}{2\sigma^2}}e^{-\gamma} = \frac{1}{\sqrt{2\pi}\sigma}e^{\frac{-(\log(1+\alpha))^2}{2\sigma^2}}\frac{1}{1+\alpha}$$

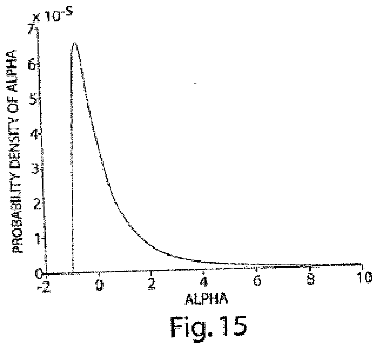

This has the form shown in Figure 15 for σ=1. Now, E(α) can be found by integrating over this pdf $E(\alpha) = \int_{-\infty}^{\infty} \alpha p_{\alpha}(\alpha)d\alpha$ which can be done numerically for multiple different σ. This gives E($s_c$) or mean($s_c$) as a function of σ. Now, this pdf can also be used to find var($s_c$):

$$\mathrm{var}(s_c) = E(s_c - E(s_c))^2 = E\left(\sum_{i=1..cn} a(1+\alpha_i) - acn - aE\left(\sum_{i=1...cn}\alpha_i\right)\right)^2$$

$$= E\left(\sum_{i=1..cn} a\alpha_i - aE\left(\sum_{i=1...cn}\alpha_i\right)\right)^2$$

$$= a^2 E\left(\sum_{i=1..cn}\alpha_i - cnE(\alpha)\right)^2$$

$$= a^2 E\left(\left(\sum_{i=1..cn}\alpha_i\right)^2 - 2cnE(\alpha)\left(\sum_{i=1..cn}\alpha_i\right) + c^2 n^2 E(\alpha)^2\right)$$

$$= a^2 E\left(cn\alpha^2 + cn(cn-1)\alpha_i\alpha_j - 2cnE(\alpha)\left(\sum_{i=1..cn}\alpha_i\right) + c^2 n^2 E(\alpha)^2\right)$$

$$= a^2 c^2 n^2 \left(E(\alpha^2) + (cn-1)E(\alpha_i\alpha_j) - 2cnE(\alpha)^2 + cnE(\alpha)^2\right)$$

$$= a^2 c^2 n^2 \left(E(\alpha^2) + (cn-1)E(\alpha_i\alpha_j) - cnE(\alpha)^2\right)$$

which can also be solved numerically using $p_\alpha(\alpha)$ for multiple different $\sigma$ to get $\mathrm{var}(s_c)$ as a function of $\sigma$. Then, we may take a series of measurements from a sample with a known number of loci c and a known number of segments n and find $std(s_c)/E(s_c)$ from this data. That will enable us to compute a value for $\sigma$. In order to estimate n, $E(s_c)=nac(1+E(\alpha))$ so

$$\hat{n} = \frac{s_c}{ac(1+E(\alpha))}$$

can be measured so that

$$std(\hat{n}) = \frac{std(s_c)}{ac(1+E(\alpha))}\ std(n)$$

When summing a sufficiently large number of independent random variables of 0-mean, the distribution approaches a Gaussian form, and thus $s_c$ (and $\hat{n}$) can be treated as normally distributed and as before we may use 5-sigma statistics:

$$std(\hat{n}) = \frac{std(s_c)}{ac(1+E(\alpha))} < 0.1$$

in order to have an error probability of 2normcdf(5,0,1) = 2.7e-7. From this, one can solve for the number of loci c.

*Sexing*

In one embodiment of the system, the genetic data can be used to determine the sex of the target individual. After the method disclosed herein is used to determine which segments of which chromosomes from the parents have contributed to the genetic material of the target, the sex of the target can be determined by checking to see which of the sex chromosomes have been inherited from the father: X indicates a female, and Y indicates a make. It should be obvious to one skilled in the art how to use this method to determine the sex of the target.

*Validation of the Hypotheses*

In some embodiments of the system, one drawback is that in order to make a prediction of the correct genetic state with the highest possible confidence, it is necessary to make hypotheses about every possible states. However, as the possible number of genetic states are exceptionally large, and computational time is limited, it may not be reasonable to test every hypothesis. In these cases, an alternative approach is to use the concept of hypothesis validation. This involves estimating limits on certain values, sets of values, properties or patterns that one might expect to observe in the measured data if a certain hypothesis, or class of hypotheses are true. Then, the measured values can tested to see if they fall within those expected limits, and/or certain expected properties or patterns can be tested for, and if the expectations are not met, then the algorithm can flag those measurements for further investigation.

For example, in a case where the end of one arm of a chromosome is broken off in the target DNA, the most likely hypothesis may be calculated to be "normal" (as opposed, for example to "aneuploid"). This is because the particular hypotheses that corresponds to the true state of the genetic material, namely that one end of the chromosome has broken off, has not been tested, since the likelihood of that state is very low. If the concept of validation is used, then the algorithm will note that a high number of values, those that correspond to the alleles that lie on the broken off section of the chromosome, lay outside the expected limits of the measurements. A flag will be raised, inviting further investigation for this case, increasing the likelihood that the true state of the genetic material is uncovered.

It should be obvious to one skilled in the art how to modify the disclosed method to include the validation technique. Note that one anomaly that is expected to be very difficult to detect using the disclosed method is balanced translocations.

*Application of the method with contaminated DNA*

In one embodiment of the system, genetic data from target DNA which has been definitely or possibly contaminated with foreign DNA can also be cleaned using the disclosed method. The concept outlined above, that of hypothesis validation, can be used to identify genetic samples that fall outside of expected limits; in the case of contaminated samples it is expected that this validation will cause a flag to be raised, and the sample can be identified as contaminated.

Since large segments of the target DNA will be known from the parental genetic data, and provided the degree of contamination is sufficiently low and sufficient SNPs are measured, the spurious data due to the foreign genetic material can be identified. The method disclosed herein should still allow for the reconstruction of the target genome, albeit with lower confidence levels. Provided that the level of contamination is sufficiently low, the hypothesis that is calculated to be most likely is still expected to correspond to the true state of the genetic material in the target DNA sample.

It should be obvious to one skilled in the art how to optimize these methods for the purpose cleaning genetic data contaminated with spurious signals due to foreign DNA.

Example of Reduction to Practice

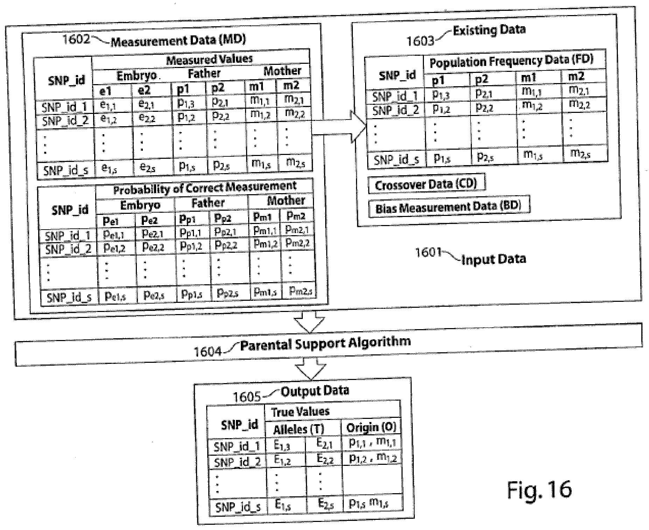

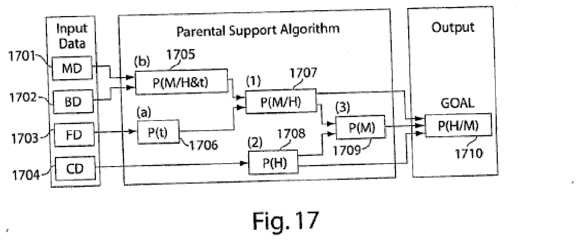

In one embodiment of the system, the method described above can be implemented using a set of algorithms which will calculate the most likely identity of each SNP in a list of relevant SNPs, as well as a confidence level for each SNP call. Described here is one possible way to implement the method disclosed in this patent. Figure 16 and Figure 17 visually represent the breakdown of this implementation of the disclosed method, the input requirements and the format of the output.

Figure 16 focuses on the input data (1601) and its format and requirements, as well as the output data (1605) and its format. Input to the algorithm consists of the measured data (1602), including input by the user, and existing data (1603) preserved in the database, that is consequently updated by the newly collected data. The measured data (MD, 1602) consists of the genetic data as measured for desired SNPs for the embryo, and the paternal and maternal alleles, as well as the accuracy, or confidence with which each of the alleles is known. The existing data (1603) consists of the population frequency data (FD), measurement bias data (BD), and crossover data (CD).

The population frequency data (FD) contains the allele frequency (for each of the values A,C,T,G) for each of the SNPs available. These data can be previously known or measured, and can be updated with newly collected data as described elsewhere in this document.

Measurement bias data (BD) captures the bias of the measurement process towards certain values. For example, assuming the true value of the allele is X=A, and probability of the correct measurement is $p_X$, the distribution of the measured value x is:

|  | A | C | T | G |
|---|---|---|---|---|
| Probability | $p_X$ | $p_C$ | $p_T$ | $p_G$ |
| probability with no bias | $p_X$ | $(1-p_X)/3$ | $(1-p_X)/3$ | $(1-p_X)/3$ |

where $p_X + p_C + p_T + p_G = 1$. If there is no bias of measurement towards any of the values then $p_C = p_T = p_G = (1-p_X)/3$. This information can be discerned from empirical and theoretical knowledge about the mechanism of the measurement process and the relevant instruments.

Crossover data (CD) consists of a database of genetic distances and crossover probabilities between pairs of snips, collected from HAPMAP data.

Together, (MD), (FD), (BD), (CD) make up the necessary input to the disclosed method (termed 'Parental Support', 1604) algorithm. This algorithm (1604) then operates on the input data to generate the output data (1605), which describes the most likely "true" value of the target's genetic data given the measured values, as well as the most likely origin of each SNP in terms of the parental alleles.

Figure 17 focuses on the structure of the algorithm itself (termed 'Parental Support') and how each of these input data are utilized by the algorithm. Working backwards: to find the most likely hypotheses it is necessary to calculate P(H|M) 1707, the probability of the hypothesis given the measurement, for all the possible hypotheses H. As described previously:

$$P(H \mid M) = \frac{P(M \mid H)}{P(M)} P(H), \;\; P(M) = \sum_{h \in S_H} P(M \mid h) P(h)$$

In order to find P(H|M) (1710), it is first necessary to find P(M|H) (1707), and P(H) (1708), for all hypotheses H. This allows the calculation of P(M), 1709 by the equation shown above. The probability of the hypothesis P(H) 1708 depends on how many crossovers are assumed and the likelihood of each of these crossovers (CD, 1704), as explained above.

$$P(M \mid H) = \sum_{t} P(M \mid H \& t) P(t)$$

P(M|H) can be calculated using the following equation: , as explained previously.

P(t), 1706 is the frequency of a particular value t for paternal and maternal alleles and is derived from population frequency data (FD, 1703). P(M|H&t), 1705 is the probability of correctly measuring the allele values of the embryo, the father, and the mother, assuming a particular "true" value t. The measurement data and accuracy entered by the user (MD, 1701), and the measurement bias database (BD, 1702) are the inputs required to calculate P(M|H&t), 1705.

A more detailed description of the method is given forthwith. Begin with SNPs R = $\{r_1,...,r_k\}$, (a set of k SNPs), and the corresponding measured identities of parents and embryo, M = $(e_1,e_2,p_1,p_2,m_1,m_2)$ , for k SNPs, identified with id's $s_1,...,s_k$, where :

$e_1 = (e_{11},e_{12},...,e_{1k})$ is the measurement on one of the chromosomes of the embryo (they don't all have to come from the same parental chromosome) for all the SNPs

$e_2 = (e_{21},e_{22},...,e_{2k})$ is the measurement on the other chromosome of the embryo

$p_1 = (p_{11},p_{12},...,p_{1k})$ is the measurement on the FIRST chromosome of the father (all coming from the same chromosome

$p_2 = (p_{21},p_{22},...,p_{2k})$ is the measurement on the SECOND chromosome of the father (all coming from the same chromosome)

$m_1 = (m_{11},m_{12},...,m_{1k})$ is the measurement on the FIRST chromosome of the mother (all coming from the same chromosome)

$m_2 = (m_{21},m_{22},...,m_{2k})$ is the measurement on the SECOND chromosome of the mother (all coming from the same chromosome)

One can also write M = $\{M_1,...,M_k\}$ where $M_i = (e_{1i},e_{2i},p_{1i},p_{2i})$

The goal of the method is to determine the "true" embryo value T = (E1,E2), i.e. the most likely case given the measurement M, where:

$E_1 = (E_{11},E_{12},...,E_{1k})$ is the measurement on the FIRST chromosome of the embryo, corresponding to the PATERNAL chromosome, $E_{1i} \in \{p_{1i},p_{2i}\}$

$E_2 = (E_{21},E_{22},...,E_{2k})$ is the measurement on the SECOND chromosome of the embryo, corresponding to the MATERNAL value, $E_{2i} \in \{m_{1i}, m_{2i}\}$

One can also write T = $\{T_1,...,T_k\}$ where $T_i = (E_{1i},E_{2i})$.

Effectively, the parental chromosome values $(p_1,p_2,m_1,m_2)$ are being used as support to check, validate and correct measured values of $(e_1,e_2)$, hence the term "Parental Support Algorithm".

To achieve this goal, all the possible hypotheses for the origin of embryo values are developed and the most likely one is chosen, given the measurement M. The hypotheses space is $S_H = \{H^1,...,H^q\}$ = {set of all the hypotheses}, where each hypothesis is of the format $H^j = (H^j_1,...H^j_k)$ where $H^j_i$ is the "mini" hypothesis for SNP i, of the format $H^j_i = (p_i^*,m_i^*)$ where $p_i^* \in \{p_{1i}, p_{2i}\}$ and $m_i^* \in \{m_{1i}, m_{2i}\}$. There are 4 different "mini" hypotheses $H^j_i$, in particular:

$H^j_i1$: $(e_{1i},e_{2i}) = \{(p_{1i},m_{1i})$ or $(m_{1i},p_{1i})\}$
$H^j_i2$: $(e_{1i},e_{2i}) = \{(p_{1i},m_{2i})$ or $(m_{2i},p_{1i})\}$
$H^j_i3$: $(e_{1i},e_{2i}) = \{(p_{2i},m_{1i})$ or $(m_{1i},p_{2i})\}$
$H^j_i4$: $(e_{1i},e_{2i}) = \{(p_{2i},m_{2i})$ or $(m_{2i},p_{2i})\}$

In theory, $S^H$ can have q = $4^k$ different members to pick from, though later this space will be limited with a maximal number of crossovers of paternal and maternal chromosomes.

The most likely hypothesis H* is chosen to be as: $H^* = \arg\max_{H \in S_H} P(H|M)$ For a particular H:

$$P(H \mid M) = \frac{P(M \mid H)}{\sum_{h \in S_H} P(M \mid h)P(h)} P(H).$$

So deriving for each hypothesis:

(1) P(M/H) is the probability of measurement M given the particular hypothesis H
(2) P(H) is the probability of the particular hypothesis H
(3) P(M) is the probability of the measurement M

After deriving P(H|M) for all H, the one with the greatest probability is chosen.

*Deriving P(M|H)*

Since measurements on each SNP are independent, for M = ($M_1$,...$M_k$) and the particular hypothesis H=($H_1$,...$H_k$) on all k SNPs then:

$$P(M \mid H) = P(M_1 \mid H_1)^* ...^* P(M_k \mid H_k)$$

For the particular SNP r, derive P($M_r$|$H_r$). For $\Omega$ = {A,C,T,G}X{A,C,T,G}X={A,C,T,G}X{A,C,T,G}, the space of all the possible values for "true" parent values ($P_{1r}$,$P_{2r}$,$M_{1r}$,$M_{2r}$), by Bayes formula is:

$$P(M_r / H_r) = \sum_{t \in \Omega} P(M_r / H_r \& (P_{1r}, P_{2r}, M_{1r}, M_{2r}) = t)^* P((P_{1r}, P_{2r}, M_{1r}, M_{2r}) = t)$$

*Deriving P($M_r$/$H_r$ & ($P_{1r}$,$P_{2r}$,$M_{1r}$,$M_{2r}$) = t)*

$M_r$ =($e_{1r}$,$e_{2r}$,$p_{1r}$,$p_{2r}$,$m_{1e}$,$m_{2r}$) is a given measurement on this SNP.

T=($E_{1r}$,$E_{2r}$,$P_{1r}$,$P_{2r}$,$M_{1r}$,$M_{2r}$) is the supposed "true" value, for t = ($P_{1r}$,$P_{2r}$,$M_{1r}$,$M_{2r}$) and ($E_{1r}$,$E_{2r}$) fixed from T by hypothesis. ($E_{1r}$ is one of $P_{1r}$,$P_{2r}$, $E_{2r}$ is one of $M_{1r}$,$M_{2r}$)

$$P(M_r = (e_{1r}, e_{2r}, p_{1r}, p_{2r}, m_{1r}, m_{2r}) / T = (E_{1r}, E_{2r}, P_{r1}, P_{2r}, M_{1r}, M_{2r})) =$$
$$P(e_{1r} / E_{1r})^* P(e_{2r} / E_{2r})^* P(p_{1r} / P_{r1})^* P(p_{2r} / P_{2r})^* P(m_{1r} / M_{1r})^* P(m_{2r} / M_{2r})$$

Given:

$p_{eri}$=P(accurately measuring the embryo value i,on SNP r)
$p_{pri}$=P(accurately measuring the father value i,on SNP r)
$p_{mri}$=P(accurately measuring the mother value i,on SNP r)

$$P(e_{1r} / E_{1r}) = \begin{cases} p_{er1} & e_{1r} = E_{1r} \\ (1-p_{er1)})^* p(e_{1r}, E_{1r}, r) & e_{1r} \neq E_{1r} \end{cases}$$
$$= I_{e_{1r}=E_{1r}} ^* p_{er1} + I_{e_{1r} \neq E_{1r}} ^* (1-p_{pr1})^* p(e_{1r}, E_{1r}, r) = F(e_{1r}, E_{1r}, p_{er1}, r)$$

where p($e_{1r}$,$E_{1r}$,r) = 1/3 if there is no measurement bias, otherwise it can be determined from experimental data, such as data from the Hapmap project..

*Deriving P(($P_{1r}$,$P_{2r}$,$M_{1r}$,$M_{2r}$) =t)*

For t =($t_1$,$t_2$,$t_3$,$t_4$):

$$P((P_{1r}, P_{2r}, M_{1r}, M_{2r}) = (t_1, t_2, t_3, t_4)) = P(P_{1r} = t_1) * P(P_{2r} = t_2) * P(M_{1r} = t_3) * P(M_{2r} = t_4)$$

Suppose there are n samples of $(P_1, P_2, M_1, M_2)$, all paternal and maternal values are assumed to be independent, and t = $(t_1, t_2, t_3, t_4)$ for $t_i$ in {A,C,T,G}

To get a particular $p_{1A} = P(P_1 = t_1)$, for $t_1$=A, assume that in absence of any data this probability could be anything between 0 and 1, so it is assigned a value of U(0,1). With the acquisition of data, this is updated with the new values and the distribution of this parameter becomes a beta distribution. Suppose that out of n observations of P1, there are h values P1=A, and w= (event $P_1$=A) and D=(data given). It is described in a prior section the form of the beta distribution B($\alpha,\beta$) with $\alpha$ = h+1, $\beta$ = n-h+1 for p(w|Data) (see equation (8)). The expected value and variance of X~B($\alpha,\beta$) distribution are:

$$EX = \frac{\alpha}{\alpha + \beta}$$

$$VX = \frac{\alpha\beta}{(\alpha + \beta)^2 (\alpha + \beta + 1)}$$

So the posterior mean value of the parameter $p_{1ra}$ = P($P_{1r}$ = A|Data) = (h+1)/(n+2)

Similarly $p_{1rB}$ = (#($p_{1r}$= B)+1)/(n+2),... $m_{2rG}$= (#($m_{2r}$ = G)+1)/(n+2), etc. Thus all the values $p_{1rA}$,...,$m_{2rG}$ have been derived and:

$$P((P_{1r}, P_{2r}, M_{1r}, M_{2r}) = (t_1, t_2, t_3, t_4)) = p_{1rt_1} * p_{2rt_2} * m_{1rt_3} * m_{2rlt_4}$$

*Deriving P(H)*

The probability of the hypothesis H = ($H_1$,...,$H_k$) with $H_i$ = ($p_i$*,$m_i$*) depends on the amount of chromosome crossover. For example,

with P(crossover) = 0 then P(H) = ¼ and H = (p*,m*) if p* in{(p11,p21,...ps1), (p12,p22,...,ps2), m* in {(m11,m21,...,ms1),(m12,m22,...,ms2)}, 0 otherwise
with P(crossover)>0 it is important to incorporate the probability of crossover between each SNP.

Hypothesis H consists of the hypothesis for paternal and maternal chromosomes for each SNP, $p_i$* $\in$ {$p_{1i}$, $p_{2i}$} and $m_i$* $\in$ {$m_{1i}$, $m_{2i}$} ,i.e. H = ($H_p$,$H_m$) where $H_p$=($p_1$*,...$p_k$*), and $H_m$=($m_1$*,... $m_k$*), which are independent. P(H) = P($H_p$)*P($H_m$). Suppose that SNP are ordered by increasing location,

$$P(H_p) = \tfrac{1}{4} \prod_{i=2}^{k} (PC_i * (1 - I_i) + (1 - PC_i) * I_i)$$

where $PC_i$ = $P(crossover(r_{i-1}, r))$ i.e. probability of crossover somewhere between SNPs $r_{i-1}, r_i$, and $I_i$ =1 if $p_i$*,$p_{i-1}$* are coming both from $p_1$ or $p_2$, and it is 0 otherwise.

*Deriving P(crossover(a, b))*

Given SNPs a,b, at base locations $l_a, l_b$ (given in bases), the probability of crossover is approximated as:

$$P(l_a, l_b) = 0.5(1 - \exp(-2G(l_a, l_b)))$$

where G($l_a,l_b$) = genetic distance in Morgans between locations $l_a, l_b$. There is no precise closed form function for G but it is loosely estimated as G($l_a,l_b$) = $||_a$-$l_b|$*1e$^{-8}$. A better approximation can be used by taking advantage of the HapMap database of base locations $s_i$, and distances G($s_i, s_{i+1}$), for i spanning over all locations. In particular,

$$G(l_a, l_b) = \sum_{l_a < s_i < l_b} G(s_i, s_{i+1})$$

, so it can be used in crossover probability.

*Deriving P(M)*

Once P(M|H) is known, P(H) can be found for all the different H in $S_H$,

$$P(M) = \sum_{H \in S_H} P(M \mid H) P(H)$$

*A more expedient method to derive the hypothesis of maximal probability*

Given the limitation of computer time, and the exponential scaling of complexity of the above method as the number of SNPs increases, in some cases it may be necessary to use more expedient methods to determine the hypothesis of maximal probability, and thus make the relevant SNP calls. A more rapid way to accomplish this follows: From before: P(H|M) = P(M|H)*P(H)/P(M), argmax $_H$ P(H|M) = argmax $_H$ and P(M|H)*P(H) = argmax $_H$ F(M,H), and the object is to find H, maximizing F(M,H).

Suppose $M_{(s,k)}$ = measurement on snips s to k, $H_{(s,k)}$ = hypothesis on snips s to k, and for shorts $M_{(k,k)} = M_k$, $H_{(k,k)} = H_k$ = measurement and hypothesis on snip k. As shown before:

$$P(M_{(1,k)} \mid H_{(1,k)}) = \prod_{i=1}^{k} P(M_i \mid H_i) = P(M_k \mid H_k) * \prod_{i=1}^{k-1} P(M_i \mid H_i) = P(M_k \mid H_k) * P(M_{(1,k-1)} \mid H_{(1,k-1)})$$

and also

$$P(H_{(1,k)}) = 1/4 * \prod_{i=2}^{k} PF(H_{i-1}, H_i) = PF(H_{k-1}, H_k) * 1/4 * \prod_{i=2}^{k-1} PF(H_{i-1}, H_i) = PF(H_{k-1}, H_k) * P(H_{(1,k-1)})$$

where

$$PF(H_{i-1}, H_i) = \begin{cases} 1 - PC(H_{i-1}, H_i) & H_{i-1} = H_i \\ PC(H_{i-1}, H_i) & H_{i-1} \neq H_i \end{cases}$$

and $PC(H_{i-1}, H_i)$ = probability of crossover between $H_{i-1}$, $H_i$

So finally, for n snips:

$$F(M, H) = P(M \mid H) * P(H) = P(M_{(1,n)} \mid H_{(1,n)}) * P(H_{(1,n)})$$
$$= P(M_{(1,n-1)} \mid H_{(1,n-1)}) * P(H_{(1,n-1)}) * P(M_n \mid H_n) * PF(H_{n-1}, H_n)$$

therefore: *F(M, H) = F(M$_{(1,n)}$, H$_{(1,n)}$)) = F(M$_{(1,n-1)}$,H$_{(1,n-1)}$)* * *P(M$_n$ | H$_n$)* * PF(H$_{n-1}$, H$_n$)* Thus, it is possible to reduce the calculation on n snips to the calculation on n-1 snips.

For H = (H$_1$,...H$_n$) hypothesis on n snips:

$$\max_{H} F(M, H) = \max_{(H_{(1,n1)}, H_n)} F(M, (H_{(1,n-1)}, H_n) = \max_{H_n} \max_{H_{(1,n-1)}} F(M, (H_{(1,n-1)}, H_n) = \max_{H_n} G(M_{(1,n)}, H_n)$$

where

$$G(M_{(1,n)}, H_n) = \max_{H_{(1,n-1)}} F(M_{(1,n)}, (H_{(1,n-1)}, H_n)) =$$

$$\max_{H_{(1,n-1)}} F(M_{(1,n-1)}, H_{(1,n-1)}) * P(M_n \mid H_n) * PF(H_{n-1}, H_n) =$$

$$P(M_n \mid H_n) * \max_{H_{(1,n-1)}} F(M_{(1,n-1)}, H_{(1,n-1)}) * PF(H_{n-1}, H_n) =$$

$$P(M_n \mid H_n) * \max_{H_{n-1}} \max_{H_{(1,n-2)}} F(M_{(1,n-1)}, (H_{(1,n-2)}, H_{n-1}) * PF(H_{n-1}, H_n) =$$

$$P(M_n \mid H_n) * \max_{H_{n-1}} PF(H_{n-1}, H_n) * G(M_{(1,n-1)}, H_{n-})$$

In summary: $\max_{H} F(M, H) = \max_{H_n} G(M_{(1,n)}, H_n)$ where G can be found recursively:, for i=2,..n

$$G(M_{(1,n)}, H_n) = P(M_n \mid H_n) * \max_{H_{n-1}} \left[ PF(H_{n-1}, H_n) * G(M_{(1,n-1)}, H_{n-1}) \right]$$

and

$$G(M_{(1,1)}, H_1) = 0.25 * P(M_1 \mid H_1).$$

The best hypothesis can be found by following the following algorithm:

Step 1: For I=1, generate 4 hypotheses for $H_1$, make $G(M_1|H_1)$ for each of these, and remember $G_1, G_2, G_3, G_4$
Step 2: For I =2: generate 4 hypothesis for $H_2$, make $G(M_{(1,2)}|H_2)$ using the above formula:

$$G(M_{(1,2)}, H_2) = P(M_2 \mid H_2) * \max_{H_1} \left[ PF(H_1, H_2) * G(M_1, H_1) \right]$$

, remember these new four $G_n$.
Repeat step 2 for I=k with $k_1 = k_{i-1}+1$ until k=n: generate 4 hypothesis for $H_k$, make $G(M_{(1,k)}|H_k)$

$$G(M_{(1,k)}, H_k) = P(M_k \mid H_k) * \max_{H_{k-1}} \left[ PF(H_{k-1}, H_k) * G(M_{(1,k-1)}, H_{k-1}) \right]$$

and remember these four $G_n$.

Since there are only four hypotheses to remember at any time, and a constant number of operations, the algorithm is linear.
To find P(M): P(HIM)= P(MIH)*P(H)/P(M) = F(M,H)/P(M))
As above:

$$P(M) = P(M_{(1,k)}) = \sum_{H_{(1,k)}} P(M_{(1,k)} \mid H_{(1,k)}) * P(H_{(1,k)})$$

$$= \sum_{H_k} P(M_K \mid H_k) \sum_{H_{(1,k-1)}} P(M_{(1,k-1)} \mid H_{(1,k-1)}) * P(H_{(1,k-1)}) * PF(H_{k-1}, H_k)$$

$$= \sum_{H_k} P(M_K \mid H_k) * W(M_{(1,k-1)} \mid H_k)$$

where $W(M_{(1,k-1)} \mid H_k) = \sum_{H_{(1,k-1)}} P(M_{(1,k-1)} \mid H_{(1,k-1)}) * P(H_{(1,k-1)}) * PF(H_{k-1}, H_k)$

W(M,H) can be solved by using recursion:

$$W(M_{(1,k-1)} \mid H_k) = \sum_{H_{(1,k-1)}} P(M_{(1,k-1)} \mid H_{(1,k-1)}) * P(H_{(1,k-1)}) * PF(H_{k-1}, H_k)$$

$$= \sum_{H_{k-1}} P(M_{k-1} \mid H_{k-1}) \sum_{H_{(1,k-2)}} P(M_{(1,k-2)} \mid H_{(1,k-2)}) * P(H_{(1,k-2)}) * PF(H_{k-2}, H_{k-1}) * PF(H_{k-1}, H_k)$$

$$= \sum_{H_{k-1}} P(M_{k-1} \mid H_{k-1}) * PF(H_{k-1}, H_k) * W(M_{(1,k-2)} \mid H_{k-1})$$

Therefore:

$$W(M_{(1,k-1)} \mid H_k) = \sum_{H_{k-1}} P(M_{k-1} \mid H_{k-1}) * PF(H_{k-1}, H_k) * W(M_{(1,k-2)} \mid H_{k-1})$$

and

$$W(M_{(1,1)} \mid H_2) = \sum_{H_1} P(M_1 \mid H_1) * 0.25 * PF(H_1, H_2)$$

The algorithm is similar to the case above, where i=2:n and in each step a new set of W(i) are generated until the final step yields the optimized W.

*Deriving the $p_1$, $p_2$, $pp_1$, $pp_2$ values from $d_1$, $d_2$, h, $pd_1$, $pd_2$, ph*

For the purpose of explanation, this section will focus on the father's diploid and haploid data, but it is important to note that the same algorithm can be applied to the mother. Let:

- $d_1$, $d_2$- allele calls on the diploid measurements
- h- allele call on the haploid measurement
- $p_{d1}$, $p_{d2}$-probabilities of a correct allele call on the each of the diploid measurements
- $p_h$- probability of a correct allele call on the haploid measurement

These data should be mapped to the following input parameters for disclosed algorithm:

- $p_1$- allele corresponding to haploid cell and one of the diploid cells
- $p_2$- allele corresponding to the remaining diploid cell
- $p_{p1}$, $p_{p2}$- probabilities of correct allele call

Since h corresponds to $d_1$, then to find the value of $p_1$ it is necessary to use h and $d_1$. Then $p_2$ will automatically correspond to $d_2$. Similarly, if h corresponds to $d_2$, then to find the value of $p_1$ it is necessary to use h and $d_2$, and thne $p_2$ will correspond to $d_1$.

The term "correspond" is used since it can mean either "be equal" or "originate with higher probability from" depending on different measurement outcomes and population frequency.

The goal of the algorithm is to calculate probabilities of "true" allele values hidden beyond results of raw measurement h, $d_1$, $d_2$, $p_h$, $p_{d1}$, $p_{d2}$ and population frequencies.

The basic algorithm steps are the following:

(i) determine whether h corresponds to $d_1$ or $d_2$ based on h, $d_1$, $d_2$, $p_h$, $p_{d1}$, $p_{d2}$ values and the population frequency data
(ii) assign the allele calls to $p_1$ and $p_2$; calculate the probabilities $p_{p1}$ and $p_{p2}$ based on step (1)

*Assigning h to $d_1$ or $d_2$*

Establish two hypotheses:

$H_1$: h corresponds to $d_1$ (h originates from $d_1$)
$H_2$: h corresponds to $d_2$ (h originates from $d_2$)

The task is to calculate probabilities of these two hypotheses given the measurement M:

$P(H_1/M(h,d_1,d_2,p_h,p_{d1},pd_2))$ and $P(H_2/M(h,d_1,d_2,p_h,p_{d1},p_{d2}))$

(To simplify the text, these will be referred to as $P(H_1/M)$ and $P(H_2/M)$) hereafter.
In order to calculate these probabilities, apply the Bayesian rule:

$$P(H_1 \mid M) = \frac{P(M \mid H_1) * P(H_1)}{P(M)} \; ; \; P(H_2 \mid M) = \frac{P(M \mid H_2) * P(H_2)}{P(M)} \; ,$$

where $P(M)=P(M/H_1)*P(H_1)+P(M/H_2)*P(H_2)$. Since hypotheses H1 and H2 are equally likely, $P(H_1)=P(H_2)=0.5$, therefore:

$$P(H_1 \mid M) = \frac{P(M \mid H_1)}{P(M \mid H_1) + P(M \mid H_2)} \; \text{and} \; P(H_2 \mid M) = \frac{P(M \mid H_2)}{P(M \mid H_1) + P(M \mid H_2)}$$

In order to calculate $P(M/H_1)$ and $P(M/H_2)$, one must consider the set of all possible values of diploid outcomes $d_1$ and $d_2$, $\Omega = \{AA,AC,...,GG\}$, i.e. any combination of A,C,T,G, so called underlying states. When the hypotheses are applied to the underlying states (i.e. accompany the assumed value of h based on hypothesis $H_1$ or $H_2$, to values $d_1$ and $d_2$), the following tables of all possible combinations (states $S=\{s_1,s_2,...,s_{16}\}$) of "true values" H, $D_1$ and $D_2$ for h, $d_1$ and $d_2$, can be generated, respectively:

| Hypothesis $H_1$: h=$d_1$ | | $\Omega=\{AA,AC,...,G\,G\}$ | | Hypothesis $H_2$: h=$d_2$ | | $\Omega =\{AA,AC,...,GG\}$ | |
|---|---|---|---|---|---|---|---|
| state | H | $D_1$ | $D_2$ | state | H | $D_1$ | $D_2$ |
| $s_1$ | A | A | A | $s_1$ | A | A | A |
| $s_2$ | A | A | C | $s_2$ | C | A | C |
| $s_3$ | A | A | T | $s_3$ | T | A | T |
| $s_4$ | A | A | G | $s_4$ | G | A | G |
| $s_5$ | C | C | A | $s_5$ | A | C | A |
| $s_6$ | C | C | C | $s_6$ | C | C | C |
| $s_7$ | C | C | T | $s_7$ | T | C | T |
| $s_8$ | C | C | G | $s_8$ | G | C | G |
| $s_9$ | T | T | A | $s_9$ | A | T | A |
| $s_{10}$ | T | T | C | $s_{10}$ | C | T | C |
| $s_{11}$ | T | T | T | $s_{11}$ | T | T | T |
| $s_{12}$ | T | T | G | $s_{12}$ | G | T | G |
| $s_{13}$ | G | G | A | $s_{13}$ | A | G | A |
| $s_{14}$ | G | G | C | $s_{14}$ | C | G | C |
| $s_{15}$ | G | G | T | $s_{15}$ | T | G | T |
| $s_{16}$ | G | G | G | $s_{16}$ | G | G | G |

Since the "true values" H, $D_1$ and $D_2$ are unknown, and only the raw measurement outcomes h, $d_1$, $d_2$, $p_h$, $p_{d1}$, $p_{d2}$, are known, the calculation of the $P(M/H_1)$ and $P(M/H_2)$ over the entire set $\Omega$ must be performed in the following manner:

$$P(M \mid H_1) = \sum_{(D_1,D_2) \in \Omega} P(M(h,d_1,d_2) \mid H_1 \& D_1, D_2) * P(D_1, D_2)$$

$$P(M \mid H_2) = \sum_{(D_1,D_2) \in \Omega} P(M(h,d_1,d_2) \mid H_2 \& D_1, D_2) * P(D_1, D_2)$$

If, for the purpose of the calculation, one assuems that $d_1$ and $d_2$, as well as $p_{d1}$ and $p_{d2}$ are independent variables, it can be shown that:

$$P(M \mid H_1) = \sum_{\Omega} P(M(h,d_1,d_2) \mid H_1 \& D_1, D_2) * P(D_1, D_2) =$$

$$\sum_{S} P(M(h) \mid H) * P(M(d_1) \mid D_1) * P(M(d_2) \mid D_2) * P(D_1) * P(D_2)$$

Consider the first three terms under the last sum above: $P(M(x)/X)$, for x in $\{h,d_1,d_2\}$.

The calculation of the probability of correct allele call (hitting the "true allele value") is based on measurement of outcome x given the true value of allele X. If the measured value x and the true value X are equal, that probability is $p_x$ (the probability of correct measurement). If x and X are different, that probability is $(1-p_x)/3$. For example, calculate the probability that the "true value" C is found under the conditions that X=C, and the measured value is x=A. The probability of getting A is $p_x$. The probability of getting C, T or G is $(1-p_x)$. So, the probability of hitting C is $(1-p_x)/3$, since one can assume that C, T and G are equally likely.

If the indicator variable $I_x$ is included in the calculation, where $I_x=1$ if x=X and $I_x=0$ if x≠X, the probabilities are as follows:

$$P(M(x)/X) = I_{\{x=X\}} * p_x + (1 - I_{\{x=X\}}) * (1/3) * (1-p_x), \text{ x in } \{h,d_1,d_2\}$$

Now consider the last two terms in $P(M|H_1)$. $P(D_1)$ and $P(D_2)$ are population frequencies of alleles A,C,T and G, that may be known from prior knowledge.

Consider the expression shown above for a particular state $s_2$, given the particular measurement $M(h = A, d_1 = G, d_2 = C)$:

$$P(M(h) \mid H) * P(M(d_1) \mid D_1) * P(M(d_2) \mid D_2) * P(D_1) * P(D_2) =$$
$$= P(M(h) = A \mid H = A) * P(M(d_1) = G \mid D_1 = A) * P(M(d_2) = C \mid D_2 = C) * P(D_1 = A) * P(D_2 = C) =$$
$$= p_h * ((1 - p_{d1})/3) * p_{d2} * f(D_1 = A) * f(D_2 = C)$$

Similarly, calculate (1) given the particular measurement (in this case $M(h=A,d_1=G,d_2=C)$) for remaining 15 states and sum over the set $\Omega$.

Now $P(M/H_1)$ and $P(M/H_2)$ have been calculated. Finally, calculate $P(H_1/M)$ and $P(H_1/M)$ as described before:

$$P(H_1 \mid M) = \frac{P(M \mid H_1)}{P(M \mid H_1) + P(M \mid H_2)}$$

$$P(H_2 \mid M) = \frac{P(M \mid H_2)}{P(M \mid H_1) + P(M \mid H_2)}$$

*Assigning the Allele Calls and Corresponding Probabilities*

Now establish four different hypotheses:

$H_{p2A}$: "true value" of $p_2$ is A
$H_{p2C}$: "true value" of $p_2$ is C
$H_{p2T}$: "true value" of $p_2$ is T
$H_{p2G}$: "true value" of $p_2$ is G
and calculate $P(H_{p2A}/M)$, $P(H_{p2C}/M)$, $P(H_{p2T}/M)$, $P(H_{p2G}/M)$. The highest value determines the particular allele call and corresponding probability.

Since the origin of $p_2$ is unknown (it is derived from $d_1$ with probability of $P(H_2/M)$ and from $d_2$ with probability $P(H_1/M)$), one must consider both cases that $p_2$ allele originates from $d_1$ or $d_2$. For Hypothesis $H_A$, applying Bayes rule, give:

$$P(H_{p2A} \mid M) = P(H_{p2A} \mid M, H_1) * P(H_1 \mid M) + P(H_{p2A} \mid M, H_2) * P(H_2 \mid M)$$

$P(H_1/M)$ and $P(H_2/M)$ have already been determined in step 1. By Bayes rule:

$$P(H_{p2A} \mid H_1, M) = \frac{P(M \mid H_1, H_{p2A}) * P(H_1, H_{p2A})}{P(H_1, M)}$$

Since $H_1$ implies that $p_2$ originates from $d_2$:

$$P(M \mid H_1, H_{p2A}) = P(M(d_2) \mid D_2 = A) = I_{\{d_2 = D_2\}} * p_{d2} + (1 - I_{\{d_2 = D_2\}}) * (1 - p_{d2})/3$$

$P(H_1,M/H_{p2A}) = P(M(d_2)/D_2 = A) = I_{\{d2=D2\}}*p_{d2} + (1 - I_{\{d2=D2\}})*(1/3)*(1-p_{d2})$, as described before. $P(H_{p2A}) = P(D_2 = A) = f_{a2}(A)$, where $f_{a2}(A)$ is obtained from population frequency data. $P(H_{1,M}) = P(H_1,M/H_{p2A})*P(H_{p2A}) + P(H_1,M/H_{p2C})*P(H_{p2C}) + P(H_1,M/H_{p2T})*P(H_{p2T}) + P(H_1,M/H_{p\,2G})*P(H_{p2G})$.
Similarly, calculate $P(H_{p2A}\&H_2/M)$.
$P(H_{p2A}/M) = P(H_{p2A}\&H_1/M) + P(H_{p2A}\&H_2/M)$, therefore the probability that $p_2$ is equal to A has been calcualted. Repeat the calculation for C,T, and G. The highest value will give the answer of $p_2$ allele call and the corresponding probability.

*Assigning the allele call to $p_1$ (allele corresponding to the haploid cell and one of the diploid cells)*

As before, we establish four different hypotheses:

$H_{p1A}$: "true value" of $p_1$ is A
$H_{p1C}$: "true value" of $p_1$ is C
$H_{p1T}$: "true value" of $p_1$ is T
$H_{p1G}$: "true value" of $p_1$ is G
and calculate $P(H_{p1A}/M)$, $P(H_{p1C}/M)$, $P(H_{p1T}/M)$, $P(H_{p1G}/M)$

Here is an elaboration of $H_{p1A}$. In the "true case" case, $p_1$ will be equal to A only if the haploid and the corresponding diploid cell are equal to A. Therefore, in order to calculate $p_1$ and $p_{p1}$ one must consider situations where haploid and corresponding diploid cell are equal. So, the hypothesis $H_{p1A}$: the "true value" of $p_1$ is A and becomes $H_{hdA}$: the "true value" of the haploid cell and corresponding diploid cell is A.

Since the origin of h is unknown (it is derived from $d_1$ with probability of $P(H_1/M)$ and from $d_2$ with probability $P(H_2/M)$), one must consider both cases, that h allele originates from $d_1$ or $d_2$, and implement that in determination of $p_1$. That means, using Bayes rule:

$$P(H_{hdA} \mid M) = P(H_{hdA} \mid M, H_1) * P(H_1 \mid M) + P(H_{hdA} \mid M, H_2) * P(H_2 \mid M)$$

As before, $P(H_1/M)$ and $P(H_2/M)$ are known from previous calculations.

$$P(H_{hdA} \mid H_1, M) = \frac{P(H_1, M \mid H_{hdA}) * P(H_{hdA})}{P(H_1, M)}$$

$$P(H_1, M/H_{hdA}) = P(M(h)/H = A)*P(M(d_1)/D_1 = A) =$$

$$= [I_{\{h=H\}}*p_h + (1-I_{\{h=H\}})*(1/3)*(1-p_h)]*[I_{\{d1=D1\}}*p_{d1} + (1-I_{\{d1=D1\}})*(1/3)*(1-p_{d1})],$$

since H1 implies that $p_1$ originates from $d_1$. $P(H_{hdA}) = P(h = A)*P(D_1 = A) = f_h(A)*f_{d1}(A)$, where $f_h(A)$ and $f_{d2}(A)$ are obtained from population frequency data. $P(H_1,M) = P(H_1,M/H_{hdA})*P(H_{hdA}) + P(H_1,M/H_{hdC})*P(H_{hdC}) + P(H_1,M/H_{hdT})*P(H_{hdT}) + P(H_1,M/H_{hdG})*P(H_{hd\,G})$

Similarly, calculate $P(H_{hdA}\&H_2/M)$.
$P(H_{hdA}/M) = P(H_{hdA}\&H_1/M) + P(H_{hdA}\&H_2/M)$ and now we have calculated the probability that $p_1$ is equal to A. Repeat the calculation for C,T, and G. The highest value will give the answer of $p_1$ allele call and corresponding probability.

*Example Input*

Two input examples are shown. The first example is of a set of SNPs with a low tendency to cosegregate, that is, SNPs spread throughout a chromosome, and the input data is shown in Table 3. The second example is of a set of SNPs with a high tendency to cosegregate, that is SNPs clustered on a chromosome, and the input data is shown in Table 4. Both sets of data include an individual's measured SNP data, the individual's parents SNP data, and the corresponding confidence values. Note that this data is actual data measured from actual people. Each row represent measurements for one particular SNP location. The columns contain the data denoted by the column header. The key to the abbreviations in the column headers is as follows:

    ◦ family_id = the unique id for each person (included for clerical reasons)
    ◦ snp_id = the SNP identification number
    ◦ e1, e2 = the SNP nucleotide values for the embryo
    ◦ p1, p2 = the SNP nucleotide values for the father
    ◦ m1, m2 = the SNP nucleotide values for the mother
    ◦ pe1, pe2 = the measurement accuracy for e1,e2
    ◦ pp1, pp2 = the measurement accuracy for p1,p2
    ◦ pm1, pm2 = the measurement accuracy for m1,m2

*Example Output*

The two examples of output data are shown in Table 5 and Table 6, and correspond to the output data from the data given in Table 3 and Table 4 respectively. Both tables show an individual's measured SNP data, the individual's parents SNP data, the most likely true value of the individual's SNP data, and the corresponding confidences. Each row represents the data corresponding to one particular SNP. The columns contain the data denoted by the column header. The key to the abbreviations in the column headers is as follows:

    ◦ snp_id = the SNP identification number
    ◦ true_value = the proposed nucleotide value for e1,e2
    ◦ true_hyp = the hypothesis for the origin of e1,e2
    ◦ ee = the measured SNP nucleotide values for e1,e2
    ◦ pp = the measured SNP nucleotide values for p1,p2
    ◦ mm = the measured SNP nucleotide values for m1,m2
    ◦ HypProb = the probability of the final hypothesis. There is only one number for the output, but due to the excel column structure, this number is replicated in all rows.

Note that this algorithm can be implemented manually, or by a computer. Table 3 and Table 4 show examples of input data for a computer implemented version of the method. Table 5 shows the output data for the input data shown in Table 3. Table 6 shows the output data for the input data shown in Table 4.

*Simulation Algorithm*

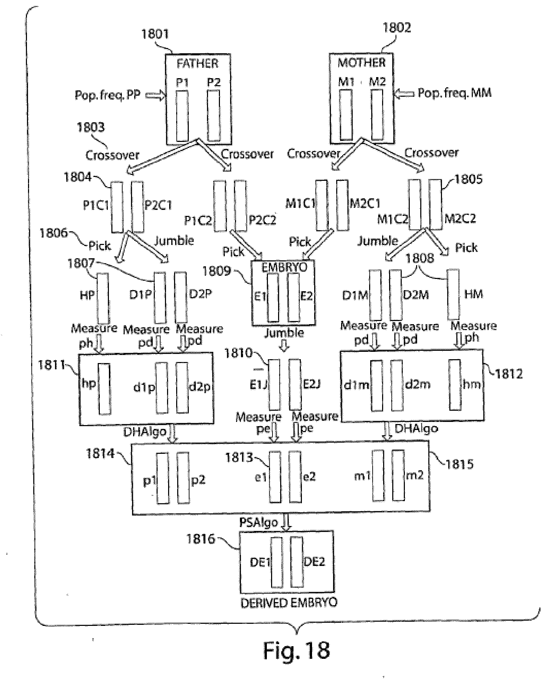

Below is a second simulation which was done to ensure the integrity of the system, and to assess the actual efficacy of the algorithm in a wider variety of situations. In order to do this, 1,000 full system simulations were run. This involves randomly creating parental genetic data, emulating meiosis *in silico* to generate embryonic data, simulating incomplete measurement of the embryonic data, and then running the method disclosed herein to clean the simulated measured embryonic data, and then comparing that "cleaned" data with the "real" data. A more detailed explanation of the simulation is given below, and the visual representation of the flow of events is given in Figure 18. Two different implementations of the theory were tested. A fuller explanation is given below.

*Simulation algorithms for DH and PS and results*

For both algorithms, the initial input variables are:

    (i) the list of SNPs to test,
    (ii) the population frequency of the maternal (popfreqlistMM) and paternal (popfreqlistPP) chromosomes,
    (iii) the probabilities of a correct allele call for haploid measurement (ph,pe), and for unordered diploid measurements (pd).

These values should be fixed based on the results from empirical data (population frequency) on relevant SNPs, and from measuring instrumentation performance (ph,pd,pe). The simulation was run for several scenarios such as most likely (informed), uniform (uninformed) and very unlikely (extreme case).

Once the above static parameters are fixed , crossover probabilities given the particular SNPs are the same for all the simulations, and will be derived ahead of the time given the databases for snip location(SNIPLOC_NAME_MAT) and genetic distance (HAPLOC_NAME_MAT).

[crossprob,snips]= GetCrossProb(snips,SNIPLOC_ NAME MAT, parameters, HAPLOC NAME MAT);

*Preliminary Simulation Loop*

The preliminary simulation loop is to demonstrate that the genetic data that will be used for the full simulation is realistic. Steps 1 through 5 were repeated 10,000 times. Note that this simulation can be run for either or both parents; the steps are identical. In this case, the simulation will be run for the paternal case for the purposes of illustration, and the references to Figure 18 will also include the corresponding maternal entry in Figure 18 in parentheses.

*Step 1: Generate original parental diploid cells (P1,P2),*

[P1,P2]=GenerateOriginalChromosomes(snips,popfreqlistPP); 1801 (1802)
Generate original paternal cells depending on the population frequency for each SNP for father cells.

*Step 2: Generate haploid and unordered diploid data for DHAlgo*

Simulate crossover of the parental chromosomes 1803 to give two sets of chromosomes, crossed over: P1C1, P2C1 and P1C2, P2C2; 1804 (1805). Pick one of the father alleles after the crossover 1806 (from the first set) for haploid allele HP 1807 (1808) in this case P1 (since there is no difference which one), and mix up the order in the diploid alleles to get (D1P,D2P) 1807 (1808).

    HP = PickOne(P1C1,P2C1);
    [D1P,D2P] = Jumble(P1,P2).

*Step 3: Introduce error to the original dataset in order to simulate measurements*

Based on given probabilities of correct measurement (ph-haploid, pd- diploid measurement), introduce error into the measurements to give the simulated measured parental data 1811 (1812).

    hp = MakeError(HP,ph);
    d1p = MakeError(D1P,pd);
    d2p = MakeError(D2P,pd).

*Step 4: Apply DHAlgo to get (p1,p2), (pp1,pp2)*

DHAlgo takes alleles from haploid cell and an unordered alleles from diploid cell and returns the most likely ordered diploid alleles that gave rise to these. DHAlgo attempts to rebuild (P1,P2), also returns estimation error for father (pp1,pp2). For comparison, the empirical algorithm that does simple allele matching is also used. The goal is to compare how much better is the disclosed algorithm, compared to the simple empirical algorithm.

$$[p1, \quad p2, \quad pp1, \quad pp2] \quad =DHAlgo(hp,d1p,d2p,ph,pd,snips,popfreqlistPP,'DH');$$

$$[p1s,p2s,pp1s,pp2s]=DHAlgo(hp,d1p,d2p,ph,pd,snips,popfreqlistPP,'ST');$$

*Step 5: Collect statistics for the run*

Compare (P1,P2) to derived (p1,p2).
[P1cmp( :,i), P2cmp( :,i),P1prob( :,i), P2prob( :,i),P1mn(i), P2mn(i)]=DHSimValidate(P1,P2,p1, p2,pp1,pp2);
Note: $(P1S_i ,P2S_i,P1P_i,P2P_i,P1A_i,P2A_i)= (I_{\{P1=p1\}}, I_{\{P2=p2\}}, p_{p1},p_{p2},p1_{acc}, p2_{acc})$, where $I_{\{P1=p1\}}$ is binary indicator array for estimation of DH algorithm accuracy for all the SNPs, similarly, for $I_{\{P2=p2\}}$. $p_{p1},p_{p2}$ are probabilities of a correct allele call derived from the algorithm, and $p1_{acc} = mean(I_{\{P1=p1\}})$, i.e. average accuracy for this run for p1, similar for $p2_{acc}$.

*Preliminary Simulation Results*

Ten thousand simulations were used to estimate the algorithm accuracy DHAccuracy.P1 = mean(P1A$_i$), DHAccuracy.P2 = mean(P2A$_i$), which shows the overall accuracy of the DH algorithm from P1,P2. On an individual SNP basis, the average accuracy on each SNP SNPAcc.P1 = mean(P1S$_i$) should agree with the average of the estimated probability of correctly measuring that SNP, SNPProb.P1 = mean(P2P$_i$), i.e. if the algorithm works correctly, the value for SNPAcc.P1should correspond closely to SNPProb.P1. The relationship between these two is reflected by their correlation.

The 10000 loops of the simulation were run for different setup scenarios:

(1) The underlying population frequency was given by existing genotyping data which is more realistic, and uniform population frequencies where A,C,T,G have the same probability on each SNP.
(2) Several combinations for measurement accuracy for haploid and unordered diploid measurements (ph,pd). Varying assumptions were made; that the measurements are both very accurate (0.95,0.95), less accurate (0.75,0.75) and inaccurate or random (0.25,0.25), as well as unbalanced combinations of (0.9, 0.5), (0.5, 0.9). What might be closest to reality may be accuracies of approximately 0.6 to 0.8.
(3) The simulation was run in all these cases for both the DHAlgorithm and simple matching STAlgorithm, in order to assess the performance of the disclosed algorithm.

The results of all these runs are summarized in Table 7.

The disclosed algorithm is performs better than the existing empirical algorithm in these simulations, especially for the realistic cases of non-uniform population frequency, and unbalanced or reduced probabilities of correct measurements. It has also been confirmed that our estimates of the algorithm accuracy for individual SNPs are very good in these cases, since the correlation between the estimated accuracy of correct allele call and simulation average accuracy is around 99% , with average ratio of 1.

In the most realistic case, for data population frequency and (ph,pd) = (0.6, 0.8), the average percent of correctly retrieved SNPs for (P1,P2) is (0.852, 0.816) in implementation 1, and (0.601, 0.673 in implementation 2..

Note that for Table 7 and Table 8 the rows beginning with "data" use population frequency data was taken from empirical results, while the rows beginning with "uniform" assume uniform populations.

It is important to note that in Table 7 and Table 8 the accuracy is defined as the average percent of SNPs where the correct SNP call was made *and* the correct chromosome of origin was identified. It is also important to note that these simulations reflect two possible implementations of the algorithm. There may be other ways to implement the algorithm that may give better results. This simulation is only meant to demonstrate that the method can be reduced to practice.

*Full Simulation Loop*

Steps 1-8 were repeated 10000 times. This is the simulation to test the full disclosed method to clean measured genetic data for a target individual using genetic data measured from related individuals, in this case, the parents.

*Step 1: Generate original parental diploid cells (P1,P2), (M1,M2)*

[P1,P2]=GenerateOriginalChromosomes(snips,popfreqlistPP); (1801)
[M1,M2]=GenerateOriginal Chromosomes(snips,popfreqlistMM); (1802)
Generate original parental cells depending on the population frequency for each SNP for mother and father cells.

*Step 2: Crossover parental cells (P1C,P2C), (MIC,M2C) (1803)*

Generate two sets of paternal cells with crossovers: first to get (P1C1,P2C1) used in DHAlgo, and second time to get (P1C2,P2C2) used in PSAlgo. (1804)
Generate two sets of maternal cells with crossovers: first to get (M1C1,M2C1) used in DHAlgo, and (M1C2,M2C2) used in PSAlgo. (1805)
[P1C1,P2C1]=Cross(P1,P2,snips,fullprob);
[P1C2,P2C2]=Cross(P1,P2,snips,fullprob);
[M1C1,M2C1]=Cross(M1,M2,snips,fullprob);

```
[M1C2,M2C2]=Cross(M1,M2,snips,fullprob);
```

*Step 3 Make haploid cell and unordered diploid cells for DHAlgo (1806)*

Pick one of the sets of paternal cells (1804, first set) for haploid cell HP, and mix up the order in the diploid cell to get (D1P,D2P) (1807). Do the same for mother cells (1805, first set) to get MH, (D1M,D2M). (1808).

```
HP = PickOne(P1C1,P2C1);
HM = PickOne(M1C1,M2C1);
[D1P,D2P] = Jumble(P1,P2);
[D1M,D2M] = Jumble(M1,M2);
```

*Step 4: Make diploid embryo cell (1809)*

Pick one of the paternal cells (1804, second set) and one of the maternal cells (1805, second set) for embryo cell. Mix up the order for measurement purposes.

```
E1 = PickOne(P1C2,P2C2);
E2 = PickOne(M1C2,M2C2);
[E1J,E2J] = Jumble(E1,E2); (1810)
```

*Step 5: Introduce error to the measurements* (1811, 1812, 1813)

Based on given measurement error (ph-haploid cells, pd- unordered diploid cells, peembryo cells), introduce error into the measurements.

```
hp = MakeError(HP,ph); (1811)
d1p = MakeError(D1P,pd); (1811)
d2p = MakeError(D2P,pd); (1811)
hm = MakeError(HM,ph); (1812)
d1m = MakeError(D1M,pd); (1812)
d2m = MakeError(D2M,pd); (1812)
e1 = MakeError(E1J,pe1); (1813)
e2 = MakeError(E2J,pe2); (1813)
```

*Step 6: Apply DHAlgo to get (p1,p2),(m1,m2), (pp1,pp2),(pm1,pm2)*

DHAlgo takes a haploid cell and an unordered diplod cell and returns the most likely ordered diploid cell that gave rise to these. DHAlgo attempts to rebuild (P1C1,P2C1) for father and (M1C1,M2C1) for mother chromosomes, also returns estimation error for father (pp1,pp2) and mother (pm1,pm2) cells.

```
[p1,p2,pp1,pp2]=DHAlgo(hp,d1p,d2p,snips,popfreqlistPP); (1814)
[m1,m2,pm1,pm2]=DHAlgo(hm,d1m,d2m,snips,popfreqlistMM); (1815)
```

*Step 7: Apply PSAlgo to get (DEI,DE2) (1816)*

PSAlgo takes rebuilt parent cells (p1,p2,m1,m2) and unordered measured embryo cell (e1,e2) to return most likely ordered true embryo cell (DE1,DE2). PS Algo attempts to rebuild (E1,E2).

```
[DE1,DE2,alldata]=PSAlgo(snips,e1,e2,p1,p2,m1,m2,pe,pp1,pp2,pm1,pm2,parameters,cr   ossprob,popfreqlistPP,popreqlistMM);
```

*Step 8: Collect desired statistics from this simulation run*

Get statistics for the run:
simdata=SimValidate(alldata,DE1,DE2,P1,P2,M1,M2,E1,E2,p1,p2,m1,m2,e1,e2,pe,pe,pp1,p p2,pm1,pm2);

*Simulation results*

Ten thousand simulations were run and the final estimates for the algorithm accuracy PSAccuracy.E1 = mean(E1A$_i$), PSAccuracy.E2 = mean(E2A$_i$), which tells us the overall accuracy of the PS algorithm from E1,E2 were calculated. On an individual SNP basis, the average accuracy on each SNP SNPAcc.E1 = mean(E1S$_i$) should agree with the average of the estimated probability of correctly measuring that SNP, SNPProb.E1 = mean(E2P$_i$), i.e. if the algorithm is written correctly, then SNPAcc.E1 should be observed to correlate to SNPProb.E1. The relationship between these two is reflected by their correlation.

Ten thousand loops of the simulation has been run for different setup scenarios:

(1) Underlying population frequency given by existing genotyping data which is more realistic, and uniform population frequencies where A,C,T,G have the same probability on each SNP.
(2) Several combinations of measurement accuracy for haploid, unordered diploid and embryo measurements (ph,pd,pe). A variety of accuracies were simulated: very accurate (0.95,0.95,0.95), less accurate (0.75,0.75,0.75) and inaccurate or random (0.25,0.25,0.25), as well as unbalanced combinations of (0.9, 0.5,0.5), (0.5, 0.9,0.9). What may be closest to reality is approximately (0.6,0.8,0.8).
(3) We ran the simulation in all these cases for both our PSAlgorithm and simple matching STPSAlgorithm, in order to assess the performance of the disclosed algortihm.

The results of these runs are summarized in the Table 8.

The disclosed algorithm is performs better than the existing empirical algorithm in these simulations, especially for the realistic cases of non-uniform population frequency, and unbalanced or reduced probabilities of correct measurements. It has also been shown that the estimates of the algorithm accuracy for individual SNPs are very good in these cases, since the correlation between the estimated accuracy of correct allele call and simulation average accuracy is around 99% , with average ratio of 1.

In the most realistic case, for data population frequency and (ph,pd,pe) = (0.6, 0.8, 0.8), the average percent of correctly retrieved SNPs for (E1,E2) is (0.777, 0.788) in implementation 1 and (0.835, 0.828) in implementation 2.. As mentioned above, the number denoting the average accuracy of algorithm refers not only to the correct SNP call, but also the identification of correct parental origin of the SNP. To be effective, an algorithm must return better results than the algorithm that simply accepts the data as it is measured. One might be surprised to see that in some cases, the accuracy of the algorithm is lower than the listed accuracy of measurement. It is important to remember that for the purposes of this simulation a SNP call is considered accurate only if it is both called correctly, and also its parent and chromosome of origin is correctly identified. The chance of getting this correct by chance is considerably lower than the measurement accuracy.

*Laboratory Techniques Necessary for Obtaining Prenatal and Embryonic Genetic Material*

There are many techniques available allowing the isolation of cells and DNA fragments for genotyping. The system and method described here can be applied to any of these techniques, specifically those involving the isolation of fetal cells or DNA fragments from maternal blood, or blastocysts from embryos in the context of IVF. It can be equally applied to genomic data *in silico,* i.e. not directly measured from genetic material.

In one embodiment of the system, this data can be acquired as described below.

*Isolation of cells*

Adult diploid cells can be obtained from bulk tissue or blood samples. Adult diploid single cells can be obtained from whole blood samples using FACS, or fluorescence activated cell sorting. Adult haploid single sperm cells can also be isolated from sperm sample using FACS. Adult haploid single egg cells can be isolated in the context of egg harvesting during IVF procedures.

Isolation of the target single blastocysts from human embryos can be done following techniques common in *in vitro* fertilization clinics. Isolation of target fetal cells in maternal blood can be accomplished using monoclonal antibodies, or other techniques such as FACS or density gradient centrifugation.

DNA extraction also might entail non-standard methods for this application. Literature reports comparing various methods for DNA extraction have found that in some cases novel protocols, such as the using the addition of N-lauroylsarcosine, were found to be more efficient and produce the fewest false positives.

*Amplification of genomic DNA*

Amplification of the genome can be accomplished by multiple methods inluding: ligation-mediated PCR (LM-PCR), degenerate oligonucleotide primer PCR (DOP-PCR), and multiple displacement amplification (MDA). Of the three methods, DOP-PCR reliably produces large quantities of DNA from small quantities of DNA, including single copies of chromosomes; this method may be most appropriate for genotyping the parental diploid data, where data fidelity is critical. MDA is the fastest method, producing hundred-fold amplification of DNA in a few hours; this method may be most appropriate for genotyping embryonic cells, or in other situations where time is of the essence.

Background amplification is a problem for each of these methods, since each method would potentially amplify contaminating DNA. Very tiny quantities of contamination can irreversibly poison the assay and give false data. Therefore, it is critical to use clean laboratory conditions, wherein pre- and post- amplification workflows are completely, physically separated. Clean, contamination free workflows for DNA amplification are now routine in industrial molecular biology, and simply require careful attention to detail.

*Genotyping assay and hybridization*

The genotyping of the amplified DNA can be done by many methods including molecular inversion probes (MIPs) such as Affymetrix's Genflex Tag Array, microarrays such as Affymetrix's 500K array or the Illumina Bead Arrays, or SNP genotyping assays such as AppliedBioscience's Taqman assay. The Affymetrix 500K array, MIPs/GenFlex, TaqMan and Illumina assay all require microgram quantities of DNA, so genotyping a single cell with either workflow would require some kind of amplification. Each of these techniques has various tradeoffs in terms of cost, quality of data, quantitative vs. qualitative data, customizability, time to complete the assay and the number of measurable SNPs, among others. An advantage of the 500K and Illumina arrays are the large number of SNPs on which it can gather data, roughly 250,000, as opposed to MIPs which can detect on the order of 10,000 SNPs, and the TaqMan assay which can detect even fewer. An advantage of the MIPs, TaqMan and Illumina assay over the 500K arrays is that they are inherently customizable, allowing the user to choose SNPs, whereas the 500K arrays do not permit such customization.

In the context of pre-implantation diagnosis during IVF, the inherent time limitations are significant; in this case it may be advantageous to sacrifice data quality for turn-around time. Although it has other clear advantages, the standard MIPs assay protocol is a relatively time-intensive process that typically takes 2.5 to three days to complete. In MIPs, annealing of probes to target DNA and post-amplification hybridization are particularly time-intensive, and any deviation from these times results in degradation in data quality. Probes anneal overnight (12-16 hours) to DNA sample. Post-amplification hybridization anneals to the arrays overnight (12-16 hours). A number of other steps before and after both annealing and amplification bring the total standard timeline of the protocol to 2.5 days. Optimization of the MIPs assay for speed could potentially reduce the process to fewer than 36 hours. Both the 500K arrays and the Illumina assays have a faster turnaround: approximately 1.5 to two days to generate highly reliable data in the standard protocol. Both of these methods are optimizable, and it is estimated that the turn-around time for the genotyping assay for the 500k array and/or the Illumina assay could be reduced to less than 24 hours. Even faster is the Taqman assay which can be run in three hours. For all of these methods, the reduction in assay time will result in a reduction in data quality, however that is exactly what the disclosure is designed to address. Some available techniques that are faster are not particularly high-throughput, and therefore are not feasible for highly parallel prenatal genetic diagnosis at this time.

Naturally, in situations where the timing is critical, such as genotyping a blastocyst during IVF, the faster assays have a clear advantage over the slower assays, whereas in cases that do not have such time pressure, such as when genotyping the parental DNA before IVF has been initiated, other factors will predominate in choosing the appropriate method. For example, another tradeoff that exists from one technique to another is one of price versus data quality. It may make sense to use more expensive techniques that give high quality data for measurements that are more important, and less expensive techniques that give lower quality data for measurements where the fidelity is not critical. Any techniques which are developed to the point of allowing sufficiently rapid high-throughput genotyping could be used to genotype genetic material for use with this method.

*A Contextual Example of the Method*

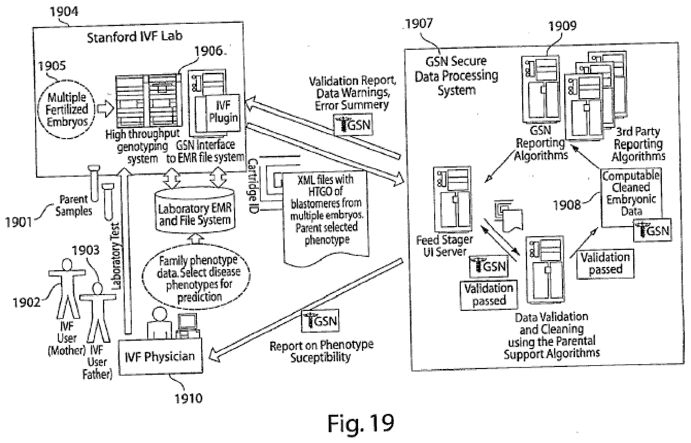

An example of how the disclosed method may be used in the context of an IVF laboratory that would allow full genotyping of all viable embryos within the time constraints of the IVF procedure is described here. The turn-around time required in an IVF laboratory, from egg fertilization to embryo implantation, is under three days. This means that the relevant laboratory work, the cleaning of the data, and the phenotypic prediction must be completed in that time. A schematic diagram of this system is shown in see Figure 19, and decribed here. This system may consist of parental genetic samples 1901 from IVF user (mother) 1902 and IVF user (father) 1903 being analyzed at IVF lab 1904 using a genotyping system. It may involve multiple eggs that are harvested from the mother 1902 and fertilized with sperm from the father 1903 to create multiple fertilized embryos 1905. It may involve a laboratory technician extracting a blastocyst for each embryo, amplifying the DNA of each blastocyst, and analyzing them using a high throughput genotyping system 1906. It may involve sending the genetic data from the parents and from the blastocyst to a secure data processing system 1907 which validates and cleans the embryonic genetic data. It may involve the cleaned embryonic data 1908 being operated on by a phenotyping algorithm 1909 to predict phenotype susceptibilities of each embryo. It may involve these predictions, along with relevant confidence levels, being sent to the physician 1910 who helps the IVF users 1902 and 1903 to select embryos for implantation in the mother 1901.

*Miscellaneous Notes Relating to Cleaning of Genetic Data*

It is important to note that the method described herein concerns the cleaning of genetic data, and as all living creatures contain genetic data, the methods are equally applicable to any human, animal, or plant that inherits chromosomes from parents. The list of animals and plants could include, but is not limited to: gorillas, chimpanzees, bonobos, cats, dogs, pandas, horses, cows, sheep, goats, pigs, cheetahs, tigers, lions, salmon, sharks, whales, camels, bison, manatees, elk, swordfish, dolphins, armadillos, wasps, cockroaches, worms, condors, eagles, sparrows, butterflies, sequoia, corn, wheat, rice, petunias, cow's vetch, sun flowers, ragweed, oak trees, chestnut trees, and head lice.

The measurement of genetic data is not a perfect process, especially when the sample of genetic material is small. The measurements often contain incorrect measurements, unclear measurements, spurious measurements, and missing measurements. The purpose of the method described herein is to detect and correct some or all of these errors. Using this method can improve the confidence with which the genetic data is known to a great extent. For example, using current techniques, uncleaned measured genetic data from DNA amplified from a single cell may contain between 20% and 50% unmeasured regions, or allele dropouts. In some cases the genetic data could contain between 1% and 99% unmeasured regions, or allel dropouts. In addition, the confidence of a given measured SNP is subject to errors as well.

In a case where the uncleaned data has an allele dropout rate of approximately 50%, it is expected that after applying the method disclosed herein the cleaned data will have correct allele calls in at least 90% of the cases, and under ideal circumstances, this could rise to 99% or even higher. In a case where the uncleaned data has an allele dropout rate of approximately 80%, it is expected that after applying the method disclosed herein the cleaned data will have correct allele calls in at least 95% of the cases, and under ideal circumstances, this could rise to 99.9% or even higher. In a case where the uncleaned data has an allele dropout rate of approximately 90%, it is expected that after applying the method disclosed herein the cleaned data will have correct allele calls in at least 99% of the cases, and under ideal circumstances, this could rise to 99.99% or even higher. In cases where a particular SNP measurement is made with a confidence rate close to 90%, the cleaned data is expected to have SNP calls with confidence rate of over 95%, and in ideal cases, over 99%, or even higher. In cases where a particular SNP measurement is made with a confidence rate close to 99%, the cleaned data is expected to have SNP calls with confidence rate of over 99.9%, and in ideal cases, over 99.99%, or even higher.

It is also important to note that the embryonic genetic data that can be generated by measuring the amplified DNA from one blastomere can be used for multiple purposes. For example, it can be used for detecting aneuploides, uniparental disomy, sexing the individual, as well as for making a plurality of phenotypic predictions. Currently, in IVF laboratories, due to the techniques used, it is often the case that one blastomere can only provide enough genetic material to test for one disorder, such as aneuploidy, or a particular monogenic disease. Since the method disclosed herein has the common first step of measuring a large set of SNPs from a blastomere, regardless of the type of prediction to be made, a physician or parent is not forced to choose a limited number of disorders for which to screen. Instead, the option exists to screen for as many genes and/or phenotypes as the state of medical knowledge will allow. With the disclosed method, the only advantage to identifying particular conditions to screen for prior to genotyping the blastomere is that if it is decided that certain PSNPs are especially relevant, then a more appropriate set of NSNPs which are more likely to cosegregate with the PSNPs of interest, can be selected, thus increasing the confidence of the allele calls of interest. Note that even in the case where SNPs are not personalized ahead of time, the confidences are expected to be more than adequate for the various purposes described herein.

*Phenotypic and Clinical Prediction*

There are many models available for predicting phenotypic data from genotypic and clinical information. Different models are more appropriate in different situations, based on the amount and type of data available. In order to choose the most appropriate method for phenotype prediction, it is often best to test multiple methods on a set of testing data, and determine which method provides the best accuracy of predictions when compared to the measured outcomes of the test data. Certain embodiments described herein include a set of methods which, when taken in combination and selected based on performance with test data, will provide a high likelihood of making accurate phenotypic predictions. First, a technique for genotype-phenotype modeling in scenario (ii) using contingency tables is described. Next, a technique for genotype-phenotype modeling in scenario (iii) using regression models built by convex optimization is described. Then, a technique for choosing the best model given a particular phenotype to be predicted, a particular patient's data, and a particular set of data for training and testing a model is described.

*The Data of Today: Modeling Phenotypic Outcomes based on Contingency Tables*

In cases where there are known genetic defects and alleles that increase the probability of disease phenotype, and where the number of predictors is sufficiently small, the phenotype probability can be modeled with a contingency table. If there is only one relevant genetic allele, the presence/absence of a particular allele can be described as A+/A- and the presence absence of a disease phenotype as D+/D-. The contingency table containing ($f_1$, $N_1$, $f_2$, $N_2$) is:

|     | D+    | D-      | #     |
|-----|-------|---------|-------|
| G+  | $f_1$ | $1-f_1$ | $N_1$ |
| G-  | $f_2$ | $1-f_2$ | $N_2$ |

$$S^2 = \frac{N_1 N_2 (N_1 + N_2)(p_1(1 - p_2) - (1 - p_1)p_2)^2}{(p_1 N_1 + p_2 N_2)((1 - p_1)N_1 + (1 - p_2)N_2)}$$

Where $f_1$ and $f_2$ represent the measured frequencies or probabilities of different outcomes and the total number of subjects is $N=N_1+N_2$. From this table, the odds ratio for the probability of having disease state D+ in the two cases of having independent variable (IV) G+ or G- can be reported as OR = $f_1(1-f_2)/f_2(1-f_1)$ with a with 95% confidence interval: $OR^{1 \pm 1.96/S}$, where S is a standard deviation. For example, using a study of breast cancer in 10,000 individuals, where M+ represents the presence of BRCA1 or BRCA2 allele:

|     | D+    | D-    | #    |
|-----|-------|-------|------|
| M+  | .563  | .437  | 1720 |
| M-  | .468  | .532  | 8280 |

This data results in an odds ratio, OR = 1.463, with confidence interval [1.31;1.62], which can be used to predict the increased probability of the occurrence of breast cancer with the given mutation. Note that contingency tables greater than two by two can be used to accommodate more independent variables or outcome variables. For example, in the case of breast cancer, the contingencies M+ and M- could be replaced with the four contingencies: BRCA1 and BRCA2, BRCA1 and not BRCA2, not BRCA1 and BRCA2, and finally not BRCA1 and not BRCA2. It is well understood by those knowledgeable in the art how to determine confidence intervals for contingency tables greater than two by two. This technique will be used when there are few enough IVs and enough data to build models with low standard deviations by counting the patients in different groups defined by different contingencies of the independent variables. This approach avoids the difficulty of designing a mathematical model that relates the different IV's to the outcome that is to be modeled, as is needed when constructing a regression model.

Note that the genetic data from particular SNPs may also be projected onto other spaces of independent variables, such as in particular the different patterns of SNPs that are recognized in the HapMap project. The HapMap project clusters individuals into bins, and each bin will be characterized by a particular pattern of SNPs. For example, consider one bin (B1) has a SNP pattern that contains BRCA1 and BRCA2, another bin (B2) has a SNP pattern that contains BRCA1 and not BRCA2, and a third bin contains a SNP pattern (B3) that is associated all other combinations of mutations. Rather than creating a contingency table representing all the different combinations of these SNPs, one may create a contingency table representing the contingencies B1, B2, and B3.

Note furthermore that the tendency of certain SNPs to occur together, as described by the HapMap project, can be used to create models that use multiple SNPs as predictors, even then the data consists of separate groups of patients where each group has had only one of the SNPs measured. This problem is commonly encountered when creating models from publicly available research papers, such as those available from OMIM, where each paper contains data on a cohort that has only one relevant SNP measured, although multiple SNPs are predictive of the phenotype. In order to illustrate this aspect which is useful for building predictive models using data available today, specific reference is made to Alzheimer's disease for which predictive models can be built based on the IVs: family history of Alzheimer's, gender, race, age, and the various alleles of three genes, namely APOE, NOS3, and ACE. In the context of this disease, a pervasive issue that applies to many diseases beyond Alzheimers is discussed: although many genes are involved in determining propensity for a particular phenotype, the vast majority of historical studies only sampled the alleles of a particular gene. In the case of Alzheimers disease, almost all study cohorts have only one gene sampled, namely APOE, NOS3, or ACE. Nonetheless, it is important to build models that input multiple genetic alleles even when the majority of available data comes from studies where only one gene is investigated. This problem is addressed in one aspect which is illustrated by considering a simplified case of two phenotype states and only two independent variables representing two relevant genes, each with just two states. Given a random variable describing the disease phenotype $D \in [D+, D-]$, and two random variables describing the genes $A \in [A+, A-]$ and $B \in [B+, B]$ the goal is to find the best possible estimate of $P(D/A,B)$. This can be found by applying Bayes Rule using $P(D/A,B) = P(A,B/D)P(D)/P(A,B)$. $P(D)$ and $P(A,B)$ are available from public data. Specifically, $P(D)$ refers to the overall prevalence of the disease in the population and this can be found from publicly available statistics. In addition, $P(A,B)$ refers to the prevalence of particular states of the genes A and B occurring together in an individual and this can be found from public databases such as the HapMap Project which has measured many different SNPs on multiple individuals in different racial groups. Note that in a preferred embodiment, all of these probabilities will be computed for particular racial groups and particular genders, for which there are probability biases, rather than for the whole human population. Once these probabilities have been determined, the challenge comes from accurately estimating $P(A,B/D)$ since the majority of cohort data provides estimates of $P(A/D)$ and $P(B/D)$. Relevant information can be found in various public databases, such as the HapMap Project, about the statistical associations between different genetic alleles i.e. about $P(A/B)$. However, given only $P(A/B)$, $P(A/D)$, $P(B/D)$ still nothing can be said of $P(A,B/D)$ since there is an unconstrained degree of freedom. Nonetheless, if some information is known about $P(A,B/D)$ from a cohort for which both genes A and B were sampled, even for just a single contingency such as (A-,B-) then the wealth of information about $P(A/D)$, $P(B/D)$, $P(A/B)$ may be leveraged to improve estimates of $P(A,B/D)$. This concept will be illustrated using contingency tables.

Consider the two contingency tables below, representing the probabilities of outcomes D+ and D- subject to the genetic states A+ and A-. This study is referred to as A. The measured frequencies for A are referred to with f and the actual probabilities that one seeks to estimate are referred to with p.

| A | D+ | D- |
|---|---|---|
| A+ | $f_1$ | $f_2$ |
| A- | $f_3$ | $f_4$ |

| A | D+ | D- |
|---|---|---|
| A+ | $p_1$ | $p_2$ |
| A- | $p_3$ | $p_4$ |

where $f_3 = 1-f_1$, $f_4 = 1-f_2$ and $p_3 = 1-p_1$, $p_4 = 1-p_2$. Let $K_1$ represent the number of subjects in the case group for A, that is, the number of subjects that have outcome D+. Let $K_2$ be the number in the control group for A, that is, the number of subjects that have outcome D-.

Similarly, consider the two contingency tables below, representing the probabilities of outcomes D+ and D- subject to the genetic states B+ and B-. This study is referred to as B. The measured frequencies are referred to with f and the actual probabilities that one seeks to estimate are referred to with p.

| B | D+ | D- |
|---|---|---|
| B+ | $f_5$ | $F_6$ |
| B- | $f_7$ | $F_8$ |

| B | D+ | D- |
|---|---|---|
| B+ | $P_5$ | $p_6$ |
| B- | $P_7$ | $p_8$ |

where $f_7 = 1-f_5$, $f_8 = 1-f_6$ and $p_7 = 1-p_5$, $p_8 = 1-p_6$. Let $K_3$ represent the number in the case group for B and let $K_4$ be the number in the control group for B. The contingency tables above represent trials where the genetic states A and B are measured separately. However, the contingency table that is ideally sought out involves the different states of A and B combined. The contingency table is shown below for a hypothetical study, referred to as AB, where f represents the measured probabilities and p represents the actual probabilities.

| AB | D+ | D- |
|---|---|---|
| A+B+ | $f_9$ | $f_{10}$ |
| A+B- | $f_{11}$ | $f_{12}$ |
| A-B+ | $f_{13}$ | $f_{14}$ |
| A-B- | $f_{15}$ | $f_{16}$ |

| AB | D+ | D- |
|---|---|---|
| A+B+ | $p_9$ | $p_{10}$ |
| A+B- | $p_{11}$ | $p_{12}$ |
| A-B+ | $p_{13}$ | $p_{14}$ |
| A-B- | $p_{15}$ | $p_{16}$ |

where $f_{15} = 1 - f_9 - f_{11} - f_{13}$, $f_{16} = 1 - f_{10} - f_{12} - f_{14}$ and $p_{15} = 1 - p_9 - p_{11} - p_{13}$ , $p16 = 1 - p_{10} - p2 - p_{14}$

Let $K_5$ be the number in case group for AB and let $K_6$ be the number in control group for AB. For notational purposes, note that $K_7 = K_9 = K_5$ and $K_8 = K_{10} = K_6$. So in fact, group sizes are:

| # | D+ | D- |
|---|---|---|
| A | $K_1$ | $K_2$ |
| B | $K_3$ | $K_4$ |
| AB | $K_5$ | $K_6$ |

Basic rules of statistics may be used to enforce dependencies between the cells of the hypothetical contingency table AB. In this example, for cells corresponding to D+, the following relationships may be enforced:

$$P(A+B-/D+) = P(A+/D+) - P(A+B+/D+)$$

$$P(A-B+/D+) = P(B+/D+) - P(A+B+/D+)$$

$$P(A-B-/D+) = 1 - P(A+/D+) - P(B+/D+) + P(A+B+/D+)$$

And similarly for cells corresponding to D-:

$$P(A+B-/D-) = P(A+/D-) - P(A+B+/D-)$$

$$P(A-B+/D-) = P(B+/D-) - P(A+B+/D-)$$

$$P(A-B-/D-) = 1 - P(A+/D-) - P(B+/D-) + P(A+B+/D-)$$

Using the notation in the contingency tables above, and leaving out the superfluous last relationship, these relationships translate to:

$$p_{11} = p_1 - p_9$$

$$p_{13} = p_5 - p_9$$

54

$$p_{12} = p_2 - p_{10}$$

$$p_{14} = p_6 - p_{10}$$

or equivalently

$$p_1 = p_9 + p_{11}$$

$$p_2 = p_{10} + p_{12}$$

$$p_5 = p_9 + p_{13}$$

$$p_6 = p_{10} + p_{14}$$

To summarize all the relationships, below is the table of all the dependencies of $p_1, ..., p_{16}$ on $p_9, ..., p_{16}$. To get a dependency between the values, the probability within the row is the summation of probabilities within the column that has value=1, for example the first row gives $p_1 = p_9 + p_{11}$.

| | $p_9$ | $p_{10}$ | $p_{11}$ | $p_{12}$ | $p_{13}$ | $p_{14}$ | $P_{15}$ | $p_{16}$ |
|---|---|---|---|---|---|---|---|---|
| $p_1$ | 1 | | 1 | | | | | |
| $p_2$ | | 1 | | 1 | | | | |
| $p_3$ | | | | | 1 | | 1 | |
| $p_4$ | | | | | | 1 | | 1 |
| $p_5$ | 1 | | | | 1 | | | |
| $p_6$ | | 1 | | | | 1 | | |
| $P_7$ | | | 1 | | | | 1 | |
| $p_8$ | | | | 1 | | | | 1 |
| $p_9$ | 1 | | | | | | | |
| $p_{10}$ | | 1 | | | | | | |
| $p_{11}$ | | | 1 | | | | | |
| $p_{12}$ | | | | 1 | | | | |
| $p_{13}$ | | | | | 1 | | | |
| $p_{14}$ | | | | | | 1 | | |
| $p_{15}$ | | | | | | | 1 | |
| $p_{16}$ | | | | | | | | 1 |

From the relationship between the frequencies and probabilities, the measurement equations $f_i = p_i + n_i$ for n=9... 16 may be created, where $n_i$ is a noise term representing the imperfect measurement of the probability $p_i$ based on frequency of occurrence $f_i$. Applying this to the relationships described above, and assuming that all the cells of contingency table AB have been measured (this is just for illustrative purposes and will be discussed below), these 10 observations may be represented:

These measurement equations may be presented in matrix notation as:

$$F = XP + N$$

Where $F = [F_1, ..., F_{16}]^T$, $P = [p_9, ..., p_{16}]^T$ and $N = [n_9, ..., n_{16}]^T$ and X is the matrix represented in the table above. This matrix equation may be used to solve for the 8 unknown coefficients, $p_9... p_{16}$. In this particular case we are solving for all the parameters $p_9... p_{16}$ If we do not have all the measurements for combined A,B genes, we need at least one measurement for D+ and one for D-. Given the relationships above, we can then fill out the rest of the table. In other words, in order to be able to fill out the contingency table for the hypothetical study AB, there desirably is at least one sample where a particular state of A and B were simultaneously measured on subjects that had outcomes of both D+ and D-. This enables one to achieve full rank for the matrix X representing the measurements made, so that the values $p_9... p_{16}$ are solved and filled in the contingency table AB. If more study data exists, further rows may be added to the bottom of the matrix X with a similar structure to that shown above.

To perform an accurate regression, a weighted regression with weights for each observation $f_i$ determined by the size of the group sample is desirable, so that studies and cells with many more observations get more weight. For the measurement equations $f_i = p_i + n_i$, the $n_i$ do not all have the same variance, and the regression is not homoscedastic. Specifically, $f_i = 1/K_i*\text{Binomial}(p_i, K_i) \sim N(p_i, p_i(1-p_i)/K_i)$ where $\text{Binomial}(p_i, K_i)$ represents a binomial distribution where each test has probability of the case outcome $p_i$ and $K_i$ tests are performed. This binomial distribution can be approximated by $N(p_i, p_i(1-p_i)/K_i)$ which is the normal distribution with mean $p_i$ and variance $p_i(1-p_i)/K_i$. Consequently, the noise may be modeled as a normal variable $n_i \sim N(0, p_i(1-p_i)/K_i)$ which has theoretical variance $V_i = p_i*(1-p_i)/K_i$. This variance can be approximated with the sample frequency $v_i = f_i*(1-f_i)/K_i$.

A weighted regression with weights for each observation i inversely proportional to variance $v_i$ was performed. The distribution of the noise matrix N as $\sim N(0, V)$ where V is a matrix with diagonal elements $[v_9,...,v_{16}]$ and all other elements are 0 may now be described. This is denoted as $V = \text{diag}([v_9,...,v_{16}])$. Similarly, let $W = \text{diag}([1/v_9,...,1/v_{16}])$. Now it is possible to solve for P using a weighted regression:

$$P = (X'WX)^{-1} X'WY$$

It is straightforward to show that the variance of P will be

$$\text{Var}(P) = (X'WX)^{-1}$$

which can be used to indicate the confidence in the determination of P.

To summarize, we have used the data from individual genes (A: $f_1,...,f_4$, B: $f_5,...,f_8$), together with data from the combination of A and B (AB: $f_9,...,f_{16}$) to help with estimating the probabilities for combination of A and B ($p_9,...,p_{16}$) and their variances ($v_9,...,v_{16}$). Finally, in our studies we mostly deal with log odds ratios, not probabilities, so we need to translate these probabilities into LORs. Generally, given the probabilities and variances for an event H as below.

|     | D+   | D-   |
| --- | ---- | ---- |
| H+  | p1   | p2   |
| H-  | 1-p1 | 1-p2 |
| V   | v1   | v2   |

The formula for the LOR is LOR = [log(p1) -log(1-p1) ]-[log(p2)-log(1-p2)], with variance (by delta method). $V = [(p1)^{-1} +(1-p1)^{-1}]^2*V(p1) + [(p2)^{-1}+(1-p2)^{-1}]^2*V(p2)$. The table below shows the probabilities, corresponding LOR and variance for combination of A,B

|      | D+       | D-       | LOR     | Var |
| ---- | -------- | -------- | ------- | --- |
| A+B+ | $P_9$    | $p_{10}$ | $lor_1$ | $V_1 = [1/p_9 +1/(1-p_9)]^2 v_9+ [1/p_{10} +1/(1-p_{10})]*v_{10}$ |
| A+B- | $P_{11}$ | $p_{12}$ | $lor_2$ | $V_2 = [1/p_{11} +1/(1-p_1)]^2 v_1+ [1/p_{12} +1/(1-p_{12})]^2 v_{12}$ |
| A-B+ | $P_{13}$ | $p_{14}$ | $lor_3$ | $V_3 = [1/p_{13} +1/(1-p_{13})]^2 v_3+ [1/p_{14}+1/(1-p_{14})]^2 v_{14}$ |
| A-B- | $P_{15}$ | $p_{16}$ | $lor_4$ | $V_4 = [1/p_{15} +1/(1-p_{15})]^2 p_{15}+[1/p_{16}+1/(1-p_{16})]^2 v_{16}$ |

This provides an estimate of the log odds ratios and respective variances.

As an illustration of this method, the technique was employed to obtain improved estimates of P(A,B/D) where D represents the state of having Alzheimers and where A and B represents two different states of the APOE and ACE gene respectively. Table 9 represents three different studies conducted by Alvarez in 1999 where only gene A was sampled; by Labert in 1998 where only gene B was sampled; and by Farrer in 2005 where genes A and B were sampled. Two sets of results have been generated from these studies, and are shown in Table 10. The first set (See Table 10, columns 2, 3, 4 and 5) analyzes all the cohorts and improves estimates of P(A,B/D) given P(A/D) and P(B/D) using the methods disclosed here. The second set (see Table 10, columns 6, 7, 8 and 9) uses only those results generated from the modern cohort of Farrer (2005) for P(A,B/D) in which both genes were sampled. The confidence bounds of predictions in the former case are considerably reduced. Note that these predictions can be further improved using data describing P(A/B) from public sources - these measurements can be added to the X matrix as described above. Note also that the techniques described here may be used to improve the estimates on the separate A, B probabilities such as P(A+/D+), P(A+/D-), P(B+/D+), and P(B-/D-) using the relationship such as p1 = p5+p7 as described above.

Note that while this method has been illustrated for only two variables A and B, it should be noted that the contingency tables can included many different IVs such as those mentioned above in the context of Alzheimer's prediction: family history of Alzheimer's, gender, race, age, and the various alleles of three genes, namely APOE, NOS3, and ACE. Continuous variables such as age can be made categorical by being categorized in bins of values in order to be suitable to contingency table formulation. In a preferred embodiment, the maximum number of IV's is used to model the probability of an outcome, with the standard deviation of the probability typically being below some specified threshold. In other words, the most specific contingencies possible may be created given the IV's available for a particular patient, while maintaining enough relevant training data for that contingency to make the estimate of the associated probability meaningful.

Note that it will also be clear to one skilled in the art, after reading this disclosure, how a similar technique for using data about disease-gene associations, gene-gene associations, and/or gene frequencies in the population can be applied to improve the accuracy of multivariable linear and nonlinear regression and logistic regression models. Furthermore, it will be clear to one skilled in the art, after reading this disclosure, how a similar technique for using data about disease-gene associations, gene-gene associations, and/or gene frequencies in the population can be applied to improve the accuracy of multivariable linear and nonlinear regression and logistic regression models by enabling the leveraging of outcome data to train the models where not all the independent variables of that are relevant to the model were measured for that outcome data. Furthermore, it will be clear to one skilled in the art, after reading this disclosure, how a similar technique for using data about disease-gene associations, gene-gene associations, and/or gene frequencies in the population can be applied to improve the accuracy of contingency table models built using other techniques such as the Expectation Maximization (EM) algorithm which is well understood in the art. These techniques will be particularly relevant to leveraging data from the HapMap project and other data contained in public databases such as National Center for Biotechnology Information (NCBI) Online Mendelian Inheritance in Man (OMIM) and dbSNP databases.

Note also, throughout the patent, that where we refer to data pertaining to an individual or a subject, this also assumes that the data may refer to any pathogen that may have infected the subject or any cancer that is infecting the subject. The individual or subject data may also refer to data about a human embryo, a human blastomere, a human fetus, some other cell or set of cells, or to an animal or plant of any kind.

*Tomorrow's Data: Modeling Multi-factorial Phenotype with Regression Models*

As more data is accumulated correlating genotype with multi-factorial phenotype, the predominant scenario will become (iii) as described above, namely it will be desirable to consider complex combinations of genetic markers in order to accurately predict phenotype, and multidimensional linear or nonlinear regression models will be invoked. Typically, in training a model for this scenario, the number of potential predictors will be large in comparison to the number of measured outcomes. Examples of the systems and methods described here include a novel technology that generates sparse parameter models for underdetermined or ill-conditioned genotype-phenotype data sets. The technique is illustrated by focusing on modeling the response of HIV/AIDS to Anti-Retroviral Therapy (ART) for which much modeling work is available for comparison, and for which data is available involving many potential genetic predictors. When tested by cross-validation with actual laboratory measurements, these models predict drug response phenotype more accurately than models previously discussed in the literature, and other canonical techniques described here.

Two regression techniques are described and illustrated in the context of predicting viral phenotype in response to Anti-Retroviral Therapy from genetic sequence data. Both techniques employ convex optimization for the continuous subset selection of a sparse set of model parameters. The first technique uses the Least Absolute Shrinkage and Selection Operator (LASSO) which applies the $l^1$ norm loss function to create a sparse linear model; the second technique uses the Support Vector Machine (SVM) with radial basis kernel functions, which applies the $\varepsilon$-insensitive loss function to create a sparse nonlinear model. The techniques are applied to predicting the response of the HIV-1 virus to ten Reverse Transcriptase Inhibitors (RTIs) and seven Protease Inhibitor drugs (PIs). The genetic data is derived from the HIV coding sequences for the reverse transcriptase and protease enzymes. Key features of these methods that enable this performance are that the loss functions tend to generate simple models where many of the parameters are zero, and that the convexity of the cost function assures that one can find model parameters to globally minimize the cost function for a particular training data set.

*The LASSO and the L$^1$ Selection Function*

When the number of predictors *M* exceeds the number of training samples *N,* the modeling problem is *overcomplete,* or *ill-posed,* since *any* arbitrary subset of *N* predictors is sufficient to yield a linear model with zero error on the training data, so long as the associated columns in the *X* matrix are linearly independent. Consequently, one is disinclined to put faith in an N-predictor model returned by a linear regression method. Suppose, however, a model with significantly fewer than *N* variables has low training error. The more sparse the model, the less probable that low training error could be a chance artifact; hence the more likely that the predictors are causally related to the dependent variable. This underlies the importance of sparse solutions in overcomplete problems, as is the case for the RTI data. A similar argument can be applied to *ill-conditioned* problems characterized by a large condition number on the matrix $X^T X$, as is the case for the PI data. In this case, the estimated parameters $\hat{b}$ are highly susceptible to the model error, as well as to measurement noise, and as a result are unlikely to generalize accurately. Overcomplete and ill-conditioned problems are typical of genetic data, where the number of possible predictors-genes, proteins, or, in our case, mutation sites-is large relative to the number of measured outcomes.

One canonical approach to such cases is subset selection. For example, with stepwise selection, at each step a single predictor is added to the model, based on having the highest *F-test* statistic indicating the level of significance with which that variable is correlated with prediction error. After each variable is added, the remaining variables may all be checked to ensure that none of them have dropped below a threshold of statistical significance in their association with the prediction error of the model. This technique has been successfully applied to the problem of drug response prediction. However, due to the discrete nature of the selection process, small changes in the data can considerably alter the chosen set of predictors. The presence or absence of one variable may affect the statistical significance associated with another variable and whether that variable is included or rejected from the model. This affects accuracy in generalization, particularly for ill-conditioned problems.

Another approach is for the values of the estimated parameters $\hat{b}$ to be constrained by means of a *shrinkage function.* A canonical shrinkage function is the sum of the squares of the parameters, and this is applied in *ridge regression* which finds the parameters according to:

$$\hat{b} = \arg\min_b \left\| y - Xb \right\|^2 + \lambda \left\| b \right\|^2 \qquad (17)$$

where $\lambda$ is a tuning parameter, typically determined by cross-validation. This method is nonsparse and does not set parameters to 0. This tends to undermine accuracy in generalization, and makes solutions difficult to interpret.

These problems are addressed by the LASSO technique. In contrast to subset selection, the LASSO does not perform discrete acceptance or rejection of predictor variables; rather it allows one to select en-masse, via a continuous subset optimization, the set of variables that together are the most effective predictors. It uses the $1^1$ norm shrinkage function:

$$\hat{b} = \arg\min_b \left\| y - Xb \right\|^2 + \lambda \sum_{i=1\ldots M} \left| b_i \right| \qquad (18)$$

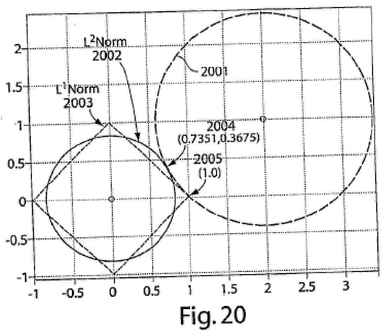

where $\lambda$ is typically set by cross-validation. The LASSO will tend to set many of the parameters to 0. Figure 20 provides insight into this feature of the LASSO, termed *selectivity.* Suppose that a model based on just two mutations is created with the training data $X = [1\ 0;\ 01]^T$, $y = [2\ 1]^T$ and the x-axis and y-axis represent the two parameters $b_1$ and $b_2$ respectively. Compare the use of the $1^1$ and $1^2$ shrinkage functions, where in both cases a solution is found that fits the training data equally well such that $\|y\text{-}Xb\|^2 = 2$. The large circle (2001), small circle (2002), and square (2003) respectively represent level curves for the cost functions $\|y\text{-}Xb\|^2$, the $1^2$ norm $\|b\|^2$, and the $1^1$ norm $|b_1|+|b_2|$. A solution for ridge regression (l$^2$) is found where the two circles meet (2004); a solution for the LASSO (l$^1$) is found where the square and the large circle intersect (2005). Due to the "pointiness" of the level curve for the $1^1$ norm, a solution is found that lies on the axis $b_1$ and is therefore sparse. This argument, extended into higher dimensions, explains the tendency of LASSO to produce sparse solutions, and suggests why the results achieved are measurably better than those reported in the literature.

The $1^1$ norm can be viewed as the most selective shrinkage function, while remaining convex. Convexity guarantees that one can find the one global solution for a given data set. A highly efficient recent algorithm, termed Least Angle Regression, is guaranteed to converge to the global solution of the LASSO in *M* steps.

Note that it will be clear to one skilled in the art, after reading this disclosure, how the $1^1$ norm can also be used in the context of logistic regression to model the probability of each state of a categorical variable. In logistic regression, a convex cost function may be formed that corresponds to the inverse of the a-posteriori probability of a set of measurements. The a-posteriori probability is the probability of the observed training data assuming the models estimates of the likelihood of each outcome. By adding to the $1^1$ norm to the convex cost function, the resulting convex cost function can be minimized to find a sparse parameter model for modeling the probability of particular outcomes. The use of $1^1$ norm for logistic regression may be particularly relevant when the number of measured outcomes is small relative to the number of predictors.

*Support Vector Machines and the L1-Norm*

SVM's may be configured to achieve good modeling of drug response and other phenotypes, especially in cases where the model involves complex interactions between the independent variables. The training algorithm for the SVM makes implicit use of the $1^1$ norm selection function. SVM's are learning algorithms that can perform real-valued function approximation and can achieve accurate generalization of sample data even when the estimation problem is ill-posed in the Hadamard sense. The ability of SVM's to accurately generalize is typically influenced by two selectable features in the SVM model and training algorithm. The first is the selection of the cost function, or the function that is to be minimized in training. The second is the selection of the kernels of the SVM, or those functions that enable the SVM to map complex nonlinear functions, involving interactions between the independent variables, using a relatively small set of linear regression parameters. These features are discussed below.

Consider modeling the phenotype for a subject i $y_i$, with a linear function approximation: $\hat{y}_i = f(x_i, b) = b^T x_i$. First, estimate $b$ by minimizing a cost function consisting of a $l^2$ shrinkage function on the parameters, together with the *"ε-insensitive loss"* function, which does not penalize errors below some $\varepsilon > 0$. The SV regression may be formulated as the following optimization:

$$\hat{b} = \arg\min_{b, \xi^-, \xi^+} \frac{\|b\|^2}{2} + C \sum_{i=1}^{N} \left( \xi_i^- + \xi_i^+ \right) \tag{19}$$

subject to the constraints:

$$y_i - b^T x_i \leq \varepsilon + \xi_i^+, i = 1 \ldots N \tag{20}$$

$$b^T x_i - y_i \leq \varepsilon + \xi_i^-, i = 1 \ldots N \tag{21}$$

$$\xi_i^+ \geq 0, \xi_i^- \geq 0, i = 1 \ldots N \tag{22}$$

The second term of the cost function minimizes the absolute value of the modeling errors, beyond the "insensitivity" threshold $\varepsilon$. Parameter $C$ allows one to scale the relative importance of the error vs. the shrinkage on the weights. This constrained optimization can be solved using the standard technique of finding the saddle-point of a Lagrangian, in order to satisfy the Kuhn-Tucker constraints. The Lagrangian, which accommodates the cost and the constraints described above, is:

$$L\left(b, \xi^+, \xi^-, \alpha^+, \alpha^-, \lambda^+, \lambda^-\right) = \frac{\|b\|^2}{2} + C \sum_{i=1}^{N} \left( \xi_i^- + \xi_i^+ \right) - \sum_{i=1}^{N} \alpha_i^- \left( y_i - b^T x + \varepsilon + \xi_i^- \right)$$
$$- \sum_{i=1}^{N} \alpha_i^- \left( y_i - b^T x + \varepsilon + \xi_i^- \right) - \sum_{i=1}^{N} \left( \lambda_i^- \xi_i^- + \lambda_i^+ \xi_i^+ \right) \tag{23}$$

Minimize with respect to the vectors of parameters $b, \zeta^-, \zeta^+$, and maximize with respect to the vectors of Lagrange multipliers $\alpha^-, \alpha^+, \lambda^-, \lambda^+$. Note that the Lagrange multipliers are desirably positive in accordance with the Kuhn-Tucker constraints. Hence, the optimal set of parameters can be found according to:

$$\left(b_*, \xi_*^+, \xi_*^-\right) = \arg\min_{b, \xi^+, \xi^-} \max_{\alpha^+, \alpha^-, \beta^+, \beta^-} L\left(b, \xi^-, \xi^+, \alpha^+, \alpha^-, \lambda^+, \lambda^-\right) \tag{24}$$

subject to

$$\alpha_i^+, \alpha_i^-, \lambda_i^+, \lambda_i^- \geq 0, i = 1 \ldots N \tag{25}$$

Since the order of minimization/maximization can be interchanged, first minimize with respect to variables $b$, $\xi_i^+$, $\xi_i^-$ by setting the partial derivatives of $L$ with respect to these variables to 0. From the resultant equations, one finds that the weight vector can be expressed in terms of

$$b = \sum_{i=1}^{N}\left(\alpha_i^+ - \alpha_i^-\right)x_i \tag{26}$$

Also from the resultant equations, eliminate variables from the Lagrangian so that one may find the coefficients $\alpha_i^+, \alpha_i^-, i = 1...N$ by maximizing the quadratic form:

$$W\left(\alpha^+, \alpha^-\right) = -\sum_{i=1}^{N}\varepsilon\left(\alpha_i^- + \alpha_i^+\right) + \sum_{i=1}^{N}y_i\left(\alpha_i^+ - \alpha_i^-\right) - \frac{1}{2}\sum_{i,j=1}^{N}\left(\alpha_i^- - \alpha_i^+\right)\left(\alpha_j^- - \alpha_j^+\right)x_i^T x_j \tag{27}$$

subject to

$$\sum_{i=1}^{N}\alpha_i^+ = \sum_{i=1}^{N}\alpha_i^- \tag{28}$$

$$0 \le \alpha_i^+ \le C, i = 1...N \tag{29}$$

$$0 \le \alpha_i^- \le C, i = 1...N \tag{30}$$

This enables the vector b to be computed and fully defines the SVM model for the ε-insensitive loss function. Note from equation (11) that the model may be characterized as

$$f(x) = \sum_{i=1}^{M}\beta_i\left(x^T x_i\right) + b_0 \tag{31}$$

where $\beta_i = \alpha_i^+ - \alpha_i^-$. The resulting model will tend to be sparse in that many of the parameters in the set $\{\beta_i, i = 1K\,M\}$ will be 0. Those vectors $x_i$ corresponding to non-zero valued $\beta_i$ are known as the support vectors of the model. The number of support vectors depends on the value of the tunable parameter $C$, the training data, and the suitability of the model. In an illustration below, it is shown how the model can now be augmented to accommodate complex nonlinear functions with the use of *kernel functions*. Next, it will be shown that the ε-insensitive loss function is related to the l[1] norm shrinkage function, and essentially achieves the same thing, namely the en-masse selection of a sparse parameter set by means of the l[1] norm.

In order to model a complex function, with possible coupling between variables, the simple inner product of Equation (17) is replaced with a kernel functions that computes a more complex interaction between the vectors. Inserting kernel functions, our function approximation in (17) takes the form:

$$f(x) = \sum_{i=1}^{N}\beta_i K\left(x, x_i\right) + \beta_0 = \sum_{i=0}^{N}\beta_i K\left(x, x_i\right) \tag{32}$$

where $K(x, x_0) = 1$ by definition. To find these parameters, use exactly the same optimization methods described above, and replace all terms $x^T x_i$ with $K(x, x_i)$. As before, compute the parameter set according to $\beta_i = \alpha_i^+ - \alpha_i^-$, by finding the arguments that maximize

$$W\left(\alpha^+, \alpha^-\right) = -\sum_{i=1}^{N}\varepsilon\left(\alpha_i^- + \alpha_i^+\right) + \sum_{i=1}^{N}y_i\left(\alpha_i^+ - \alpha_i^-\right) - \frac{1}{2}\sum_{i,j=1}^{N}\left(\alpha_i^- - \alpha_i^+\right)\left(\alpha_j^- - \alpha_j^+\right)K\left(x_i^T x_j\right) \tag{33}$$

subject to the same constraints as above. For the SVM results described above, radial basis kernel functions were selected.

Now, to illustrate the implicit use of the l$^1$ norm: consider that instead of trying to optimize equation (17) one begins with the optimization:

$$\beta^* = \arg\min_\beta \int_{-\infty}^{\infty} \left( f(x) - \sum_{i=0}^{N} \beta_i K(x_i, x) \right)^2 dx + \varepsilon \sum_{i=0}^{N} |\beta_i| \qquad (34)$$

where the l$^1$ shrinkage has been explicitly used to constrain the values of $\beta$, and the data fitting error, instead of being defined over discrete samples of training data, is defined over the domain of the hypothetical function being modeled. Now, make the variable substitutions: $\beta_i = \alpha_i^+ - \alpha_i^-$; $\alpha_i^+, \alpha_i^- \geq 0$, $\alpha_i^+\alpha_i^- \geq 0$, $i = 1...N$. Then the optimization may be recast as:

$$W(\alpha^+, \alpha^-) = -\sum_{i=1}^{N} \varepsilon(\alpha_i^- + \alpha_i^+) + \sum_{i=1}^{N} y_i(\alpha_i^+ - \alpha_i^-) - \frac{1}{2}\sum_{i,j=1}^{N}(\alpha_i^- - \alpha_i^+)(\alpha_j^- - \alpha_j^+)K(x_i, x_j) \qquad (35)$$

subject to the constraints

$$\alpha_i^+, \alpha_i^- \geq 0 \qquad (36)$$

$$\alpha_i^+ \alpha_i^- = 0 \qquad (37)$$

This solution, which has different constraints, will nonetheless coincide with that of the $\varepsilon$-insensitive loss function if both the value C for the SV method is chosen sufficiently large that the constraints $0 \leq \alpha_i^+, \alpha_i^- \leq C$ can simply become the constraints (21) and (22) and also one of the basis functions is constant, as in equation (17) for our case. In this case, one does not require the additional constraint $\sum_{i=1}^{N}\alpha_i^+ = \sum_{i=1}^{N}\alpha_i^-$ that is used by the SV method. Note that constraint (25) is already implicit in Equations (15) since the constraints (8) and (9) cannot be simultaneously active, so one of the Lagrange multipliers $\alpha_i^+$ or $\alpha_i^-$ should be slack, or 0.

Under these conditions, one can see that the $\varepsilon$-insensitive loss function achieves sparse function approximation, implicitly using the approach of an l$^1$ shrinkage function.

*Example of Multi-factorial Phenotype Prediction: Modeling HIV-1 Drug Response*

Current approaches to predicting phenotypic outcomes of salvage ART do not demonstrate good predictive power, largely due to a lack of statistically significant outcome data, combined with the many different permutations of drug regimens and genetic mutations. This field has a pressing need both for the integration of multiple heterogeneous data sets and the enhancement of drug response prediction.

The models demonstrated herein used data from the Stanford HIVdb RT and Protease Drug Resistance Database for training and testing purposes. This data consists of 6644 *in vitro* phenotypic tests of HIV-1 viruses for which reverse transcriptase (RT) or protease encoding segments have been sequenced. Tests have been performed on ten reverse transcriptase inhibitors (RTI) and seven protease inhibitors (PI). The RTIs include lamivudine (3TC), abacavir (ABC), zidovudine (AZT), stavudine (D4T), zalcitabine (DDC), didanosine (DDI), delaviradine (DLV), efavirenz (EFV), nevirapine (NVP) and tenofovir (TDF). The PIs include ampranavir (APV), atazanavir (ATV), nelfinavir (NFV), ritonavir (RTV), saquinavir (SQV), lopinavir (LPV) and indinavir (IDV)).

For each drug, the data has been structured into pairs of the form $(x_i, y_i)$, $i$ = 1K $N$, where $N$ is the number of samples constituting the training data, $y_i$ is the measured drug fold resistance (or phenotype), and $x_i$ is the vector of mutations plus a constant term, $x_i = [1 \; x_{i1}, x_{i2} \, \text{K} \, x_{iM}]^T$, where $M$ is the number of possible mutations on the relevant enzyme. Assume element $x_{im}$=1 if the $m^{th}$ mutation is present on $i^{th}$ sample, and set $x_{im}$=0 otherwise. Each mutation is characterized both by a codon locus and a substituted amino acid. Mutations that do not affect the amino acid sequence are ignored. Note that only mutations present in more than 1% percent of the samples for each drug are included in the set of possible predictors for a model, since it is improbable that mutations associated with resistance would occur so infrequently. The measurement $y_i$ represents the fold resistance of the drug for the mutated virus as compared to the wild type. Specifically, $y_i$ is the log of the ratio of the IC$_{50}$ (the concentration of the drug required to slow down replication by 50%) of the mutated virus, as compared to the $IC_{50}$ of the wild type virus. The goal is to develop a model for each drug that accurately predicts $y_i$ from $x_i$. In order to perform batch optimization on the data, stack the independent variables in an $N$ by $M+1$ matrix, $X = [x_1, x_2$ K $x_N]^T$, and stack all observations in a vector $y = [y_1, y_2$ K $y_N]^T$.

The performance of each algorithm is measured using cross-validation. For each drug, the first-order correlation coefficient R is calculated between the predicted phenotypic response of the model and the actual measured *in vitro* phenotypic response of the test data.

$$R = \frac{\left(\hat{y} - \bar{\hat{y}}\vec{1}\right)^T \left(y - \bar{y}\vec{1}\right)}{\left\|\hat{y} - \bar{\hat{y}}\vec{1}\right\|_2 \left\|y - \bar{y}\vec{1}\right\|_2} \qquad (38)$$

Where vector $\hat{y}$ is the prediction of phenotypes $y$, $\bar{y}$ denotes the mean of the elements in vector $y$ and $\vec{1}$ denotes the vector of all ones. For each drug and each method, the data is randomly subdivided in the ratio 9:1 for training and testing, respectively. In one example, ten different subdivisions are performed in order to generate the vector $\hat{y}$ and $R$ without any overlap of training and testing data. This entire process may then be repeated ten times to generate ten different values of $R$. The ten different values of $R$ are averaged to generate the $R$ reported. The standard deviation of $R$ is also determined for each of the models measured over the ten different experiments to ensure that models are being compared in a statistically significant manner.

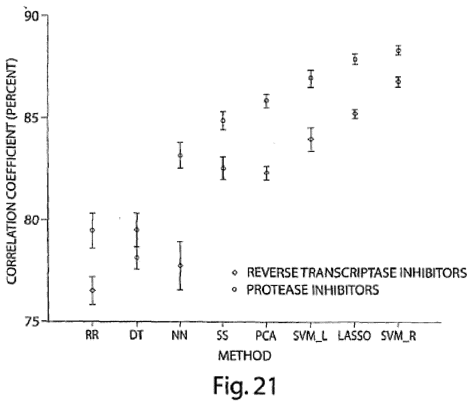

Table 11 displays the results of the above mentioned models for the PI drugs; Table 12 displays the results for the ten RTI drugs. Results are displayed in terms of correlation coefficient $R$, averaged over ten subdivisions of the training and test data. The estimated standard deviation of the mean value of $R$, computed from the sample variance, is also displayed. The number of available samples for each drug is shown in the last row. The methods tested, in order of increasing average performance, are: i) RR - Ridge Regression, ii) DT - Decision Trees, iii) NN - Neural Networks, iv) PCA - Principal Component Analysis, v) SS - Stepwise Selection, vi) SVM_L - Support Vector Machines with Linear Kernels, vii) LASSO - Least Absolute Shrinkage and Selection Operator, and viii) SVM - Support Vector Machines with Radial Basis Kernels. The information in the last columns of Table 11 and Table 12 is depicted in Figure 21. The circles in Figure 21 display the correlation coefficient R averaged over ten different experiments for each PI, and averaged over the seven different PIs. The diamonds in Figure 21 display the correlation coefficient R averaged over ten different experiments for each RTI, and averaged over the ten different RTIs. The one standard deviation error bars are also indicated.

Wherever modeling techniques involve tuning parameters, these have been adjusted for optimal performance of the technique as measured by cross-validation, using a grid search approach. In all cases, the grid quantization was fine enough that the best performing parameters from the grid were practically indistinguishable from the optimal parameters for the given data, since the difference in the prediction due to grid quantization lay below the experimental noise floor.

Although there are strong trends in the data, it should be noted that due to differences in the number of samples, interactions of the underlying genetic predictors, and other idiosyncrasies in the data that vary between drugs, the R achieved by each algorithm may vary from drug to drug. This variation may be seen by studying the individual drug columns of Table 11 (columns 3 to 9) and Table 12 (columns 3 to 12).

Of all the methods, SVM performs best, slightly outperforming LASSO (P<0.001 for the RTIs; P=0.18 for the PIs). The performance of SVM trained with the ε-insensitive loss function is considerably better than that of previously reported methods based on the support vector machine. SVM, which uses nonlinear kernel functions, outperforms SVM_L which uses linear kernel functions, and which is also trained using the ε-insensitive loss function (P = 0.003 for RTIs; P < 0.001 for PIs). The SVM considerably outperforms the other nonlinear technique which uses neural networks and which does not create a convex cost function (P<0.001 for both RTIs and PIs). The LASSO technique, which trains a linear regression model using a convex cost function and continuous subset selection, considerably outperforms the SS technique (P<0.001 for both PIs and RTIs). The top five methods, namely SS, PCA, SVM_L, LASSO, SVM_R, all tend to generate models that are sparse, or have a limited number of non-zero parameters.

In order to illustrate the subset of mutations selected as predictors, certain embodiments disclosed herein focus on the second-best performing model, namely the LASSO, which creates a linear regression model that, unlike SVM, does not attempt to emulate nonlinear or logical coupling between the predictors. Consequently, it is straightforward to show how many predictors are selected. Table 13 shows the number of mutations selected by the LASSO as predictors for each PI drug (Table 13, row 4), together with the number of mutations (Table 13, row 3), and the total number of samples (Table 13, row 2), used in training each model. The same table is shown for the RTIs (Table 14, same rows correspond to the same items).

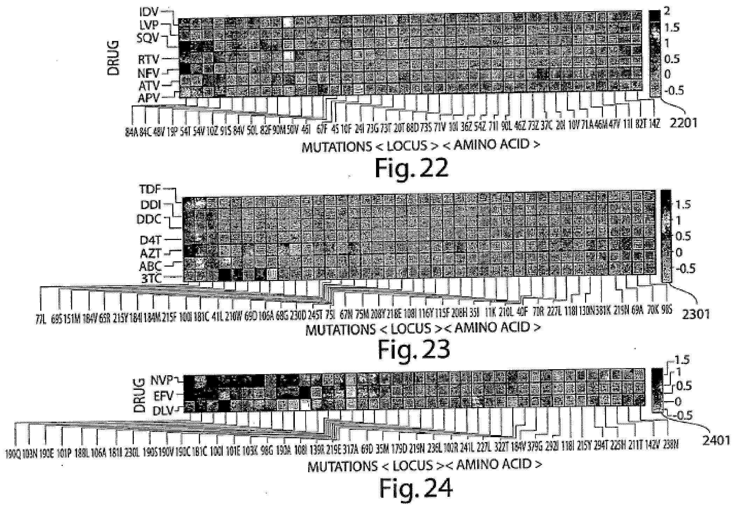

The selected mutations may also enhance understanding of the causes of drug resistance. Figures 22, 23 and 24 show the value of the parameters selected by the LASSO for predicting response to PI, Nucleoside RTIs (NRTIs) and Non-Nucleoside RTIs (NNRTIs) respectively. Each row in the figures represents a drug; each column represents a mutation. Relevant mutations are on the protease enzyme for PI drugs, and on the RT enzyme for NRTI and NNRTI drugs. The shading of each square indicates the value of the parameter associated with that mutation for that drug. As indicated by the color-bar on the right (2201, 2301 and 2401, respectively), those predictors that are shaded darker are associated with increased resistance; those parameters that are shaded lighter are associated with increased susceptibility. The mutations are ordered from left to right in order of decreasing magnitude of the average of the associated parameter. The associated parameter is averaged over all rows, or drugs, in the class. Those mutations associated with the forty largest parameter magnitudes are shown. Note that for a particular mutation, or column, the value of the parameter varies considerably over the rows, or the different drugs in the same class.

For the algorithms RR, DT, NN, and SS, the model was not trained on all genetic mutations, but rather on a subset of mutations occurring at those sites that have been determined to affect resistance by the Department of Health and Human Services (DHHS). The reduction in the number of independent variables was found to improve the performance of these algorithms. In the case of the SVM_L algorithm, best performance for RTIs was achieved using only the DHHS mutation subset, while best performance for PIs was achieved by training the model on all mutations. For all other algorithms, best overall performance was achieved by training the model on all mutations.

The set of mutations shown in Figures 22, 23 and 24 that were selected by the LASSO as predictors, but are not currently associated with loci determined by the DHHHS to affect resistance, are: for PIs - 19P, 91S, 67F, 4S, 37C, 11I, 14Z; for NRTIs - 68G, 203D, 245T, 208Y, 218E, 208H, 35I, 11K, 40F, 281K; and for NNRTIs - 139R, 317A, 35M, 102R, 241L, 322T, 379G, 292I, 294T, 211T, 142V. Note that in some cases, such as for the LASSO and the SVM, the performance for particular drugs, such as LPV, was significantly improved (P<0.001) when all mutations were included in the model (R = 86.78, Std. dev = 0.17) as compared to the case when only those loci recognized to affect resistance by DHHS were included (R = 81.72, Std. dev. = 0.18). This illustrates that other mutations, beyond those recognized by the DHHS, may play a role in drug resistance.

The use of convex optimization techniques has herein been demonstrated to achieve continuous subset selection of sparse parameter sets in order to train phenotype prediction models that generalize accurately. The LASSO applies the $l^1$ norm shrinkage function to generate a sparse set of linear regression parameters. The SVM with radial basis kernel functions and trained with the $\varepsilon$-insensitive loss function generates sparse nonlinear models. The superior performance of these techniques may be explained in terms of the convexity of their cost functions, and their tendency to produce sparse models. Convexity assures that one can find the globally optimal parameters for a particular training data set when there are many potential predictors. Sparse models tend to generalize well, particularly in the context of underdetermined or ill-conditioned data, as is typical of genetic data. The $l^1$ norm may be viewed as the most selective convex function. The selection of a sparse parameter set using a selective shrinkage function exerts a principle similar to Occam's Razor: *when many possible theories can explain the observed data, the most simple is most likely to be correct.* The SVM, which uses an $l^2$ shrinkage function together with an $\varepsilon$-insensitive loss function, tends to produce an effect similar to the explicit use of the $l^1$ norm as a shrinkage function applied to the parameters associated with the support vectors.

Techniques using the $l^1$ shrinkage function are often able to generalize accurately when the number of IVs is large, and the data is undetermined or ill-conditioned. Consequently, it is possible to add nonlinear or logical combinations of the independent variables to the model, and expect that those combinations that are good predictors will be selected in training. The SVM is able to model interactions amongst the independent variables with the use of nonlinear kernel functions, such as radial basis functions, which perform significantly better than linear kernel functions. Consequently, without changing the basic concepts disclosed herein, the performance of the LASSO may be enhanced by adding logical combinations of the independent variables to the model. Logical terms can be derived from those generated by a decision tree, from those logical interactions described by expert rules, from the technique of logic regression, or even from a set of random permutations of logical terms. An advantage of LASSO is that the resulting model will be easy to interpret, since the parameters directly combine independent variables, or expressions involving independent variables, rather than support vectors. The robustness of the LASSO to a large number of independent variables in the model is due both to the selective nature of the $l^1$ norm, and to its convexity.

Other techniques exist that use shrinkage function more selective than the $l^1$ norm. For example, log-shrinkage regression uses a shrinkage function derived from coding theory which measures the amount of information residing in the model parameter set. This technique uses the log function as a shrinkage function instead of the $l^1$-norm and is consequently non-convex. While offering a theoretically intriguing approach for seeking a sparse set of parameters, the non-convexity of the penalty function means that solving the corresponding regression is still computationally less tractable than the LASSO, and for large sets of predictors may yield only a local rather than a global minimum for the given data.

The techniques described here may be applied to creating linear and nonlinear regression models for a vast range of phenotype prediction problems. They are particularly relevant when the number of potential genetic predictors is large compared to the number of measured outcomes.

*Simplifying a Regression Model by Mapping Genetic Independent Variables into a Different Space*

Note that, as described above, in cases where complex combinations of genetic markers are considered, it is possible to project the SNP variables onto another variable space in order to simplify the analysis. This variable space may represent known patters of mutations, such as the clusters or bins described by the HapMap project. In other words, rather than the vector $x_i$ representing particular SNP mutations as described above, it may represent whether the individual falls into particular HapMap clusters or bins. For example, following the notation above, imagine there is a vector $x_i=[x_{i1}, x_{i2} ... x_{iB}]^T$ where $B$ is the number of relevant HapMap bins. One can set element $x_{ib}=1$ if the individuals SNPS pattern falls into the $b^{th}$ bin and 0 otherwise. Alternatively, if the overlap between the individuals SNPs and a particular bin is incomplete, and it may not be desirable to simply place the individual in a category "other", then one may set each $x_{ib}$ equal to the fraction of overlap between his pattern of SNPs and that of bin $b$. Many other techniques are possible to formulate the regression problem without changing the concepts disclosed herein.

*Model Selection by Cross Validation for Outcome Prediction*

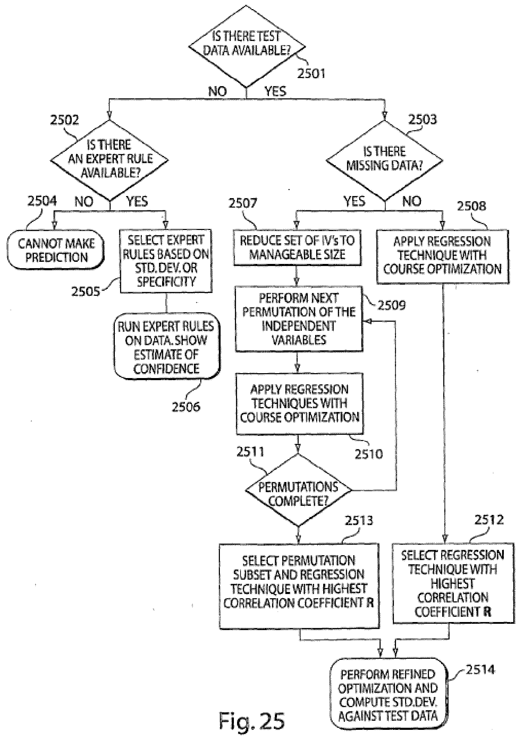

In what has preceded this discussion, different phenotype prediction techniques involving expert rules, contingency tables, linear and nonlinear regression were described. Now a general approach to selecting from a set of modeling techniques which is best to model a particular categorical or non-categorical outcome for a particular subject is described, based on the use of training data. Figure 25 provides an illustrative flow diagram for the system. The process described in Figure 25 is a general approach to selecting the best model given the data that is available for a particular patient, the phenotype being modeled, and a given set of testing and training data, and the process is independent of the particular modeling techniques. In a preferred embodiment the set of modeling techniques that may be used include expert rules, contingency tables, linear regression models trained with LASSO or with simple least-squares where the data is no under-determined, and nonlinear regression models using support vector machines.

The process begins 2501 with the selection of a particular subject and a particular dependent variable (DV) that will be modeled, or - if it's a categorical variable - for which the probability may be modeled. The system then determines 2502 the set of Independent Variables (IVs) that are associated with that subject's record and which may be relevant to modeling the outcome of the DV. The human user of the system may also select that subset of IVs that the user considers to be possible relevant to the model. The system then checks 2503a to see whether a model has already been trained and selected for the given combination of independent variables and the given dependent variable to be modeled. If this is the case, and the data used for training and testing the ready-made model is not out of date, the system will go directly to generating a prediction 2519 using that model. Otherwise, the system will extract from the database all other records that have the particular DV of interest and which may or may not have the same set of IV's as the particular subject of interest. In so doing, the system determines 2503b whether data is available for training and testing a model. If the answer is no, the system checks 2515 to see if there are any expert rules available to predict the outcome based on a subset of the IV's available for the subject. If no expert rules are available then the system exits 2504 and indicates that it cannot make a valid prediction. If one or more expert rules are available, then the system will select 2505 a subset of expert rules that are best suited to the particular subject's data. In a preferred embodiment, the selection of which expert rule to apply to a subject will be based on the level of confidence in that expert rule's estimate. If no such confidence estimate is available, the expert rules can be ranked based on their level of specificity, namely based on how many of the IVs available for the subject of interest the expert rules uses in the prediction. The selected subset of expert rules is then used to generate a prediction 2506.

If it is determined 2503b that data is available, the system will check 2516 to determine whether or not there is any data missing in the test and training data. In other words, for all those records that include the relevant DV, the system will check to see if all those records have exactly the same set of IVs as are available for the patient of interest and which may be potential predictors in the model. Typically, the answer will be 'no' since different information will be available on different patients. If there is missing data, the system will go through a procedure to find that subset of IV's that should be used to make the best possible prediction for the subject. This procedure is time-consuming since it involves a multiple rounds of model training and cross-validation. Consequently, the first step in this procedure is to reduce 2507 the set of IVs considered to a manageable size based on the available computational time. In a preferred embodiment, the set of IVs are reduced based on there being data on that IV for a certain percentage of the subjects that also have the DV available. The set of IVs can be further reduced using other techniques that are known in the art such as stepwise selection which assumes a simple linear regression model and selects IVs based on the extent to which they are correlated with the modeling error. The system then enters a loop in which every combination of the remaining IVs is examined. In a preferred embodiment the following states for each IV and the DV are considered: each IV can either be included or not included in the model and for numerical data for an IV or DV that is positive for all subjects, the data may or may not be preprocessed by taking its logarithm. For each particular combination of inclusions/exclusions and pre-processing of the IVs and the DV, a set of modeling technique is applied 2510.

Most modeling techniques will have some tuning parameter that can be optimized or tuned based on a grid-search approach using cross-validation with the test data. For example, for the LASSO technique discussed above, many values will be explored for the variable parameter $\lambda$. For each value of $\lambda$, the regression parameters may be trained, and the model predictions may be compared with the measured values of test data. Similarly, for the support vector machine approach discussed above, the tuning parameters to be optimized using a grid-search approach include C, $\varepsilon$ and possibly parameters describing the characteristics of the kernel functions. For techniques based on contingency tables, the tunable parameter may correspond to the highest standard deviation that can be accepted from a contingency table model, while making the contingencies as specific as possible for the given subject, as discussed above.

Many different metrics may be used to compare the model predictions with test data in order to optimize the tunable parameters and select among models. In a preferred embodiment, the standard deviation of the error is used. In other embodiments, one may use the correlation coefficient R between the predicted and measured outcomes. In the context of logistic regression or contingency tables, one may also use the maximum a-posteriori probability, namely the probability of the given set of test data assuming the model's prediction of the likelihood of each test outcome. Whatever metric is used, that value of the tuning parameter is selected that optimizes the value of the metric, such as minimizing the standard deviation of the prediction error if the standard deviation of the prediction error is used as a test metric. Since model training and cross-validation is a slow process, at this stage 2510 the grid that defines the different tuning parameters to be examined is set coarsely, based on the amount of available time, so that only a rough idea of the best model and best tuning parameters can be obtained.

Once all the different IV/DV combinations have been examined in this way 2511, the system selects that combination of IVs/DV, that model and those tuning parameters that achieved the best value of the test metric. Note that if there is no missing data then the system will skip the step of checking all combinations of the IVs/DV. Instead, the system will examine the different modeling techniques and tuning parameters 2508, and will select that modeling method and set of tuning parameters that maximizes the test metric. The system then performs refined tuning of the best regression model, using a more finely spaced grid, and for each set of tuning parameter values, determines the correlation with the test data. The set of tuning parameters is selected that produces the best value of the test metric. The system then determines 2518 whether or not the test metric, such as the standard deviation of the prediction error, is below or below a selected threshold so that the prediction can be considered valid. For example, in one embodiment, a correlation coefficient of R > 0.5 is desirable for a prediction to be deemed valid. If the resultant test metric does not meet the threshold then no prediction can be made 2517. If the test metric meets the requisite threshold, a phenotype prediction may be produced, together with the combination of IV's that was used for that prediction and the correlation coefficient that the model achieved with the test data.

*Illustrating Model Selection by Cross Validation in Cancer Cohorts with Missing Data*

In order to demonstrate this aspect, a focus was on utilizing the genetic and phenotypic data sets related to colon cancer that can be found in PharmGKB which is part of the National Institute of Health's Pharmacogenomic Research Network and has a mission to discover how individual genetic variations contribute to different drug response. For this dataset, a key challenge was missing information. Ideally, one would like to apply the regression techniques described above to automatically select an IV subset for the model from all IVs that are available on a particular patient. However, this limits the amount of data that is available from other patients for training and testing the model. Consequently, for datasets containing few enough IVs, it is possible to search through all possible subsets of the independent variables. For each, as described above, one can extract that set of patients for which the required outcome has been measured, and the relevant set of independent variables is available. As described above, one can also search the space of possible ways to preprocess the included independent variables, such as taking the logs of positive numeric independent variables. For each combination of independent variables included and independent variable preprocessing techniques, the model is trained and tested by cross-validation with test data. That model is selected which has the best cross-validation with test data. Once a model has been created for a given set on IVs, that model is applied to new patient data submitted with the same set of IVs without requiring the exhaustive model search.

This technique has been used to predict clinical side effects for colorectal cancer drug Irinotecan. Severe toxicity is commonly observed in cancer patients receiving Irinotecan. Data was included which describes the relationships between Irinotecan pharmacokinetics and side effects with allelic variants of genes coding for Irinotecan metabolizing enzymes and transporters of putative relevance. Patients were genotyped for variations in the genes encoding MDR1 P-glycoprotein (ABCB1), multidrug resistance- associated proteins MRP-1 (ABCC1) and MRP-2 (ABCC2), breast cancer resistance protein (ABCG2), cytochrome P450 isozymes (CYP3A4, CYP3A5), carboxylesterases (CES1, CES2), UDP glucuronosyl-transferases (UGT1A1, UGT1A9), and the hepatic transcription factor TCF1. The phenotypic data that is associated with the genetic sequence data for this study is described in Table 15.

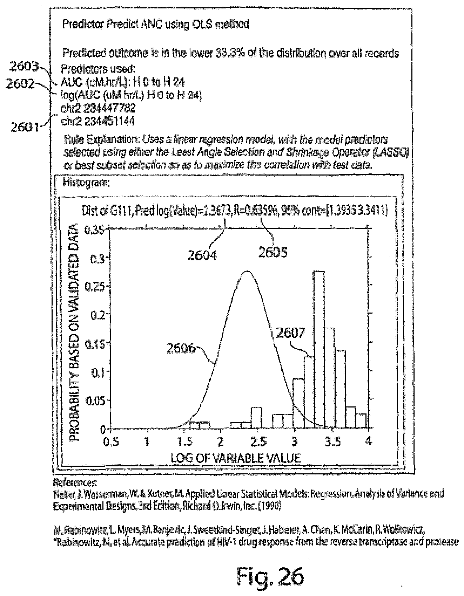

Figure 26 illustrates a model of prediction outcome for colon cancer treatment with irinotecan given the available PharmGKB data that was submitted using the pharmacogenomic translation engine. In Figure 26, the model shows the relevant genetic loci (2601), the indicators used, in this casethe log of CPT-11 area-under-the concentration curve (AUC) from 0-24 hours (2602) and the log of SN-38 AUC from 0-24 hours (2603) to predict the log of the Nadir of Absolute Neutrophil Count from day 12 to day 14 (2604). Cross-validating the model with test data, a correlation coefficient of R=64% was achieved (2605). The empirical standard deviation of the model prediction is shown (2606) superimposed against the histogram of outcomes that were used to train the model (2607). These statistics can be used to make an informed treatment decision, such as to forgo irinotecan treatment completely or to administer a second drug, such as granulocyte colony stimulating factor, to prevent a low ANC and resultant infections.

*Enhanced Diagnostic Reporting*

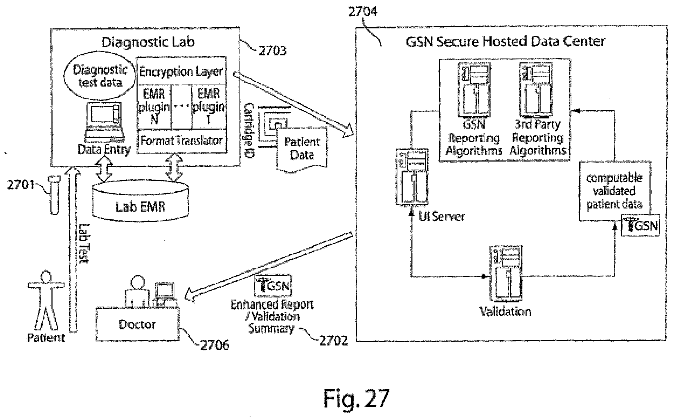

In the context of disease treatment, the generated phenotypic data is of most use to a clinician who can use the data to aid in selecting a treatment regimen. In one aspect, the phenotypic predictions will be contextualized and organized into a report for the clinician or patient. In another aspect, the system and method disclosed herein could be used as part of a larger system (see Figure 27) wherein a diagnostic lab 2703 validates data from lab tests 2701 and medical reports 2702, and sends it to a data center 2704 where it is integrated into a standard ontology, analyzed using the disclosed method, and an enhanced diagnostic report 2705 could be generated and sent to the physician 2706.

One possible context in which a report may be generated would be related to predicting clinical outcomes for colon cancer patients being treated with irinotecan. It may take into consideration concepts such as contraindications for treatment, dosing schedules, side effect profiles. Examples of such side effects include myelosuppression and late-onset diarrhea which are two common, dose-limiting side effects of irinotecan treatment which require urgent medical care. In addition, severe neutropenia and severe diarrhea affect 28% and 31% of patients, respectively. Certain UGT1A1 alleles, liver function tests, past medical history of Gilbert's Syndrome, and identification of patient medications that induce cytochrome p450, such as anti-convulsants and some anti-emetics, are indicators warranting irinotecan dosage adjustment.

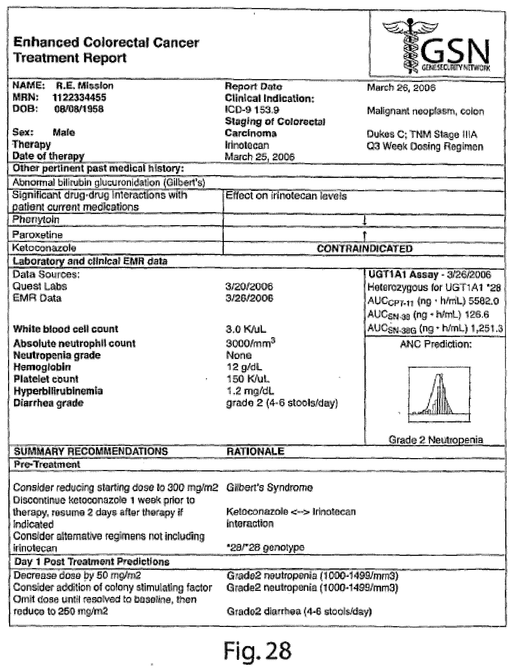

Figure 28 is a mock-up of an enhanced report for colorectal cancer treatment with irinotecan that makes use of phenotype prediction. Prior to treatment, the report takes into account the patient's cancer stage, past medical history, current medications, and UGT1A1 genotype to recommend drug dosage. Roughly one data after the first drug dosage, the report includes a prediction of the expected Nadir of the patient's absolute neutrophil count in roughly two weeks time, based on the mutations in the UGT1A1 gene and metabolites (e.g. SN-38, CPT-11) measured from the patient's blood. Based on this prediction, the doctor can make a decision whether to give the patient colony stimulating factor drugs, or change the Irinotecan dosage. The patient is also monitored for blood counts, diarrhea grade. Data sources and justification for recommendations are provided.

*Combinations of the Aspects*

As noted previously, given the benefit of this disclosure, other aspects, features and embodiments may implement one or more of the methods and systems disclosed herein. Below is a short list of examples illustrating situations in which the various disclosures can be combined in a plurality of ways.

One example could utilize a variety of genotyping measurement techniques in a way that would optimize the value of each. For example, a lab could use an technique that is expensive but can give high quality data in cases with low signal, such as AppliedBioscience's Taqman assay, to measure the target DNA, and use a technique that is less expensive but requires a greater amount of genetic material to give good quality data, such as Affymetrix's 500K Genechip, or MIPS, to measure the parental DNA.

Another example could be a situation in which a couple undergoing IVF treatment have eggs harvested from the woman, and fertilized with sperm from the man, producing eight viable embryos. A blastocyst is harvested from each embryo, and the genomic data from the blastocysts are measured using Taqman Genotyping Assay. Meanwhile, the diploid data is measured from tissue taken from both parents using Molecular Inversion Probes. Haploid data from one of the man's sperm, and one of the woman's eggs is also measured using MIPs. The genetic data of the parents is used to clean the SNP data of the eight blastocysts. The cleaned genetic data is then used to allow predictions to be made concerning the potential phenotypes of the embryos. Two embryos are selected which have the most promising profile, and allowed to implant in the woman's uterus.

Another example could be a situation where a pregnant woman whose husband has a family history of Tay-Sachs disease wants to know if the fetus she is carrying is genetically susceptible, but she does not want to undergo amniocentesis, as it carries a significant risk of miscarriage. She has her blood drawn, some fetal DNA is isolated from her blood, and that DNA is analyzed using MIPs. She and her husband had already had their full genomic data analyzed previously and it is available *in silico.* The doctor is able to use the *in silico* knowledge of the parental genomes and the method disclosed herein to clean the fetal DNA data, and check if the critical gene that is responsible for Tay-Sachs disease is present in the genome of the fetus.

Another example could be a situation where a 44-year old pregnant woman is concerned that the fetus she is carrying may have Downs Syndrome. She is wary of having an intrusive technique used for pre-natal diagnosis, given a personal history of miscarriages, so she chooses to have her blood analyzed. The health care practitioner is able to find fetal cells in the maternal blood sample, and using the method disclosed herein, together with the knowledge of the woman's own genetic data, is able to diagnose for aneuploidy.

Another example could be a situation where a couple are undergoing IVF treatment; they have eggs harvested from the woman, and fertilized with sperm from the man, producing nine viable embryos. A blastocyst is harvested from each embryo, and the genomic data from the blastocysts are measured using an Illumina Bead Assay. Meanwhile, the diploid data is measured from tissue taken from both parents using Molecular Inversion Probes. Haploid data from the father's sperm is measured using the same method. There were no extra eggs available from the mother, so bulk diploid tissue samples are taken from her own father and mother, and a sperm sample from her father. They are all analyzed using MIPs and the method disclosed herein is used to provide a genetic analysis for the mother's genome. That data is then used, along with the father's diploid and haploid data, to allow a highly accurate analysis of the genetic data of each of the blastocysts. Based on the phenotypic predictions, the couple chooses three embryos to implant.

Another example could be a situation where a racehorse breeder wants to increase the likelihood that the foals sired by his champion racehorse become champions themselves. He arranges for the desired mare to be impregnated by IVF, and uses genetic data from the stallion and the mare to clean the genetic data measured from the viable embryos. The cleaned embryonic genetic data allows the breeder to find relevant genotypic-phenotypic correlations and select the embryos for implantation that are most likely to produce a desirable racehorse.

Another example could be a situation where a pregnant woman wants to know whether the fetus she is carrying is predisposed towards any serious illness. The father has since passed away, and so the haploid and diploid data generated from the father's brother and the father's father are used to help clean the genetic data of the fetus, measured from fetal cells gathered during fetal blood sampling. A company contracted by the health care practitioner uses the cleaned fetal genetic data to provide a list of phenotypes that the fetus is likely to exhibit, along with the confidence of each prediction.

Another example could be an amniocentesis lab that must occasionally contend with contaminated fetal genetic data due to poor laboratory techniques. The disclosed method could be used to clean the contaminated fetal genetic data using maternal and paternal genetic data. One could imagine a situation where a laboratory is able to cut costs by relaxing sterility procedures, knowing that the disclosed method would be able to compensate for an increased rate of contaminating DNA.

Another example could be a situation in which a woman in her forties is undergoing IVF to get pregnant. She wants to screen the embryos to select the one(s) that are least likely to have a genetic illness, and are most likely to implant and carry to term. The IVF clinic she is using harvests a blastocyst from each of the viable embryos, and uses standard procedures to amplify the DNA, and measure key SNPs. The technician then uses the methods disclosed herein to screen for chromosomal imbalances, and also to find and clean the genetic data of the embryos to make predictions about the phenotypic predispositions of each embryo.

Another example could be a situation where a pregnant woman has amniocentesis, and the genetic material in the fetal cells in the blood sample are used, along with the methods described herein to screen for aneuploidy and other chromosomal abnormalities.

One example could be a situation in which a non-linear model using Support Vector Machine with radial basis kernel functions and a norm loss function utilizes genetic and phenotypic data of a human adult to predict the likelihood of early onset Alzheimer's disease, and to suggest possible lifestyle changes and exercise regimens which may delay the onset of the disease.

Another example could be a situation in which a linear model using the LASSO technique utilizes the genetic and phenotypic data of an adult woman afflicted with lung cancer, along with genetic data of the cancer to generate a report for the woman's physicians predicting which pharmaceuticals will be most effective in delaying the progression of the disease.

Another example could be a situation in which a plurality of models are tested on aggregated data consisting of genetic, phenotypic and clinical data of Crohn's disease patients, and then the non-linear regression model that is found to be the most accurate utilizes the phenotypic and clinical data of an adult man to generate a report suggesting certain nutritional supplements that are likely to alleviate the symptoms of his Crohn's disease.

Another example could be a situation in which a model utilizing contingency tables built from data acquired through the Hapmap project, and utilizing genetic information gathered from a blastocyst from an embryo are used to make predictions regarding likely phenotypes of a child which would result if the embryo were implanted.

Another example could be a situation where linear regression models utilizing genetic information of the strain of HIV infecting a newborn are used to generate a report for the baby's physician suggesting which antiretroviral drugs give her the greatest chance of reaching adulthood if administered.

Another example could be a situation where a new study is published suggesting certain correlations between the prevalence of myocardial infarctions in middle aged women and certain genetic and phenotypic markers. This then prompts the use of a non-linear regression model to reexamine the aggregate data of middle aged data, as well as genetic and phenotypic data of identified individuals whose data is known to the system, and the model then identifies those women who are most at risk of myocardial infarctions, and generates reports that are sent to the women's respective physicians informing them of the predicted risks.

Another example could be a situation where a plurality of models are tested on aggregated data of people suffering from colon cancer, including the various drug interventions that were attempted. The model that is found to allow the best predictions is used to identify the patients who are most likely to benefit from an experimental new pharmaceutical, and those results are used by the company which owns the rights to the new pharmaceutical to aid them in conducting their clinical trials.

*Definitions*

SNP (Single Nucleotide Polymorphism): A specific locus on a chromosome that tends to show inter-individual variation.

To call a SNP: to interrogate the identity of a particular base pair, taking into account the direct and indirect evidence.

To call an allele: to call a SNP.

To clean genetic data: to take imperfect genetic data and correct some or all of the errors, using genetic data of related individuals and the method describe herein.

Imperfect genetic data: genetic data with any of the following: allele dropouts, unclear base pair measurements, incorrect base pair measurements, spurious signals, or missing measurements.

Confidence: the statistical likelihood that the called SNP, allele, or set of alleles correctly represents the real genetic state of the individual.

Multigenic: affected by multiple genes, or alleles.

Noisy genetic data: incomplete genetic data, also called incomplete genetic data.;

Uncleaned genetic data: genetic data as measured, that is, with no method has been used to correct for the presence of noise in the raw genetic data; also called crude genetic data.

Direct relation: mother, father, son, or daughter.

Chromosomal Region: a segment of a chromosome, or a full chromosome.

Parental Support: the name sometimes used for the disclosed method of cleaning genetic data. Section of a chromosome: a section of a chromosome that can range in size from one base pair to the entire chromosome.

Table 1

| OMIM Summary of disease-linked genes | | | | | |
|---|---|---|---|---|---|
| | Autosomal | X-Linked | Y-Linked | Mitochondrial | Total |
| * Gene with known sequence | 9916 | 457 | 48 | 37 | 10458 |
| Gene with known sequence and phenotype | 359 | 30 | 0 | 0 | 389 |
| # Phenotype description, molecular basis known | 1652 | 147 | 2 | 26 | 1827 |
| % Mendelian phenotype or locus, molecular basis unknown | 1362 | 135 | 4 | 0 | 1501 |
| Phenotypes with suspected Mendelin basis | 2092 | 147 | 2 | 0 | 2241 |
| Total | 15381 | 916 | 56 | 63 | 16416 |

Table 2

| Technique Descrintion | Formula Applied to compute N= | Assay Name | E(m1) | E(m0) | Std(m1) | Std(m0) | Copy No. Compare | Mosaicism | N | Price per SNP | Additional Costs | Factor Sigma | 1-Conf | Segments | Cells per Well | Total Cells w/o PCR | Cost |
|---|---|---|---|---|---|---|---|---|---|---|---|---|---|---|---|---|---|
| | | | | | | | | | | | | | | | | | |
| | | | | | | | | | | | | | | | | | |
| | | | | | | | | Features of Different Techniques of Screening for Aneuploidy | | | | | | | | | |
| Comp of means no reference sample | 2^2*5^2*s^2/(mf-mm)^2 | Taqman | 32.26 | 30.75 | 0.597 | 0.597 | 2 vs 1 | 100.0% | 16 | $0.20 | 30 | 5 | 287507 | 5 | 50 | 800 | $46.00 |
| Comp of means with ref. sample | 2^2*5^2*(2*s^2)/m1 -m0)^2 | Taqman | 32.26 | 30.75 | 0.597 | 0.597 | 2 vs 1 | 100.0% | 32 | $0.20 | 30 | 5 | 287507 | 5 | 50 | 1600 | $62.00 |
| Comp of means no reference sample | 4*(2^2)* (0.95*s^2+0 .05*s^2)/(0.05^2*(m 1-m0)^2) | Taqman | 32.26 | 30.75 | 0.597 | 0.597 | 2 vs 1 | 50% | 1001 | $0.20 | 30 | 2 | 230502 | 5 | 50 | 50050 | $1,031.00 |
| Comp of means with ref. sample | 4*(2^2)* (1.95*s^2+0 .05*s^2)/(0.05^2*(m 1-m0)^2) | Taqman | 32.26 | 30.75 | 0.597 | 0.597 | 2 vs 1 | 50% | 2002 | $0.20 | 30 | 2 | 230502 | 5 | 50 | 100100 | $2,032.00 |
| Comp of means no reference sample | 4*(2^2)* (0.95*s^2+0 .05*s^2)/(0.05^2*(m 1-m0)^2) | MPs | 32.26 | 30.75 | 0.597 | 0.597 | 2 vs 1 | 50% | 1001 | $0.01 | 200 | 2 | 230502 | 5 | 50 | 50050 | $250.05 |
| Comp of means with ref. sample | 4*(2^2)* (1.95*s^2+0 .05*s^2)/(0.05^2*(m 1-m0)^2) | MPs | 32.26 | 30.75 | 0.597 | 0.597 | 2 vs 1 | 50% | 2002 | $0.01 | 200 | 2 | 230502 | 5 | 50 | 100100 | $300.10 |
| Comp of means no reference sample | 4*(2^2)*((1-ff)*s^2+ff*s^2)/(ff^2 * (m1-m0)^2); ff=0.081 | Taqman | 32.26 | 30.75 | 0.597 | 0.597 | 2 vs 1 | 8.1% | 384 | $0.20 | 30 | 2 | 230502 | 5 | 50 | 19200 | $414.00 |
| Comp of means no reference sample | 4*(1^2)*((1-ff)*s^2+ff*s^2)/(ff^2 * (m1-m0)^2); ff=0.058 | Taqman | 32.26 | 30.75 | 0.597 | 0.597 | 2 vs 1 | 4.1% | 384 | $0.20 | 30 | 1 | 1.59601 | 5 | 50 | 19200 | $414.00 |

| Features of Different Techniques of Screening for Aneuploidy | | | | | | | | | | | | | | | | | | |
|---|---|---|---|---|---|---|---|---|---|---|---|---|---|---|---|---|---|---|
| Technique Descrintion | Formula Applied to compute N= | Assay Name | E(m1) | E(m0) | Std(m1) | Std(m0) | Copy No. Compare | Mosaicism | N | Price per SNP | Additional Costs | Factor Sigma | 1-Conf | Segments | Cells per Well | Total Cells w/o PCR | Cost |
| Comp of means no reference sample | $4*(2\wedge2)*((1-ff)*s\wedge2+ff*s\wedge2)/(ff\wedge2 * (m1-m0)\wedge2)$; ff=0.19 | Taaman | 32.26 | 30.75 | 0.597 | 0.597 | 2 vs 1 | 19.0% | 70 | $0.20 | 30 | 2 | 230502 | 5 | 50 | 3600 | $100.00 |
| Comp of means no reference sample | $2\wedge2*5\wedge2*s\wedge2/(m\ 1-m0)\wedge2$ | qPCR | 27.65 | 26.64 | 0.53 | 0.53 | 2 vs 1 | 100.0% | 27 | $0.15 | 30 | 5 | 287507 | 5 | 50 | 1350 | $50.25 |
| Comp of means with ref. sample | $2\wedge2*5\wedge2*(2*s\wedge2)/(m\ 1-m0)\wedge2$ | qPCR | 27.65 | 26.64 | 0.53 | 0.53 | 2 vs 1 | 100.0% | 55 | $0.15 | 30 | 5 | 287507 | 5 | 50 | 2750 | $71.25 |
| Comp of means no reference sample | $4*(2\wedge2)* (0.95*s\wedge2+0\ .05*s\wedge2)/(0.05\wedge2*(m\ 1-m0)\wedge2)$ | qPCR | 27.65 | 26.64 | 0.53 | 0.53 | 2 vs 1 | 50% | 1754 | $0.15 | 30 | 2 | 230502 | 5 | 50 | 87700 | $1,345.50 |
| Comp of means with ref. sample | $4*(2\wedge2)*(1.95*s\wedge2+0\ .05*s\ 2)/(0.05\wedge2*(m\ 1-m0)\wedge2)$ | aPCR | 27.65 | 26.64 | 0.53 | 0.53 | 2 vs 1 | 50% | 3508 | $0.15 | 30 | 2 | 230502 | 5 | 50 | 175400 | $2,661.00 |
| Comp of means no reference sample | $4*(2\wedge2)*(1.ff)*s\wedge2+ff\ *s\wedge2)/(ff\wedge2* (m1-m0)\wedge2)$; ff=0.107 | aPCR | 27.65 | 26.64 | 0.53 | 0.53 | 2 vs 1 | 10.7% | 384 | $0.15 | 30 | 2 | 230502 | 5 | 50 | 19200 | $318.00 |
| Comp of means no reference sample | $4*(1\wedge2)*(1.ff)*s\wedge2+ff\ *s\wedge2)/(ff\wedge2* (m1-m0)\wedge2)$; ff=0.054 | qPCR | 27.65 | 26.64 | 0.53 | 0.53 | 2 vs 1 | 5.4% | 384 | $0.15 | 30 | 1 | 1.59601 | 5 | 50 | 19200 | $318.00 |
| Comp of means no reference sample | $4*(2\wedge2)*(1.ff)*s\wedge2+ff\ *s\wedge2)/(ff\wedge2* (m1-m0)\wedge2)$; ff=0.19 | qPCR | 27.65 | 26.64 | 0.53 | 0.53 | 2 vs 1 | 19.0% | 121 | $0.15 | 30 | 2 | 230502 | 5 | 50 | 6050 | $120.75 |

EP 3 373 175 B1

70

Table 3

| snp_id | e1 | e2 | p1 | p2 | m1 | m2 | pe1 | pe2 | pp1 | pp2 | pm1 | pm2 |
|--------|----|----|----|----|----|----|-----|-----|-----|-----|-----|-----|
| 101100940 | C | T | T | C | C | T | 0.9538 | 0.8902 | 0.8626 | 0.8580 | 0.8654 | 0.9101 |
| 101164838 | T | C | T | C | T | C | 0.9359 | 0.9521 | 0.9406 | 0.9253 | 0.9957 | 0.8770 |
| rs1463589 | C | C | T | C | C | C | 0.9428 | 0.9928 | 0.9841 | 0.9266 | 0.8661 | 0.9798 |
| 101028396 | C | G | C | G | C | C | 0.9252 | 0.8792 | 0.9246 | 0.9856 | 0.9819 | 0.8631 |
| 101204217 | A | G | G | A | G | G | 0.9799 | 0.9843 | 0.9194 | 0.9478 | 0.9438 | 0.9709 |
| 101214313 | A | G | G | A | G | A | 0.8513 | 0.9863 | 0.9521 | 0.9707 | 0.8570 | 0.9639 |
| 101231593 | G | A | G | G | A | A | 0.9857 | 0.9653 | 0.8908 | 0.9036 | 0.9431 | 0.9832 |
| rs1426442 | G | C | C | G | C | G | 0.9338 | 0.9278 | 0.9469 | 0.9514 | 0.8766 | 0.9017 |
| rs7486852 | C | C | C | T | T | C | 0.9566 | 0.9616 | 0.9390 | 0.8673 | 0.8785 | 0.8889 |
| 101266729 | A | G | A | G | A | G | 0.9238 | 0.9500 | 0.9026 | 0.9855 | 0.8760 | 0.9381 |

Table 4

| snp_id | e1 | e2 | p1 | p2 | m1 | m2 | pe1 | pe2 | pp1 | pp2 | pm1 | pm2 |
|--------|----|----|----|----|----|----|-----|-----|-----|-----|-----|-----|
| 101019515 | G | G | G | G | G | G | 0.9134 | 0.8768 | 0.8666 | 0.9690 | 0.8679 | 0.8599 |
| 101100940 | C | T | T | C | C | T | 0.9538 | 0.8902 | 0.8626 | 0.8580 | 0.8654 | 0.9101 |
| 101160854 | A | A | A | A | A | A | 0.8705 | 0.9769 | 0.8763 | 0.8870 | 0.9311 | 0.9553 |
| rs4980809 | A | G | G | A | A | G | 0.9638 | 0.9951 | 0.9582 | 0.9621 | 0.9197 | 0.9199 |
| 101058479 | G | A | G | A | G | A | 0.9003 | 0.9885 | 0.8906 | 0.9235 | 0.9787 | 0.8792 |
| 101236938 | G | G | G | G | G | A | 0.8528 | 0.9710 | 0.8810 | 0.9249 | 0.9274 | 0.9891 |
| rs7137405 | T | T | T | T | T | A | 0.9360 | 0.9918 | 0.9148 | 0.9558 | 0.9135 | 0.9388 |
| 101251161 | G | G | G | G | G | G | 0.9802 | 0.8620 | 0.9372 | 0.8501 | 0.9891 | 0.8679 |
| 101270051 | G | G | G | G | G | A | 0.9004 | 0.9643 | 0.9778 | 0.9060 | 0.9943 | 0.8962 |
| rs215227 | G | G | G | G | G | A | 0.9244 | 0.9236 | 0.9629 | 0.8575 | 0.9019 | 0.9362 |
| 101245075 | G | G | G | G | G | G | 0.9958 | 0.8593 | 0.9129 | 0.8504 | 0.8534 | 0.9866 |
| 101158538 | A | G | A | G | G | G | 0.9471 | 0.8909 | 0.8710 | 0.9581 | 0.8961 | 0.9046 |
| rs2535386 | A | A | A | A | A | A | 0.9273 | 0.9479 | 0.9867 | 0.8918 | 0.9264 | 0.9750 |
| rs6489653 | T | T | T | T | T | T | 0.9453 | 0.9776 | 0.9051 | 0.8547 | 0.9636 | 0.9532 |
| 101137205 | C | G | C | C | G | G | 0.8619 | 0.9503 | 0.9029 | 0.9426 | 0.8845 | 0.9282 |
| 101089311 | T | C | C | C | C | T | 0.8844 | 0.9381 | 0.9719 | 0.8636 | 0.9186 | 0.9652 |
| 101205712 | A | A | A | A | A | A | 0.8513 | 0.9226 | 0.8755 | 0.8999 | 0.9193 | 0.8535 |
| 101124605 | G | G | G | G | G | G | 0.8981 | 0.9093 | 0.9075 | 0.8676 | 0.8931 | 0.9258 |
| 101025989 | G | T | T | G | G | T | 0.9695 | 0.9016 | 0.8722 | 0.8821 | 0.9787 | 0.9273 |
| rs4766370 | T | A | A | T | T | A | 0.8886 | 0.9166 | 0.8762 | 0.8767 | 0.9890 | 0.8536 |

Table 5

| snp_id | true_value | true_hyp | ee | pp | mm | SnipProb | HypProb |
|--------|-----------|----------|----|----|----|----------|---------|
| 101100940 | CT | p2 m2 | CT | TC | CT | 0.8416 | 0.5206 |
| 101164838 | CT | p2 m1 | TC | TC | TC | 0.9061 | 0.5206 |
| rs1463589 | CC | p2 m1 | CC | TC | CC | 0.9946 | 0.5206 |
| 101028396 | GC | p2 m1 | CG | CG | CC | 0.9791 | 0.5206 |
| 101204217 | AG | p2 m2 | AG | GA | GG | 0.9577 | 0.5206 |
| 101214313 | GA | p1 m2 | AG | GA | GA | 0.9308 | 0.5206 |
| 101231593 | GA | p1 m2 | GA | GG | AA | 1.0000 | 0.5206 |
| rs1426442 | CG | p1 m2 | GC | CG | CG | 0.9198 | 0.5206 |
| rs7486852 | CC | p1 m2 | CC | CT | TC | 0.9138 | 0.5206 |
| 101266729 | AG | p1 m2 | AG | AG | AG | 0.9296 | 0.5206 |

71

Table 6

| snp_id | true_value | true_hyp | ee | pp | mm | SnipProb | HypProb |
|--------|-----------|----------|-----|-----|-----|----------|---------|
| 101019515 | GG | p1 m1 | GG | GG | GG | 1.0000 | 0.9890 |
| 101100940 | TC | p1 m1 | CT | TC | CT | 0.9946 | 0.9890 |
| 101160854 | AA | p1 m1 | AA | AA | AA | 1.0000 | 0.9890 |
| rs4980809 | GA | p1 m1 | AG | GA | AG | 0.9961 | 0.9890 |
| 101058479 | GG | p1 m1 | GA | GA | GA | 0.9957 | 0.9890 |
| 101236938 | GG | p1 m1 | GG | GG | GA | 1.0000 | 0.9890 |
| rs7137405 | TT | p1 m1 | TT | TT | TA | 1.0000 | 0.9890 |
| 101251161 | GG | p1 m1 | GG | GG | GG | 1.0000 | 0.9890 |
| 101270051 | GG | p1 m1 | GG | GG | GA | 1.0000 | 0.9890 |
| rs215227 | GG | p1 m1 | GG | GG | GA | 1.0000 | 0.9890 |
| 101245075 | GG | p1 m1 | GG | GG | GG | 1.0000 | 0.9890 |
| 101158538 | AG | p1 m1 | AG | AG | GG | 0.9977 | 0.9890 |
| rs2535386 | AA | p1 m1 | AA | AA | AA | 1.0000 | 0.9890 |
| rs6489653 | TT | p1 m1 | TT | TT | TT | 1.0000 | 0.9890 |
| 101137205 | CG | p1 m1 | CG | CC | GG | 1.0000 | 0.9890 |
| 101089311 | CC | p1 m1 | TC | CC | CT | 0.9940 | 0.9890 |
| 101205712 | AA | p1 m1 | AA | AA | AA | 1.0000 | 0.9890 |
| 101124605 | GG | p1 m1 | GG | GG | GG | 1.0000 | 0.9890 |
| 101025989 | TG | p1 m1 | GT | TG | GT | 0.9973 | 0.9890 |
| rs4766370 | AT | p1 m1 | TA | AT | TA | 0.9973 | 0.9890 |

Table 7

| | | | DHAlgorithm1 | | DHAlgorithm2 | |
|---------|------|------|-----------|-----------|-----------|-----------|
| Pop.Freq | ph | pd | P1accuracy | P2accuracy | P1accuracy | P2accuracy |
| data | 0.95 | 0.95 | 0.982 | 0.951 | 0.95 | 0.906 |
| data | 0.75 | 0.75 | 0.891 | 0.811 | 0.749 | 0.618 |
| data | 0.25 | 0.25 | 0.71 | 0.71 | 0.253 | 0.25 |
| data | 0.5 | 0.9 | 0.849 | 0.838 | 0.499 | 0.768 |
| data | 0.9 | 0.5 | 0.942 | 0.734 | 0.898 | 0.347 |
| data | 0.6 | 0.8 | 0.852 | 0.816 | 0.601 | 0.673 |
| uniform | 0.95 | 0.95 | 0.95 | 0.906 | 0.949 | 0.905 |
| uniform | 0.75 | 0.75 | 0.749 | 0.612 | 0.749 | 0.612 |
| uniform | 0.25 | 0.25 | 0.25 | 0.248 | 0.25 | 0.25 |
| uniform | 0.5 | 0.9 | 0.69 | 0.669 | 0.501 | 0.671 |
| uniform | 0.9 | 0.5 | 0.901 | 0.412 | 0.901 | 0.413 |
| uniform | 0.6 | 0.8 | 0.678 | 0.618 | 0.6 | 0.618 |

Table 8

| | | | | PSAlgorithm1 | | PSAlgorithm2 | |
|---------|------|------|------|-----------|-----------|-----------|-----------|
| Pop.Freq | ph | pd | pe | P1accuracy | P2accuracy | P1accuracy | P2accuracy |
| data | 0.95 | 0.95 | 0.95 | 0.834 | 0.815 | 0.928 | 0.931 |
| data | 0.75 | 0.75 | 0.75 | 0.797 | 0.769 | 0.819 | 0.819 |
| data | 0.25 | 0.25 | 0.25 | 0.711 | 0.682 | 0.703 | 0.687 |
| data | 0.5 | 0.9 | 0.9 | 0.849 | 0.838 | 0.866 | 0.864 |
| data | 0.9 | 0.5 | 0.5 | 0.792 | 0.809 | 0.756 | 0.752 |
| data | 0.6 | 0.8 | 0.8 | 0.777 | 0.788 | 0.835 | 0.828 |
| uniform | 0.95 | 0.95 | 0.95 | 0.673 | 0.631 | 0.898 | 0.901 |

(continued)

| Pop.Freq | ph | pd | pe | PSAlgorithm1 | | PSAlgorithm2 | |
|---|---|---|---|---|---|---|---|
| | | | | P1accuracy | P2accuracy | P1accuracy | P2accuracy |
| uniform | 0.75 | 0.75 | 0.75 | 0.549 | 0.497 | 0.635 | 0.65 |
| uniform | 0.25 | 0.25 | 0.25 | 0.239 | 0.249 | 0.252 | 0.25 |
| uniform | 0.5 | 0.9 | 0.9 | 0.601 | 0.611 | 0.814 | 0.818 |
| uniform | 0.9 | 0.5 | 0.5 | 0.459 | 0.391 | 0.449 | 0.468 |
| uniform | 0.6 | 0.8 | 0.8 | 0.544 | 0.511 | 0.672 | 0.679 |

Table 9

| Farrer (2005) | | | | Alvarez (1999) | D+ | D- |
|---|---|---|---|---|---|---|
| | D+ | D- | | A+ | 161/350 | 176/400 |
| A+B+ | 56/151 | 25/206 | | A- | 189/350 | 224/400 |
| A+B- | 26/151 | 69/206 | | Labert (1998) | D+ | D- |
| A-B+ | 43/151 | 20/206 | | B+ | 334/573 | 104/509 |
| A-B- | 26/151 | 20/206 | | B- | 239/573 | 405/509 |

Table 10

| prob. | | | | | | | | |
|---|---|---|---|---|---|---|---|---|
| P(A+B+/D+) | 0.28 | 0.024 | 0.24 | 0.32 | 0.37 | 0.039 | 0.29 | 0.45 |
| P(A+B+/D-) | 0.18 | 0.016 | 0.15 | 0.21 | 0.17 | 0.026 | 0.12 | 0.22 |
| P(A+B-/D+) | 0.22 | 0.027 | 0.16 | 0.27 | 0.28 | 0.037 | 0.21 | 0.35 |
| P(A+B-/D-) | 0.18 | 0.018 | 0.15 | 0.22 | 0.12 | 0.023 | 0.08 | 0.16 |
| P(A-B+/D+) | 0.31 | 0.026 | 0.26 | 0.36 | 0.33 | 0.038 | 0.26 | 0.4 |
| P(A-B+/D-) | 0.05 | 0.017 | 0.02 | 0.09 | 0.1 | 0.021 | 0.06 | 0.14 |
| P(A-B-/D+) | 0.2 | 0.032 | 0.13 | 0.26 | 0.02 | 0.011 | 0 | 0.04 |
| P(A-B-/D-) | 0.58 | 0.034 | 0.52 | 0.65 | 0.61 | 0.034 | 0.64 | 0.68 |

Table 11

| Method | Meas | APV | ATV | NFV | RTV | SQV | LPV | IDV | Average |
|---|---|---|---|---|---|---|---|---|---|
| RR | Mean | 79.30 | 69.44 | 78.37 | 87.96 | 82.93 | 75.35 | 82.81 | 79.45 |
| | Std. dev | 0.26 | 0.90 | 1.18 | 0.49 | 0.60 | 1.36 | 0.65 | 0.86 |
| NN | Mean | 83.16 | 68.45 | 87.59 | 90.88 | 89.10 | 73.67 | 89.07 | 83.13 |
| | Std. dev | 0.52 | 1.22 | 0.19 | 0.36 | 0.15 | 0.82 | 0.10 | 0.61 |
| PCA | Mean | 86.49 | 73.52 | 87.65 | 91.98 | 88.72 | 84.79 | 87.86 | 85.86 |
| | Std. dev | 0.19 | 0.79 | 0.14 | 0.12 | 0.12 | 0.26 | 0.08 | 0.33 |
| DT | Mean | 77.32 | 65.12 | 83.51 | 85.57 | 83.63 | 89.05 | 82.54 | 78.11 |
| | Std. dev | 0.33 | 0.93 | 0.15 | 0.21 | 0.31 | 0.87 | 0.34 | 0.54 |
| SS | Mean | 85.25 | 73.27 | 89.18 | 91.65 | 89.81 | 77.15 | 87.79 | 84.87 |
| | Std. dev | 0.03 | 1.02 | 0.14 | 0.10 | 0.08 | 0.39 | 0.08 | 0.42 |
| SVM L | Mean | 87.48 | 75.24 | 88.95 | 92.24 | 89.53 | 87.13 | 88.12 | 86.96 |
| | Std. dev | 0.15 | 1.05 | 0.08 | 0.09 | 0.10 | 0.23 | 0.10 | 0.42 |

(continued)

| Method | Meas | APV | ATV | NFV | RTV | SQV | LPV | IDV | Average |
|---|---|---|---|---|---|---|---|---|---|
| LASSO | Mean | 89.17 | 76.60 | 89.88 | 93.47 | 91.00 | 86.78 | 88.61 | 87.93 |
| | Std. dev | 0.08 | 0.62 | 0.07 | 0.16 | 0.07 | 0.17 | 0.07 | 0.26 |
| SVM R | Mean | 88.22 | 82.27 | 89.92 | 92.29 | 89.48 | 86.19 | 90.28 | 83.38 |
| | Std. dev | 0.03 | 0.46 | 0.06 | 0.07 | 0.08 | 0.32 | 0.08 | 0.22 |
| Samples | | 577 | 142 | 617 | 510 | 598 | 253 | 579 | |

Table 12

| Method | Meas | 3TC | ABC | AZT | D4T | DDC | DDI | DLV | EFV | NVP | TDF | Average |
|---|---|---|---|---|---|---|---|---|---|---|---|---|
| RR | Mean | 87.28 | 74.56 | 78.3 | 79.78 | 70.96 | 69.65 | 80.86 | 78.12 | 73.47 | 71.85 | 76.43 |
| | Std. dev | 0.72 | 1.15 | 0.34 | 0.61 | 1.06 | 0.66 | 0.17 | 0.29 | 0.63 | 0.56 | 0.69 |
| NN | Mean | 94.33 | 74.96 | 71.12 | 80.29 | 73.42 | 65.79 | 84.15 | 80.5 | 84.05 | 68.71 | 77.73 |
| | Std. dev | 0.26 | 0.50 | 2.45 | 0.82 | 0.89 | 1.99 | 0.43 | 0.60 | 0.50 | 1.23 | 1.18 |
| PCA | Mean | 92.23 | 83.3 | 84.14 | 88.83 | 85.28 | 85.19 | 80.88 | 77.43 | 77.34 | 68.36 | 82.30 |
| | Std. dev | 0.11 | 0.23 | 0.16 | 0.17 | 0.12 | 0.23 | 0.21 | 0.25 | 0.32 | 0.38 | 0.34 |
| DT | Mean | 94.86 | 81.93 | 81.32 | 80.87 | 73.86 | 78.36 | 81.92 | 76.78 | 85.96 | 58.91 | 79.48 |
| | Std. dev | 0.22 | 0.31 | 0.56 | 0.59 | 0.70 | 0.61 | 0.30 | 0.92 | 0.36 | 2.05 | 0.84 |
| SS | Mean | 94.91 | 94.43 | 81.86 | 86.88 | 86.99 | 82.17 | 83.97 | 80.15 | 76.5 | 67.27 | 82.52 |
| | Std. dev | 0.07 | 0.22 | 0.42 | 0.32 | 0.17 | 0.67 | 0.21 | 0.38 | 0.56 | 1.39 | 0.56 |
| SVM_L | Mean | 94.31 | 85.27 | 84.04 | 89.09 | 85.6 | 83.53 | 87.07 | 83.78 | 78.77 | 67.9 | 83.94 |
| | Std. dev | 0.12 | 0.21 | 0.21 | 0.15 | 0.17 | 0.32 | 0.16 | 0.24 | 0.46 | 1.57 | 0.58 |
| LASSO | Mean | 95.48 | 86.32 | 85.54 | 91.37 | 87.54 | 85.74 | 85.16 | 82.31 | 82.19 | 70.58 | 85.22 |
| | Std. dev | 0.07 | 0.16 | 0.11 | 0.10 | 0.14 | 0.24 | 0.12 | 0.20 | 0.20 | 0.41 | 0.20 |
| SVM_R | Mean | 95.94 | 86.84 | 89.39 | 89.30 | 86.29 | 85.40 | 86.33 | 84.61 | 85.38 | 78.75 | 86.82 |
| | Std. dev | 0.11 | 0.19 | 0.13 | 0.12 | 0.26 | 0.23 | 0.20 | 0.28 | 0.27 | 0.49 | 0.25 |
| Samples | | 356 | 354 | 353 | 356 | 319 | 354 | 395 | 384 | 401 | 96 | |

Table 13

| Drug | APV | ATV | NFV | RTV | SQV | LVP | IDV |
|---|---|---|---|---|---|---|---|
| #Samples | 577 | 142 | 617 | 510 | 598 | 253 | 579 |
| #Mutations | 320 | 320 | 320 | 320 | 320 | 320 | 320 |
| #Predictions | 120 | 50 | 90 | 120 | 120 | 100 | 110 |

Table 14

| Drug | 3TC | ABC | AZT | D4T | DDC | DDI | DLV | EFV | NVP | TDF |
|---|---|---|---|---|---|---|---|---|---|---|
| #Samples | 356 | 354 | 353 | 356 | 319 | 354 | 395 | 384 | 401 | 96 |
| #Mutations | 791 | 791 | 791 | 791 | 791 | 791 | 791 | 791 | 791 | 791 |
| #Predictors | 50 | 70 | 100 | 110 | 80 | 100 | 170 | 120 | 120 | 60 |

Table 15

| Subject ID | PharmGKB Accession IDs for subjects in the PharmGKB |
|---|---|
| Gender | Male, female, or unknown |
| Race/Ethnicity | As defined by the NIH Office of Management and Budget with self-reported information in parenthesis |
| Dose (mg/m2) | Adjusted dose of irinotecan |
| BSA (m2) | Body Surface Area in meters squared |
| Dose (mg) | Dose of irinotecan in milligrams |
| CPT 11AUC24 | Irinotecan, SN-38, and SN-38 G area under concentration curve from 0 to 24 hours, on day 1 of cycle 1, measured in reverse phase HPLC method with fluorescence detection (hr*ng/mL) |
| SN38 AUC24 | |
| SN38 GAUC24 | |
| APC AU C24 | 7-ethyl-10 [4-N-(5-aminopeptanoic acid)-1-piperidino]-carbonyloxycamptothecin (APC) area under concentration curve from 0 to 24 hours (hr*ng/mL) |
| ANC Nadir | Nadir values of absolute neutrophil counts (ANC) after the first irinotecan dose (day 12-14) |
| Diarrhea Grade | Toxicity was assessed using Common Toxicity Criteria version 2.0 Diarrhea observed during cycle of treatment. |

## Claims

1. An *in vitro* method wherein the incomplete or noisy measurement of multiple SNP loci on a given segment of a given chromosome of a target individual is used to determine the number of instances of the given segment in the genome of the target individual, the method comprising:

    (i) creating a set of one or more hypotheses about the number of instances of the given segment present in the genome of the target individual,
    (ii) measuring the amount of genetic data for some or all of the possible alleles at a plurality of SNP loci on the given segment, using tools and/or techniques taken from the group comprising Molecular Inversion Probes (MIP), Genotyping Microarrays, the Taqman SNP Genotyping Assay, quantitative PCR, the Illumina Genotyping System, other genotyping assays, fluorescent in-situ hybridization (FISH), and combinations thereof;
    (iii) determining the relative probability of each of the hypotheses given the measurements of the target individual's genetic data and possibly a related individual's genetic data where the determination of the relative probability of each hypothesis is made using quantitative techniques that do not make allele calls, wherein the quantitative techniques that do not make allele calls comprise matched filtering, and where the mean and standard deviation for the measurement of each locus is either known, unknown or uniform; and
    (iv) using the relative probabilities associated with each hypothesis to determine the number of instances of the chromosomal segment in the genome of the target individual;
    wherein the determination of the number of the given chromosome segment in the target individual is to screen for a chromosomal abnormality, that abnormality taken from a list comprising monosomy, uniparental disomy, trisomy, other aneuploidies, unbalanced translocation, and combinations thereof and further wherein the target individual is taken from a group consisting of a human fetus, a human embryo, a non-human fetus and a non-human embryo.

2. The method of claim 1, wherein the target individual's genetic data has been amplified using tools and or techniques taken from the group comprising Polymerase Chain Reaction (PCR), Ligand mediated PCR, degenerative oligonucleotide primer PCR, Multiple Displacement Amplification, allele-specific amplification and combinations thereof.

3. The method of claim 1, wherein the target individual's genetic data has been measured by analyzing substances taken from the group comprising the target individual's bulk diploid tissue, one or more diploid cells taken from the target individual, one or more blastocysts taken from the target individual, extra-cellular genetic material found on the target individual, extra-cellular genetic material from the target individual found in maternal blood, cells from the target individual found in maternal blood, genetic material known to have originated from the target individual, and combinations thereof.

4. The method of claim 1, wherein the determination of the number of chromosomes or chromosome segments in the target is used for the purpose of embryo selection in the context of in-vitro fertilization.

5. The method of claim 1, wherein the determination of the number of chromosomes or chromosome segments of the target is used for the purpose of prenatal genetic diagnosis.

6. A system configured to accomplish the method of claim 1.

7. A computer-implemented system configured to accomplish the method of claim 1.

**Patentansprüche**

1. In-vitro Verfahren, wobei die unvollständige oder verrauschte Messung mehrerer SNP-Loci auf einem gegebenen Segment eines gegebenen Chromosoms eines Zielindividuums verwendet wird, um die Anzahl des Vorkommens des gegebenen Segments im Genom des Zielindividuums zu bestimmen, wobei das Verfahren Folgendes umfasst:

(i) Erzeugen eines Satzes von einer oder mehreren Hypothesen über die Anzahl des Vorkommens des gegebenen Segments, das in dem Genom des Zielindividuums vorhanden ist,
(ii) Messen der Menge von genetischen Daten für einige oder alle möglichen Allele an einer Vielzahl von SNP-Loci auf dem gegebenen Segment, unter Verwendung von Werkzeugen und/oder Techniken, die aus der Gruppe, umfassend Molekulare Inversionssonden (MIP), Mikroarrays zur Genotypisierung, den Taqman-SNP-Genotypisierungsassay, quantitative PCR, das Illumina-Genotypisierungssystem, andere Genotypisierungsassays, Fluoreszenz-in-situ-Hybridisierung (FISH) und Kombinationen davon, entnommen werden;
(iii) Bestimmen der relativen Wahrscheinlichkeit jeder der Hypothesen angesichts der Messungen der genetischen Daten des Zielindividuums und möglicherweise der genetischen Daten eines verwandten Individuums, wo die Bestimmung der relativen Wahrscheinlichkeit jeder Hypothese unter Verwendung quantitativer Techniken erfolgt, die keine Allel-Calls vornehmen, wobei die quantitativen Techniken, die keine Allel-Calls vornehmen, ein Match-Filtern umfassen, und wo der Mittelwert und die Standardabweichung für die Messung jedes Locus entweder bekannt, unbekannt oder einheitlich ist; und
(iv) Verwenden der relativen Wahrscheinlichkeiten, die jeder Hypothese zugeordnet sind, um die Anzahl des Vorkommens des Chromosomensegments im Genom des Zielindividuums zu bestimmen;

wobei die Bestimmung der Anzahl des gegebenen Chromosomensegments in dem Zielindividuum zu einem Screening auf eine chromosomale Anomalie dient, wobei diese Anomalie aus einer Liste entnommen wird, die Monosomie, uniparentale Disomie, Trisomie, andere Aneuploidien, unbalancierte Translokationen und Kombinationen davon umfasst, und wobei das Zielindividuum ferner aus einer Gruppe entnommen wird, die aus einem menschlichen Fötus, einem menschlichen Embryo, einem nicht-menschlichen Fötus und einem nicht-menschlichen Embryo besteht.

2. Verfahren nach Anspruch 1, wobei die genetischen Daten des Zielindividuums amplifiziert wurden unter Verwendung von Werkzeugen und/oder Techniken, die aus der Gruppe, die Polymerase-Kettenreaktion (PCR), Liganden-vermittelte PCR, degenerative Oligonukleotid-Primer-PCR, Multiple Displacement Amplification, allelspezifische Amplifikation und Kombinationen davon umfasst, entnommen werden.

3. Verfahren nach Anspruch 1, wobei die genetischen Daten des Zielindividuums gemessen wurden, indem Substanzen analysiert wurden, die aus der Gruppe entnommen werden, die das diploide Gesamtgewebe des Zielindividuums, eine oder mehrere diploide Zellen, die dem Zielindividuum entnommen werden, eine oder mehrere Blastozysten, die dem Zielindividuum entnommen werden, extrazelluläres genetisches Material, das auf dem Zielindividuum gefunden wird, extrazelluläres genetisches Material von dem Zielindividuum, das im mütterlichen Blut gefunden wird, genetisches Material, von dem bekannt ist, dass es vom Zielindividuum stammt, und Kombinationen davon umfasst.

4. Verfahren nach Anspruch 1, wobei die Bestimmung der Anzahl von Chromosomen oder Chromosomensegmenten in dem Ziel zum Zweck einer Embryoselektion im Zusammenhang mit In-vitro-Fertilisation verwendet wird.

5. Verfahren nach Anspruch 1, wobei die Bestimmung der Anzahl von Chromosomen oder Chromosomensegmente des Ziels zum Zweck einer pränatalen genetischen Diagnose verwendet wird.

6. System, das konfiguriert ist, um das Verfahren nach Anspruch 1 durchzuführen.

7. Computerimplementiertes System, das konfiguriert ist zum Durchführen des Verfahrens nach Anspruch 1.

**Revendications**

1. Procédé *in vitro,* dans lequel la mesure incomplète ou bruyante de plusieurs loci SNP sur un segment donné d'un chromosome donné d'un individu cible est utilisée pour déterminer le nombre d'occurrences du segment donné dans le génome de l'individu cible, le procédé comprenant :

   (i) la création d'un ensemble d'une ou de plusieurs hypothèses quant au nombre d'occurrences du segment donné présente dans le génome de l'individu cible,
   (ii) la mesure de la quantité de données génétiques pour une partie ou l'ensemble des allèles possibles en une pluralité de loci SNP sur le segment donné, à l'aide d'outils et/ou de techniques choisi(e)s dans le groupe comprenant les sondes d'inversion moléculaire (MIP), les puces à ADN de génotypage, le dosage de génotypage SNP Taqman, la PCR quantitative, le système de génotypage Illumina, d'autres essais de génotypage, l'hybridation in situ en fluorescence (FISH) et des combinaisons de ceux-ci;
   (iii) la détermination de la probabilité relative de chacune des hypothèses compte tenu des mesures des données génétiques de l'individu cible et éventuellement des données génétiques d'un individu apparenté où la détermination de la probabilité relative de chaque hypothèse est effectuée en utilisant des techniques quantitatives qui ne procèdent pas à des appels d'allèles, dans lequel les techniques quantitatives qui n'effectuent pas d'appels d'allèles comprennent un filtrage adapté, et où la moyenne et l'écart type pour la mesure de chaque locus sont connus, inconnus ou uniformes ; et
   (iv) l'utilisation des probabilités relatives associées à chaque hypothèse pour déterminer le nombre d'occurrences du segment chromosomique dans le génome de l'individu cible ;

   dans lequel la détermination du nombre du segment chromosomique donné chez l'individu cible sert à dépister une anomalie chromosomique, cette anomalie étant choisie dans une liste comprenant la monosomie, la disomie uniparentale, la trisomie, d'autres aneuploïdies, une translocation déséquilibrée et des combinaisons de celles-ci et dans lequel l'individu cible est en outre choisi dans un groupe consistant en un fœtus humain, un embryon humain, un fœtus non-humain et un embryon non-humain.

2. Procédé selon la revendication 1, dans lequel les données génétiques de l'individu cible ont été amplifiées en utilisant des outils et/ou des techniques choisi(e)s dans le groupe comprenant la réaction en chaîne par polymérase (PCR), la LM-PCR, la DOP-PCR, l'amplification par déplacement multiple, l'amplification spécifique à un allèle et des combinaisons de celles-ci.

3. Procédé selon la revendication 1, dans lequel les données génétiques de l'individu cible ont été mesurées en analysant des substances choisies dans le groupe comprenant le tissu diploïde global de l'individu cible, une ou plusieurs cellules diploïdes prélevées de l'individu cible, un ou plusieurs blastocystes prélevés de l'individu cible, du matériel génétique extracellulaire trouvé sur l'individu cible, du matériel génétique extracellulaire de l'individu cible trouvé dans le sang maternel, des cellules de l'individu cible trouvées dans le sang maternel, du matériel génétique connu pour provenir de l'individu cible, et des combinaisons de ceux-ci.

4. Procédé selon la revendication 1, dans lequel la détermination du nombre de chromosomes ou de segments chromosomiques dans la cible est utilisée pour la sélection d'embryons dans le contexte de la fécondation in vitro.

**5.** Procédé selon la revendication 1, dans lequel la détermination du nombre de chromosomes ou de segments chromosomiques de la cible est utilisée pour le diagnostic génétique prénatal.

**6.** Système configuré pour exécuter le procédé selon la revendication 1.

**7.** Système mis en œuvre sur ordinateur configuré pour exécuter le procédé selon la revendication 1.

Fig. 1

Fig. 2

Fig. 3

Sample 0: Mean 29.974 and Std 1.320 N=148 Loci=37

Fig. 4

Sample 1: Mean 31.439 and Std 1.592 N=148 Loci=37

Fig. 5

Measurements: Mean 1.465 and Std 0.990. Adjusted Std 0.700 N=74 Loci=37

DIFFERENCE BETWEEN MALE AND FEMALE
MEASUREMENTS FOR EACH LOCUS

## Fig. 6

Sample 1: Mean 32.259 and Std 1.460

Ct CROSSOVER OF TAQMAN ASSAY
MEASURED IN CYCLE NUMBER

## Fig. 7

Sample 0: Mean 30.750 and Std 1.202

Fig. 8

Sample 1: 20 Loci: 2 Runs per locus. 0.3ng per well Ave Std Dev 0.597

Fig. 9

Sample 0: Mean 26.640 and Std 1.148

Ct CROSSOVER OF qPCR ASSAY
MEASURED IN CYCLE NUMBER

Fig. 10

Sample 1: Mean 27.652 and Std 1.401

Ct CROSSOVER OF qPCR ASSAY
MEASURED IN CYCLE NUMBER

Fig. 11

Measurements: Mean 1.012 and Std 0.750 Adjusted Std 0.530

Fig. 12

Fig. 13

Fig. 14

Fig. 15

Fig. 16

Fig. 17

Fig. 18

Fig. 19

Fig. 20

Fig. 21

Fig. 22

MUTATIONS < LOCUS > < AMINO ACID >

Fig. 23

MUTATIONS < LOCUS >< AMINO ACID >

Fig. 24

MUTATIONS < LOCUS >< AMINO ACID >

EP 3 373 175 B1

Fig. 25

Predictor Predict ANC using OLS method

Predicted outcome is in the lower 33.3% of the distribution over all records

Predictors used:
AUC (uM.hr/L): H 0 to H 24
log(AUC (uM hr/L) H 0 to H 24)
chr2 234447782
chr2 234451144

Rule Explanation: *Uses a linear regression model, with the model predictors selected using either the Least Angle Selection and Shrinkage Operator (LASSO) or best subset selection so as to maximize the correlation with test data.*

Histogram:

Dist of G111, Pred log(Value)=2.3673, R=0.63596, 95% cont=[1.3935 3.3411]

References:
Neter, J. Wasserman, W. & Kutner, M. Applied Linear Statistical Models: Regression, Analysis of Variance and Experimental Designs, 3rd Edition, Richard D. Irwin, Inc. (1990)

M. Rabinowitz, L. Myers, M. Banjevic, J. Sweetkind-Singer, J. Haberer, A. Chan, K. McCarin, R. Wolkowicz, *Rabinowitz, M. et al. Accurate prediction of HIV-1 drug response from the reverse transcriptase and protease

## Fig. 26

Fig. 27

EP 3 373 175 B1

**Enhanced Colorectal Cancer Treatment Report**

GSN
GENE SECURITY NETWORK

| NAME: R.E. Mission | Report Date | March 26, 2006 |
|---|---|---|
| MRN: 1122334455 | Clinical Indication: | |
| DOB: 08/08/1958 | ICD-9 153.9 | Malignant neoplasm, colon |
| | Staging of Colorectal | |
| Sex: Male | Carcinoma | Dukes C; TNM Stage IIIA |
| Therapy | Irinotecan | Q3 Week Dosing Regimen |
| Date of therapy | March 25, 2006 | |

| Other pertinent past medical history: | | |
|---|---|---|
| Abnormal bilirubin glucuronidation (Gilbert's) | | |
| Significant drug-drug interactions with patient current medications | Effect on irinotecan levels | |
| Phenytoin | ↓ | |
| Paroxetine | ↑ | |
| Ketoconazole | CONTRAINDICATED | |

| Laboratory and clinical EMR data | | |
|---|---|---|
| Data Sources: | | UGT1A1 Assay - 3/26/2006 |
| Quest Labs | 3/20/2006 | Heterozygous for UGT1A1 *28 |
| EMR Data | 3/26/2006 | $AUC_{CPT-11}$ (ng · h/mL) 5582.0 |
| | | $AUC_{SN-38}$ (ng · h/mL) 126.6 |
| White blood cell count | 3.0 K/uL | $AUC_{SN-38G}$ (ng · h/mL) 1,251.3 |
| Absolute neutrophil count | 3000/mm$^3$ | ANC Prediction: |
| Neutropenia grade | None | |
| Hemoglobin | 12 g/dL | |
| Platelet count | 150 K/uL | |
| Hyperbilirubinemia | 1.2 mg/dL | |
| Diarrhea grade | grade 2 (4-6 stools/day) | |
| | | Grade 2 Neutropenia |

| SUMMARY RECOMMENDATIONS | RATIONALE |
|---|---|
| **Pre-Treatment** | |
| Consider reducing starting dose to 300 mg/m2 | Gilbert's Syndrome |
| Discontinue ketoconazole 1 week prior to therapy, resume 2 days after therapy if indicated | Ketoconazole <--> Irinotecan Interaction |
| Consider alternative regimens not including irinotecan | *28/*28 genotype |
| **Day 1 Post Treatment Predictions** | |
| Decrease dose by 50 mg/m2 | Grade2 neutropenia (1000-1499/mm3) |
| Consider addition of colony stimulating factor | Grade2 neutropenia (1000-1499/mm3) |
| Omit dose until resolved to baseline, then reduce to 250 mg/m2 | Grade2 diarrhea (4-6 stools/day) |

## Fig. 28

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6720140 B, Hartley
- US 6489135 B, Parrott
- US 20040033596 A, Threadgill
- US 5994148 A, Stewart
- US 5635366 A, Cooke
- US 7058616 B, Larder
- US 6958211 B, Vingerhoets
- US 7058517 B, Denton
- US 7035739 B, Schadt
- US 6025128 A, Veltri
- US 5824467 A, Mascarenhas
- US 2004137470 A

### Non-patent literature cited in the description

- **RABINOWITZ, M. et al.** Accurate prediction of HIV-1 drug response from the reverse transcriptase and protease amino acid sequences using sparse models created by convex optimization. *Bioinformatics*, 2006, vol. 22 (5), 541-9